# EUROPEAN PATENT SPECIFICATION

(11) **EP 3 485 040 B1**
(45) Date of publication and mention of the grant of the patent: **14.04.2021**
(21) Application number: 17749378.0
(22) Date of filing: 17.07.2017
(51) Int. Cl.: C12Q 1/68, A61K 48/00, C12N 15/63

(54) **B-CELL-MIMETIC CELLS**
B-ZELL-MIMETISCHE ZELLEN
CELLULES MIMÉTIQUES DE LYMPHOCYTE B

(30) Priority: 18.07.2016 EP 16180000; 23.11.2016 EP 16200258
(43) Date of publication of application: 22.05.2019
(73) Proprietor: ETH Zurich, 8092 Zurich (CH)
(72) Inventor: FUSSENEGGER, Martin, 5506 Mägenwil (CH); XIE, Mingqi, 4051 Basel (CH)
(74) Representative: Latscha Schöllhorn Partner AG
(86) International application number: PCT/EP2017/067981
(87) International publication number: WO 2018/015330

(56) References cited:
- WO-A1-00/04171
- WO-A2-01/68828
- JAESEOK HAN ET AL: "Remission of Diabetes by Insulin Gene Therapy Using a Hepatocyte-specific and Glucose-responsive Synthetic Promoter", MOLECULAR THERAPY, vol. 19, no. 3, 1 March 2011 (2011-03-01), pages 470-478, XP055041439, ISSN: 1525-0016, DOI: 10.1038/mt.2010.255

## Description

The present invention relates to β-cell-mimetic cells. Methods for producing β-cell-mimetic cells as well as methods of use of β-cell-mimetic cells as a medicament and methods of use of β-cell-mimetic cells for the prevention, delay of progression or treatment of a metabolic disease in a subject are also provided.

### BACKGROUND OF THE INVENTION

Diabetes mellitus is a complex and progressive disease with a pathophysiology involving metabolic impairments that can lead to many clinical complications. Diabetes mellitus is currently estimated to affect at least 415 million people (1 in 11 adults) worldwide (Diabetes Atlas 7Th Edition, International Diabetes Federation, 2015), a number already exceeding the value for 2025 predicted a decade ago. The most characteristic feature of diabetic patients is a chronically elevated blood-glucose level, known as hyperglycaemia, that results from either an absolute loss of pancreatic insulin-producing β-cells (type-1 diabetes, T1D) or a progressive exhaustion of active β-cells due to environmental factors such as a sedentary lifestyle, malnutrition, or obesity (type-2 diabetes, T2D). Unless sufficiently treated in time, sustained hyperglycaemia can initiate many pathologic cascades that result in more severe disorders such as cardiovascular disease, renal failure, the metabolic syndrome, neuropathic pain, hormone dysfunction and cancer. Therefore, improved glycaemic control by a therapeutic intervention that either enables tightly controlled insulin delivery or restores a patient's β-cell function will be of utmost importance in diabetes treatment.

Because T1D patients suffer from complete insulin deficiency due to a selective autoimmune destruction of β-cells treatment options focus on a disciplined or automated supply of exogenous insulin. By contrast, the number of possible drug targets for T2D therapy is higher due to the progressive and multifactorial nature of this disease type. For example, incretin hormones (e.g., GLP-1-analogues) improve the efficiency of the exhausting β-cells to secrete insulin upon glucose stimulation. In recent years, studies capitalizing on the high capacity of mammalian cells to produce insulinogenic components within a patient have gained increased attention because they promise effective drug production, delivery and dosage. For example, the regeneration of functional glucose-responsive insulin-secreting β-cells from stem cells (Pagliuca FW et al., Cell 159, 428-439 (2014)) represents a major breakthrough for treating T1D: transplantation of these *ex vivo* reprogrammed cells into T1D patients would directly restore their defective glucose-stimulated insulin expression. Approaches based on the delivery of glucose-responsive insulin expression elements into extrapancreatic mammalian cell types (Han J et al., WJG 18, 6420-6426 (2012)) can protect against fundamental diabetic vulnerabilities such as autoimmune (re)-targeting in T1D (Aguayo-Mazzucato C and Bonner-Weir S, Nat Rev Endocrinol 6, 139-148 (2010)) and metabolic stress-induced β-cell apoptosis in T2D (Marzban L et al., Diabetes 55, 2192-2201 (2006)). Recently, synthetic biology-inspired rational circuit design has led to the engineering of immunoprotective implants that enable trigger-inducible insulin- (Stanley S et al., Nat Med 21, 92-98 (2015)) or GLP1-expression (Ye H et al., Science 332, 1565-1568 (2011)) with traceless and non-invasive signals. WO 01/68828 describes transfection of mucosal cells with the glucose-dependent insulinotropic polypeptide (GIP) promoter operably linked to insulin. WO 00/04171 discloses hepatocytes transfected with adenovirus constructs containing insulin under control of a chimeric serum albumin promoter containing glucose-inducible regulatory elements (GIREs) based on CACGTG motifs. An *in vivo* gene therapy approach using a viral vector to deliver a glucose-responsive synthetic promoter into the liver of mice has been described by Han J et al., Molecular Therapy 19, 470-478 (2011). However, neither of these approaches combines direct glucose sensing, endogenous real-time control of therapeutic dosage, and straightforward engineering of non-stem-cell human cells.

### SUMMARY OF THE INVENTION

The invention provides therapeutically applicable β-cell-mimetic cells and methods for producing such β-cell-mimetic cells. The β-cell-mimetic cells of the present invention comprise a carbohydrate-inducible transcriptional system that directly senses extracellular carbohydrate concentrations and is capable to coordinate the dose-dependent transcription of therapeutic proteins such as e.g. insulin and GLP-1. The system mimics core functions of pancreatic β-cells, which sense carbohydrates as glucose via a mechanism that combines glycolysis and stimulus-secretion coupling. Implanted β-cell-mimetic cells corrected insulin deficiency and self-sufficiently abolished persistent hyperglycaemia in T1D mice. Similarly, glucose-inducible GLP-1 transcription improved endogenous glucose-stimulated insulin release and glucose tolerance in T2D mice. The β-cell-mimetic cells of the present invention are useful for the treatment of metabolic diseases such as e.g. metabolic diseases selected from the group consisting of T1D, T2D, metabolic syndrome and cardiovascular disease.

Thus, in a first aspect, the invention relates to a recombinant cell comprising a nucleic acid construct comprising a promoter which is responsive to carbohydrate metabolism of said recombinant cell, wherein the promoter is operably linked to a gene coding for a therapeutic protein, wherein the recombinant cell further comprises a nucleic acid construct coding for a cellular component for sensing extracellular carbohydrates, wherein the cellular component for sensing extracellular carbohydrates is a voltage-gated calcium channel and wherein the promoter which is responsive to carbohydrate metabolism is responsive to a physiological effect of membrane depolarization caused by the carbohydrate metabolism of said recombinant cell.

In a further aspect, the invention relates to an encapsulated cell comprising a recombinant cell.

In a further aspect, the invention relates to a recombinant cell or an encapsulated recombinant cell for use as a medicament.

In a further aspect, the invention relates to a recombinant cell or an encapsulated recombinant cell for use in a method for the prevention, delay of progression or treatment of a metabolic disease in a subject.

In a further aspect, the invention relates to a method of producing a recombinant cell expressing a therapeutic protein, said method comprising the steps of:
(a) obtaining a population of cells;
(b) transfecting said population of cells with a nucleic acid construct comprising a promoter which is responsive to a physiological effect of membrane depolarization caused by a product of the carbohydrate metabolism of said cell , wherein the promoter is operably linked to a gene coding for a therapeutic protein, and wherein said population of cells is further transfected with a nucleic acid construct encoding a cellular component for sensing extracellular carbohydrates, wherein the cellular component for sensing extracellular carbohydrates is a voltage-gated calcium channel;
(c) incubating the population of transfected cell in the presence of carbohydrates for a sufficient time to permit the transfected cells to express a therapeutic protein.

In a further aspect, the invention relates to a method to deliver a nucleic acid construct to a cell, wherein the nucleic acid construct comprises a promoter which is responsive to a physiological effect of membrane depolarization caused by a product of the carbohydrate metabolism of said cell and wherein said cell is further transfected with a nucleic acid construct encoding a cellular component for sensing extracellular carbohydrates, wherein the cellular component for sensing extracellular carbohydrates is a voltage-gated calcium channel, wherein the promoter is operably linked to a gene coding for a therapeutic protein, comprising administering said nucleic acid construct to said cell, whereby said gene coding for a therapeutic protein is expressed in said cell in response to carbohydrate stimulation.

In a further aspect the present invention relates to a method for producing a therapeutic protein in vivo in a mammal, said method comprising:
(a) providing an in vitro population of recombinant cells into an implantable semi-permeable device;
(b) implanting the device with the cell population into a mammalian host; and
(c) maturing the cell population in said device in vivo such that at least some cells of the cell population are cells that produce a therapeutic protein in response to carbohydrate stimulation in vivo.

In a further aspect, the invention relates to an in vitro cell culture comprising the recombinant cell, wherein said recombinant cell is expressing a protein, preferably a therapeutic protein in the presence of carbohydrates.

### BRIEF DESCRIPTION OF THE DRAWINGS

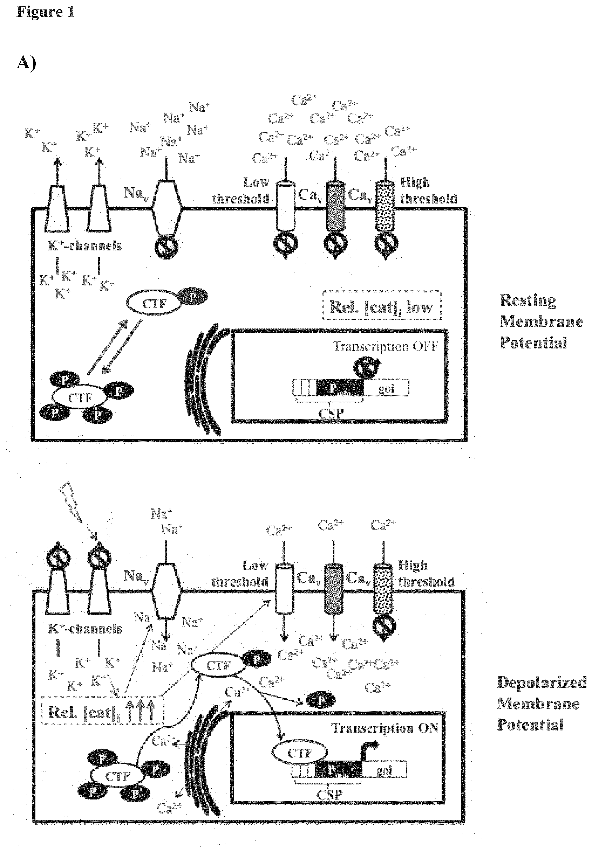

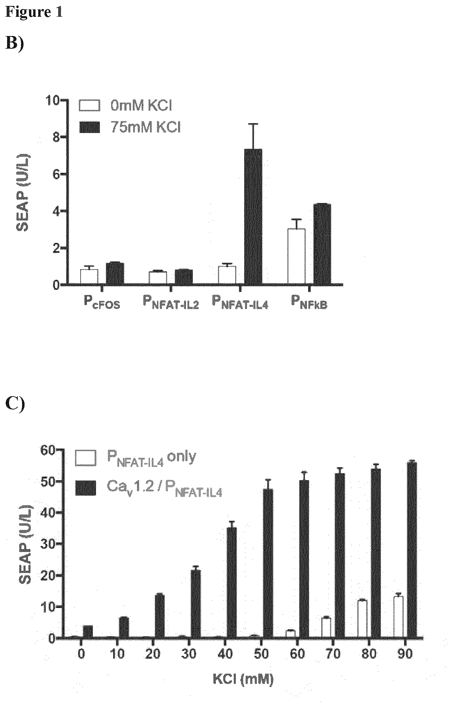

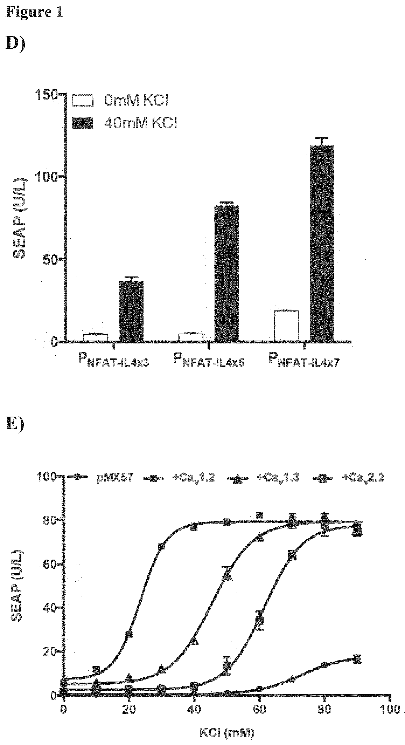

**Figure 1** shows engineering of an excitation-transcription coupling system in mammalian cells. **(A)** Scheme of a synthetic excitation-transcription coupling system. At resting membrane potentials, basic ion currents keep the relative concentration of intracellular cations (rel.[cat.]ᵢ) low and the plasma membrane hyperpolarized. Gene expression from calcium-specific promoters (CSPs) remains inactive. Stimuli that lead to a successive increase in intracellular cations either by blocking outward potassium channels (K⁺-channels) or by inducing the entry of cations result in membrane depolarization. Depending on the degree of depolarization and the subsequent instant membrane potential, different activation threshold-dependent voltage-gated sodium (Naᵥ) or calcium (Caᵥ) channels open and amplify the depolarization signal. Sustained increases of intracellular calcium levels activate various calcium-regulated transcription factors (CTFs) that translocate to the nucleus and initiate reporter gene transcription from synthetic cognate CSPs. **(B)** Calcium-specific promoters activated by chemically induced membrane depolarization. HEK-293 cells were transfected with pMX53 (P_{cFOS}-SEAP-pA), pHY30 (P_{NFAT-IL2}-SEAP-pA), pMX56 (P_{NFAT-IL4}-SEAP-PA) or pKR32 (P_{NFkB}-SEAP-pA) and grown in cell culture medium containing 0 or 75 mM potassium chloride (KCl). SEAP levels in the culture supernatants were profiled at 48 h after the addition of KCl. (C) Caᵥ1.2-amplified excitation-transcription coupling. HEK-293 cells were co-transfected with pMX56 (P_{NFAT-IL4}-SEAP-pA) and either Cav1.2 (pCaV1.2/pCavb3/pCaVα2δ1; 1:1:1, w/w) or pcDNA3.1(+), and the cells were grown for 48 h in cell culture medium containing different KCl concentrations before SEAP levels in the culture supernatants were profiled. **(D)** Optimization of the P_{NFAT-IL4}-promoter. HEK-293 cells were co-transfected with equal amounts of Caᵥ1.2 and SEAP expression vectors driven by promoter architectures containing three (pMX56, (NFAT_{IL4})₃-Pₘᵢₙ-SEAP-pA), five (pMX57, (NFAT_{IL4})₅-Pₘᵢₙ-SEAP-pA) or seven (pMX58, (NFAT_{IL4})₇-Pₘᵢₙ-SEAP-pA) mouse IL4-derived NFAT repeat (NFAT_{IL4}) sequences. Transfected cells were grown for 48 h in cell culture medium containing 0 or 40 mM KCl before SEAP levels in culture supernatants were profiled. **(E)** Activation threshold-dependent excitation-transcription coupling. HEK-293 cells were co-transfected with pMX57 and either pcDNA3.1(+), Caᵥ1.2 (pCaV1.2/pCavb3/pCaVα2δ1; 1:1:1, w/w), Caᵥ1.3 (pCaV1.3/pCavb3/pCaVα2δ1; 1:1:1, w/w) or Caᵥ2.2 (pCav2.2/pCavb3/pCaVα2δ1; 1:1:1, w/w), and the cells were grown for 48 h in cell culture medium containing different KCl concentrations before SEAP levels in the culture supernatants were profiled. All data presented are mean ± SD, n ≥ 5.

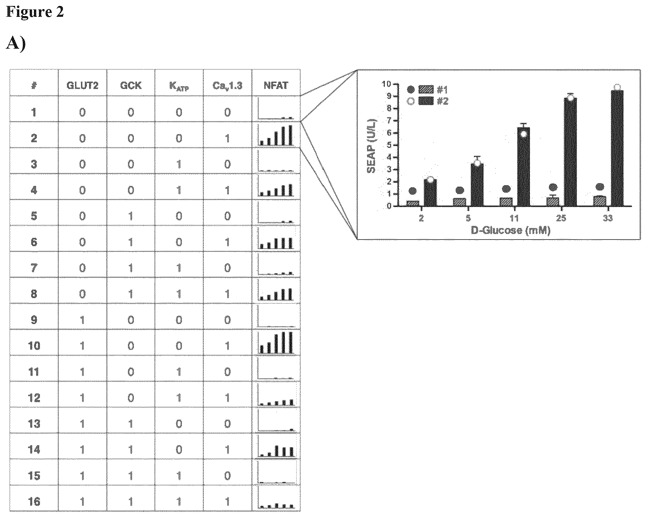

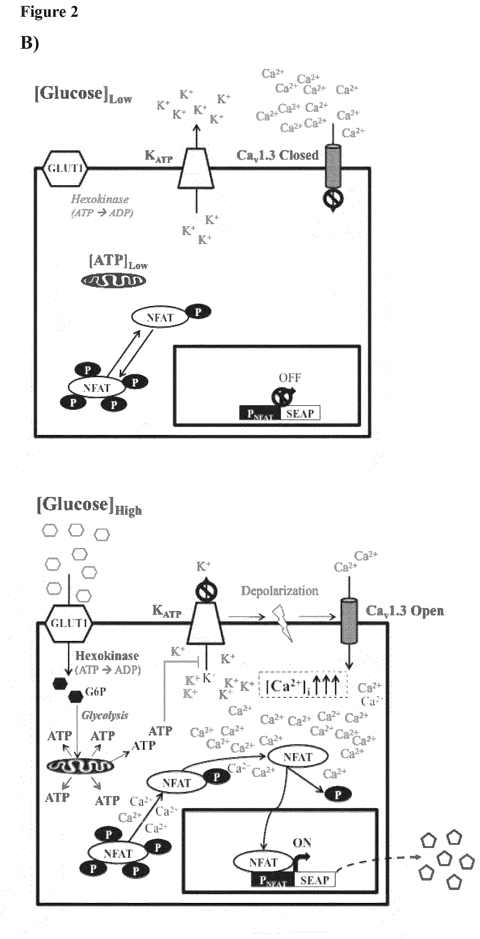

**Figure 2** shows glucose sensing in extrapancreatic human cells. **(A)** Contributory analysis of ectopically expressed glucose-sensing components in extrapancreatic human cells. HEK-293 cells were co-transfected with pMX57 and either (1) mammalian expression vectors for hGLUT2 (pcDNA3.2/v5-DEST hGlut2), GCK (pMX90), K_{ATP} (pCMV-hSUR1/pCMV6-hKir6.2; 1:1, w/w) and Caᵥ1.3 (pCaV1.3/pCavb3/pCaVα2δ1; 1:1:1, w/w) or (0): equal amounts of pcDNA3.1(+) (P_{hCMV}-MCS-pA). Twenty-four hours after transfection and cultivation in low glucose medium (2 mM), D-glucose was added to the indicated final concentrations. SEAP levels in the culture supernatants were scored at 48 h after the addition of D-glucose. Data presented are mean ± SD, n ≥ 5; Circles indicate simulation results. **(B)** Schematic representation of a hypothetical glucose-sensing mechanism in HEK-293 cells. Low levels of extracellular glucose are insufficient in inducing membrane depolarization to activate voltage-gated Caᵥ1.3 channels. By contrast, higher levels of extracellular glucose are taken up by mammalian cells to generate increased amounts of ATP. The subsequent closure of ATP-sensitive potassium channels (K_{ATP}) activates Caᵥ1.3, resulting in increased Ca²⁺ influx and the calcineurin-dependent activation of NFAT-regulated transcription units.

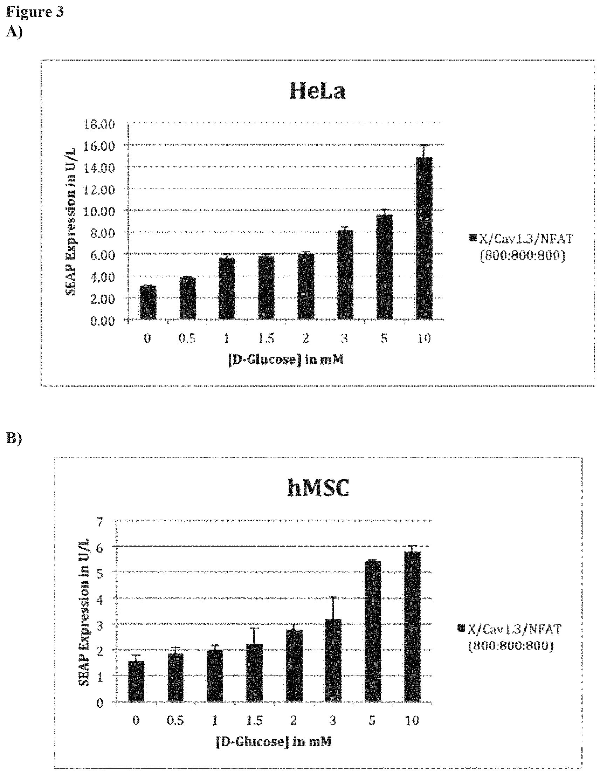

**Figure 3** shows Caᵥ1.3/P_{NFAT-IL4}-regulated SEAP expression in HeLa and human MSCs. (A) HeLa and (B) hMSCs were co-transfected with equal plasmid amounts of Caᵥ1.3 (pCaV1.3/pCavb3/pCaVα2δ1; 1:1:1, w/w), pMX57 and pcDNA3.1(+), and the cells were cultured in glucose-free medium for 12 h before different concentrations of D-glucose were added. Forty-eight hours after the addition of D-glucose, the SEAP levels in the culture supernatants were scored. Data presented are mean ± SD, n ≥ 3.

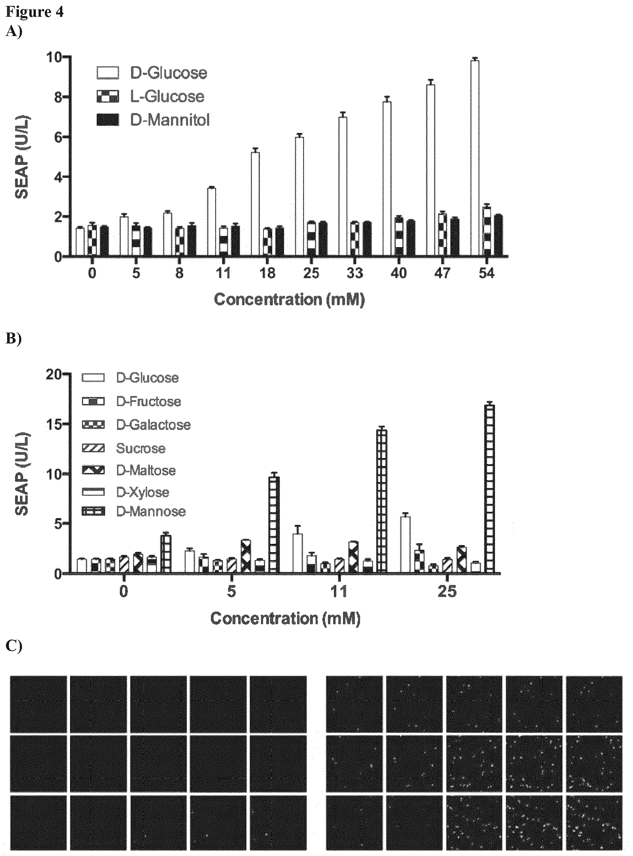

**Figure 4** shows characterization of Caᵥ1.3/P_{NFAT-IL4}-constituted excitation-transcription coupling systems. (A-B) Substrate specificity of Caᵥ1.3/pMX57-transgenic mammalian cells. HEK-293 cells were co-transfected with Caᵥ1.3 (pCaV1.3/pCavb3/pCaVα2δ1; 1:1:1, w/w) and pMX57, and the cells were cultured in glucose-free medium for 12 h before different (A) glucose isomers and osmotic controls or **(B)** nutritional sugar compounds were added. Forty-eight hours after the addition of control compounds, the SEAP levels in the culture supernatants were scored. Data presented are mean ± SD, n ≥ 3. **(C)** Right: Fluorescence micrographs profiling representative TurboGFP expression in HEK-293 cells co-transfected with Caᵥ1.3 and pFS119 ((NFAT_{IL4})₅-Pₘᵢₙ-TurboGFP:dest1-pA) and cultured in medium containing different concentrations of D-glucose (D-Glc) or potassium chloride (KCl). Left: Control cells transfected with pcDNA3.1(+) and pFS119.

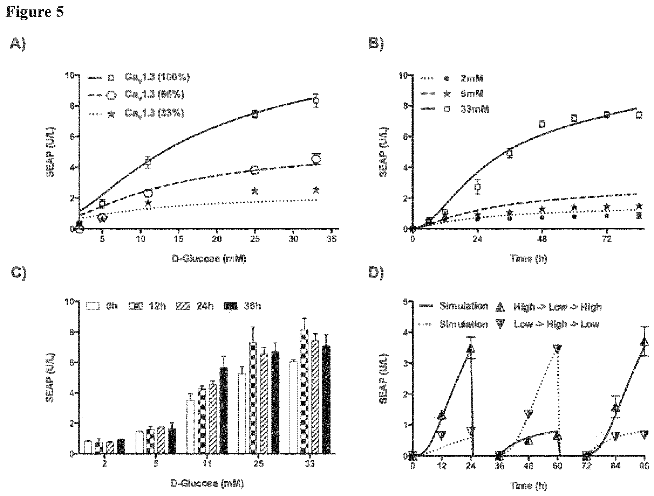

**Figure 5** shows characterization of the Caᵥ1.3-transgenic HEK-293_{NFAT-SEAP1} cell line. **(A)** Caᵥ1.3-dependent glucose sensing. Twenty-four hours after the transfection of HEK-293_{NFAT-SEAP1} cells with different amounts of Caᵥ1.3 expression vectors (pCaV1.3/pCavb3/pCaVα2δ1; 1:1:1, w/w) and cultivation in low-glucose medium (2 mM), D-glucose was added at the indicated final concentrations. The SEAP levels in the culture supernatants were scored at 48 h after the addition of D-glucose. Curves show corresponding simulations for data that was (100%) and was not (66% and 33%) used for calibrating the model. Data presented are mean ± SD, n ≥ 5. **(B)** SEAP expression kinetics. Twelve hours after cultivation in low-glucose medium (2 mM), Caᵥ1.3-transgenic HEK-293_{NFAT-SEAP1} cells were grown in cell culture medium containing different concentrations of D-glucose. The SEAP levels in the culture supernatants were profiled every 12 h. Solid and dashed curves show corresponding model-based simulations. Data presented are mean ± SD, n ≥ 5. **(C)** Time-delayed glucose responsiveness of the Caᵥ1.3/ HEK-293_{NFAT-SEAP1} system. Caᵥ1.3-transgenic Caᵥ1.3/HEK-293_{NFAT-SEAP1} cells were cultured in low-glucose medium (2 mM) for 0-36 h before D-glucose was added at the indicated final concentrations. Forty-eight hours after the addition of D-glucose, the SEAP levels in the culture supernatants were profiled. Data presented are mean ± SD, n ≥ 3. **(D)** Reversibility of the synthetic excitation-transcription coupling system. Caᵥ1.3-transgenic Caᵥ1.3/HEK-293_{NFAT-SEAP1} cells were cultured in high-D-glucose medium (40 mM) or low-D-glucose medium (5 mM) for 72 h while resetting the cell density to 0.75 x 10⁶ cells/mL and alternating D-glucose concentrations every 24 h followed by extensive washing over 12 h. The SEAP levels in the culture supernatants were profiled every 12 h within 24 h intervals of exposure to high/low glucose. Solid and dashed curves show corresponding model-based simulations. Data presented are mean ± SD, n ≥ 4.

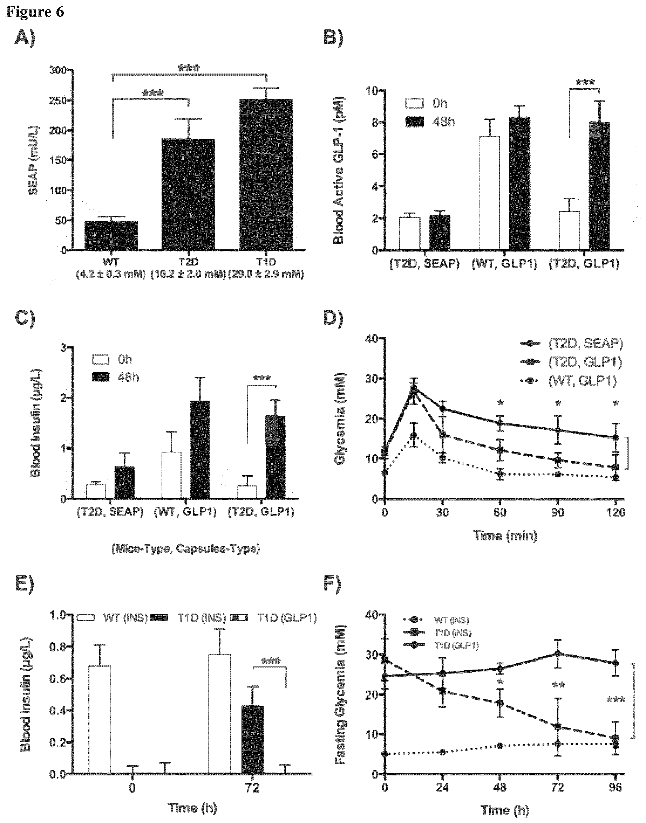

**Figure 6** shows Caᵥ1.3-dependent glucose sensing and antidiabetic potential in diabetic mice. **(A)** Dose-dependent glycaemia-induced SEAP expression in different diabetic mouse models. HEK-293_{NFAT-SEAP1} cells were transfected with Caᵥ1.3 and microencapsulated into alginate-poly-(L-lysine)-alginate beads. Capsules (1 x 10⁴; 500 cells/capsule) were implanted into mice suffering from different types of diabetes. The SEAP levels in the bloodstream of treated animals were quantified 48 h after implantation. **(B, C)** Self-sufficient GLP-1 expression in wild-type and type-2 diabetic mice. HEK-293 cells were co-transfected with Caᵥ1.3 and pMX115 ((NFAT_{IL4})₉-Pₘᵢₙ-shGLP1-pA), and the cells were then microencapsulated into alginate-poly-(L-lysine)-alginate beads (GLP-1 capsules). Control implants consisted of equally encapsulated Caᵥ1.3-transgenic HEK-293_{NFAT-SEAP1} cells (SEAP capsules). Capsules (1 x 10⁴; 500 cells/capsule) were implanted into wild-type (WT) or type-2 diabetic (T2D) mice. (B) GLP-1 and (C) insulin levels in the blood were profiled at 48 h after implantation. **(D)** Intraperitoneal glucose tolerance test (IPGTT) of wild-type and type-2 diabetic mice. Forty-eight hours after implantation and prior to serum collection, the same groups of mice as in (B and C) received an intraperitoneal injection of aqueous 2 g/kg D-glucose, and the glycaemic profile of each animal was tracked every 30 min. All data are shown as the mean ± SEM, and the analysis as performed with a two-tailed t-test (n = 8 mice). * P < 0.05, ** P < 0.01, *** P < 0.001 vs. control. **(E)** Self-sufficient insulin expression in wild-type and type-1 diabetic mice. HEK-293 cells were co-transfected with Caᵥ1.3 and pMX100 ((NFAT_{IL4})₉- Pₘᵢₙ-mINS-pA), and the cells were then microencapsulated into alginate-poly-(L-lysine)-alginate beads (INS capsules). Control implants consisted of equally encapsulated Caᵥ1.3/pMX115-transgenic HEK-293 cells (GLP-1 capsules). Capsules (1 x 10⁴; 500 cells/capsule) were implanted into wild-type (WT) or type-1 diabetic (T1D) mice. Serum insulin levels (detection limit: 0.2 µg/L) were profiled at 72 h after capsule implantation and 4 h after food intake. **(F)** Self-sufficient glycaemic control of Caᵥ1.3/pMX100-transgenic implants. The fasting glycaemia of the same groups of mice as in (E) was tracked for 96 h after capsule implantation. All data in (E-F) are shown as the mean ± SEM, and the statistical analysis was performed with a two-tailed t-test (n = 8 mice). * P < 0.05, ** P < 0.01, *** P < 0.001 vs. control.

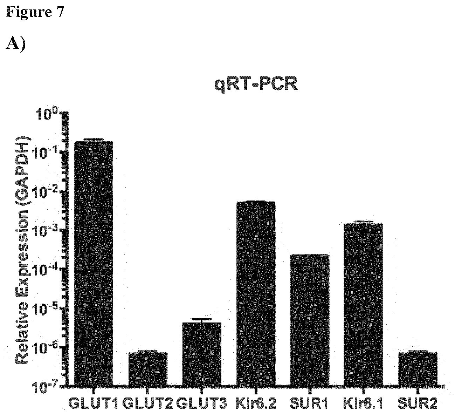

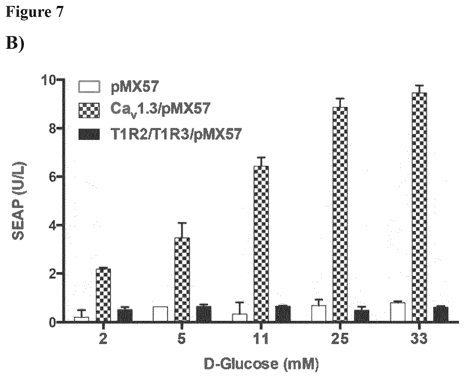

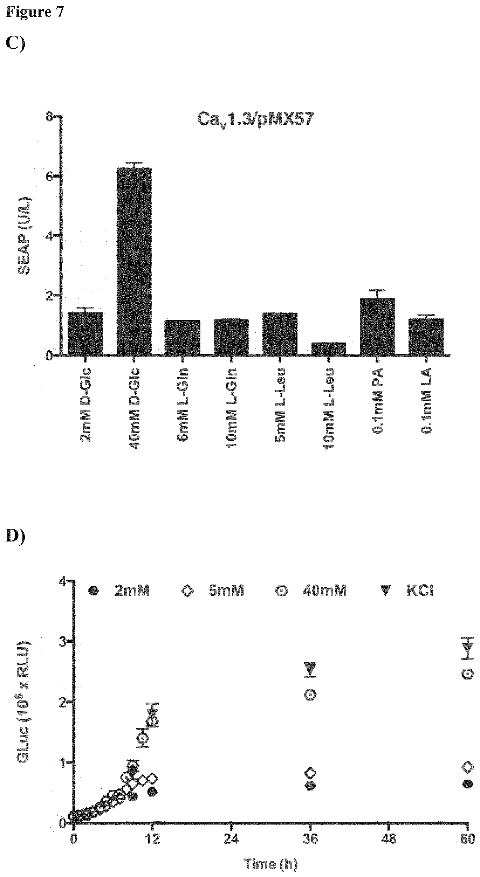

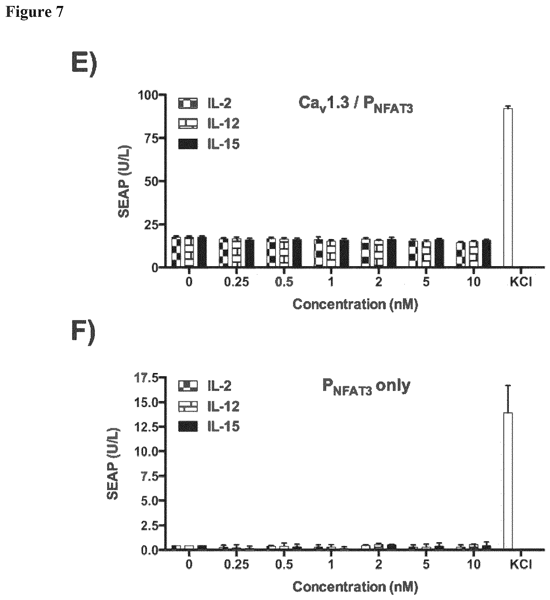

**Figure 7** shows glucose-sensor control experiments. (A) Quantitative RT-PCR-based expression profiling of endogenous glucose transporters and K_{ATP}-channels in HEK-293 using specific primers shown in Table S2. Transcription levels were normalized to glyceraldehyde 3-phosphate dehydrogenase (GAPDH) transcripts by setting undetermined values to a maximum Ct of 40 cycles. Data are mean ±SD, n=3. (B) Comparison of different glucose sensors in mammalian cells. HEK-293 cells were co-transfected with 1000ng pMX57 and 1000ng of either pcDNA3.1(+), Caᵥ1.3 (pCaV1.3 / pCaVb3 / pCaVα2δ1; 1:1:1, w/w) or the full sweet taste receptor componentry (pT1R2 / pT1R3 / pGNAT3; 1:1:1, w/w). 24h after transfection and cultivation in low glucose medium (2mM), D-glucose was added to final concentrations as indicated on the x-axis. SEAP levels in the culture supernatants were scored at 48h after addition of D-glucose. Data presented are mean ± SD, n ≥ 5. (C) Substrate specificity of Caᵥ1.3/pMX57-transgenic mammalian cells. HEK-293 cells were co-transfected with Caᵥ1.3 (pCaV1.3 / pCaVb3 / pCaVα2δ1; 333ng each) and pMX57 (1000ng) and cultivated in glucose-free medium for 12h before different concentrations of D-glucose (D-Glc), glutamine (L-Gln), leucine (L-Leu), palmitic acid (PA) or linoleic acid (LA) were added. 48h after addition of control compounds, SEAP levels in the culture supernatants were scored. Data presented are mean ± SD, n ≥ 3. (D) GLuc expression kinetics. HEK-293 cells were co-transfected with Caᵥ1.3 (pCaV1.3 / pCaVb3 / pCaVα2δ1; 333ng each) and pWH29 ((NFAT_{IL4})₅-Pₘᵢₙ-GLuc-pA; 1000ng) and cultivated in low-glucose medium (2mM) for 12h before different concentrations of D-Glucose (5 or 40mM) or KCl (40mM) were added. GLuc levels in the culture supernatants were profiled at different time points after the addition of inducer compounds as indicated on the x-axis. All data presented are mean ± SD, n ≥ 3. (E, F) Insensitivity of Cav1.3/pMX57-transgenic HEK-293 cells to cytokine signaling. HEK-293 cells were cotransfected with pMX57 (P_{NFAT3}-SEAP-pA; 1000ng) and either (E) Caᵥ1.3 (pCaV1.3/pCaVb3/pCaVα2δ1; 333ng each) or (F) pcDNA3.1(+) (1000ng) and cultivated in glucose-free medium for 12h before different concentrations of recombinant human interleukin 2 (IL-2), interleukin 12 (IL-12) and interleukin 15 (IL-15) or potassium chloride (40mM KCl; positive control) were added and SEAP levels were profiled in the culture supernatants after 48h. Data presented are mean ±SD, n≥3.

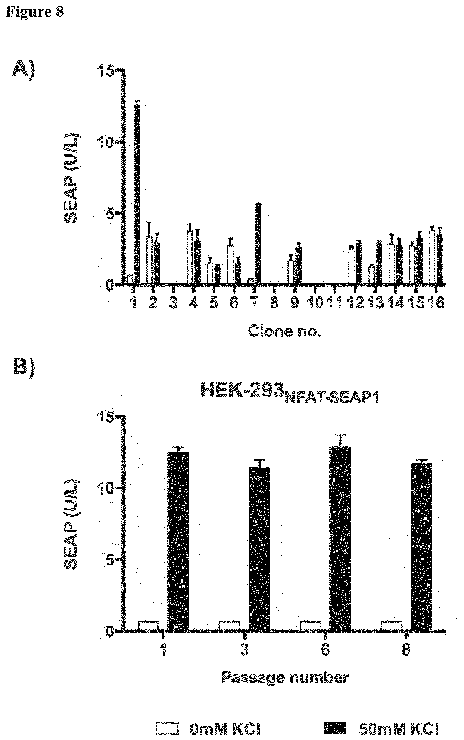

**Figure 8** shows design and construction of the stable HEK-_{293NFAT-SEAP1} cell line. (A) Depolarization-stimulated SEAP expression of different P_{NFAT-IL4}-transgenic cell clones (HEK-293_{NFAT-SEAP1}). HEK-293 cells were stably transfected with pMX57 ((NFAT_{IL4})₅-Pₘᵢₙ-SEAP-pA) and 16 randomly selected cell clones were profiled for their depolarization-stimulated SEAP regulation performance by cultivating them for 48h in the presence (50mM) or absence (0mM) of potassium chloride (KCl). (B) Stable depolarization-stimulated SEAP expression of the HEK-293_{NFAT-SEAP1} cell line. 5x 10⁴ HEK-293_{NFAT-SEAP1} cells from different generations were cultivated for 48h in the presence (50mM) or absence (0mM) of potassium chloride (KCl) before SEAP levels in the culture supernatants were scored. All data presented are mean ± SD, n ≥ 3.

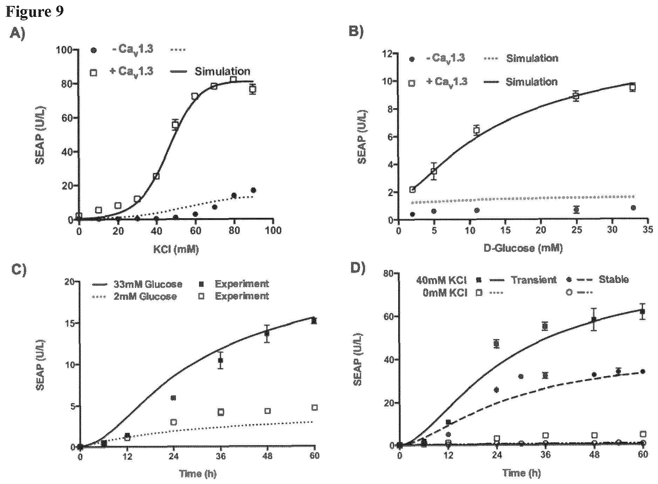

**Figure 9** shows experimental data (symbols) and simulation results (lines) for D-glucose- and KCl-stimulated SEAP expression in vitro. (A) Cav1.3-dependent excitation-transcription coupling. HEK-293 cells were co-transfected with pMX57 and either pcDNA3.1(+) (-Caᵥ1.3) or Caᵥ1.3 (+Caᵥ1.3), and the cells were grown for 48 h in cell culture medium containing different KCl concentrations before SEAP levels in the culture supernatants were profiled (see also Fig. IE). All data presented are mean ± SD, n ≥ 5. (B) D-glucose activated P_{NFAT3}-activation. HEK-293 cells were co-transfected with Caᵥ1.3 and pMX57, and the cells were cultured in low-glucose medium (2 mM) for 12 h before D-glucose was added to the indicated final concentrations. Forty-eight hours after the addition of control compounds, the SEAP levels in the culture supernatants were scored. Data presented are mean ± SD, n ≥ 3. (C, D) SEAP expression kinetics. Twelve hours after transfection of 9 x 10⁵ HEK-293 cells with pMX57 (P_{NFAT3}-SEAP-pA) and Caᵥ1.3 (pCaV1.3/pCaVb3/ pCaVα2δ1; 1:1:1, w/w), culture supernatants were exchanged by fresh medium containing different D-glucose- (C) and KCl- (D) concentrations. SEAP levels in the culture supernatants were profiled every 12 h. Circles represent equally treated Caᵥ1.3-transgenic HEK-_{293NFAT-SEAP1} cells. Data presented are mean ± SD, n ≥ 3.

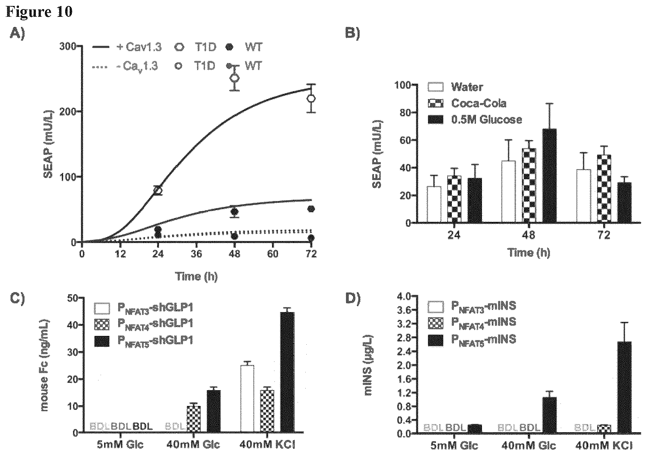

**Figure 10** shows glucose-sensor in vivo control experiments. (A) Caᵥ1.3-dependent SEAP-expression kinetics in vivo. Wild-type (WT, black) or type 1 diabetic (T1D, white) mice were implanted with 1 x 10⁴ microencapsules (500 cells /capsule) containing HEK-293_{NFAT-SEAP1} cells transfected with either pcDNA3.1(+) (-Caᵥ1.3) or Caᵥ1.3 (+Caᵥ1.3), and SEAP levels in the bloodstream were quantified every 24h (opaque or hollow circles). Solid or dashed curves show corresponding model-based simulations. The data are shown as the mean ± SD, n=8 mice. (B) Diet-induced glucose sensing in vivo. Wild-type mice were implanted with 1 x 10⁴ microcapsules (500 x Caᵥ1.3- transgenic HEK-293_{NFAT-SEAP1} cells/capsule) and received 4x daily oral administrations of 200µl water, Coca-Cola ® or aqueous D-glucose (0.5M). SEAP levels in the bloodstream were quantified every 24h after capsules implantation. The data are shown as the mean ≥ SD, n=8 mice. (C, D) Optimization of the P_{NFAT}-IL4-promoter for glucose- and depolarization-stimulated (C) shGLP1- and (D) mINS-expression. (C) HEK-293 cells were co-transfected with 1000ng Caᵥ1.3 and 1000ng of pMX61 ((NFAT_{IL4})₅-Pₘᵢₙ-shGLP1-pA), pMX117 ((NFAT_{IL4})₇-Pₘᵢₙ-shGLP1-pA) or pMX115 ((NFAT_{IL4})₉-Pₘᵢₙ-shGLP1-pA) and cultivated in low-glucose medium (2mM) for 12h before different concentrations of D-glucose (Glc) or potassium chloride (KCl) were added. 48h after addition of control compounds, murine IgG levels in the culture supernatants were quantified (BDL: below detection limit of 9.375ng/mL). (D) HEK-293 cells were co-transfected with 1000ng Caᵥ1.3 and 1000ng of pMX68 ((NFAT_{IL4})₅-Pmin-mINS-pA), pMX99 ((NFAT_{IL4})₇-Pₘᵢₙ-mINS-pA) or pMX100 ((NFAT_{IL4})₉-Pₘᵢₙ-mINSpA) and cultivated in low-glucose medium (2mM) for 12h before different concentrations of D-glucose (Glc) or potassium chloride (KCl) were added. 48h after addition of control compounds, murine insulin levels in the culture supernatants were quantified (BDL: below detection limit of 0.2µg/L). All data presented are mean ± SD, n ≥ 3.

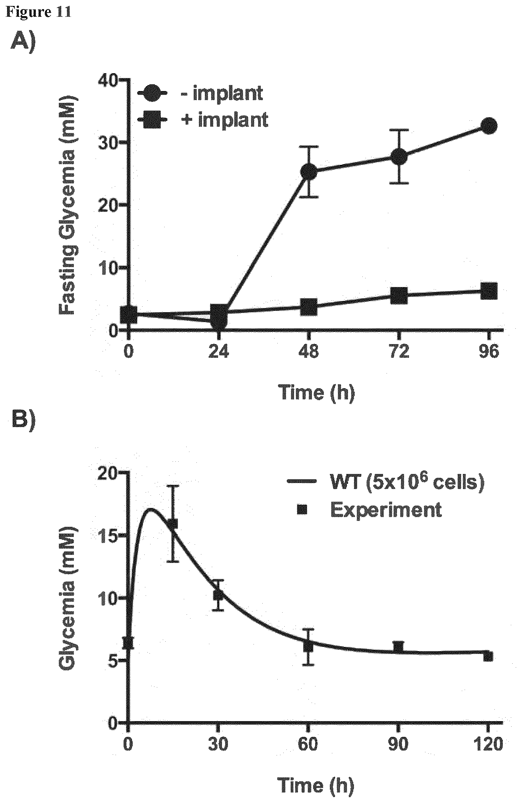

**Figure 11** shows (A) Glycaemic control in healthy and T1D mice. (Circles) Fasting CD-1 Swiss albino mice (2x 18h/day) were injected with a single dose of freshly diluted alloxan monohydrate (ALX; 200 mg/kg in 300µL phosphate buffered saline) and fasting glycaemia was measured every 24h after ALX injection. (Squares) Equally treated CD-1 Swiss albino mice harboring implants containing 5 x 10⁶ microencapsulated Cav1.3/pMX100-transgenic HEK-293 cells. Fasting glycaemia data are shown as the mean ± SD, n = 6 mice. (B) Model simulations for glucose tolerance in healthy WT-mice. Forty-eight hours after implantation of 5 x 10⁶ Caᵥ1.3 transgenic HEK-293_{NFAT-SEAP1} cells, CD-1 Swiss albino mice received an intraperitoneal injection of aqueous 2 g/kg D-glucose, and the glycaemic profile of each animal was tracked every 30 min. The curve shows a corresponding model-based simulation. All data shown as the mean ± SD, n = 8 mice.

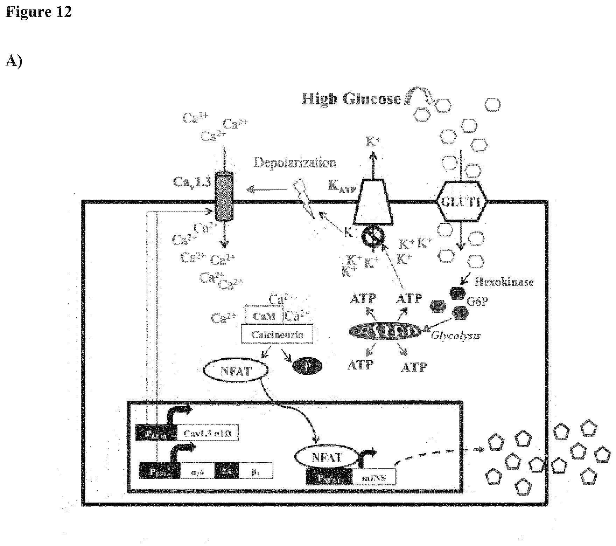

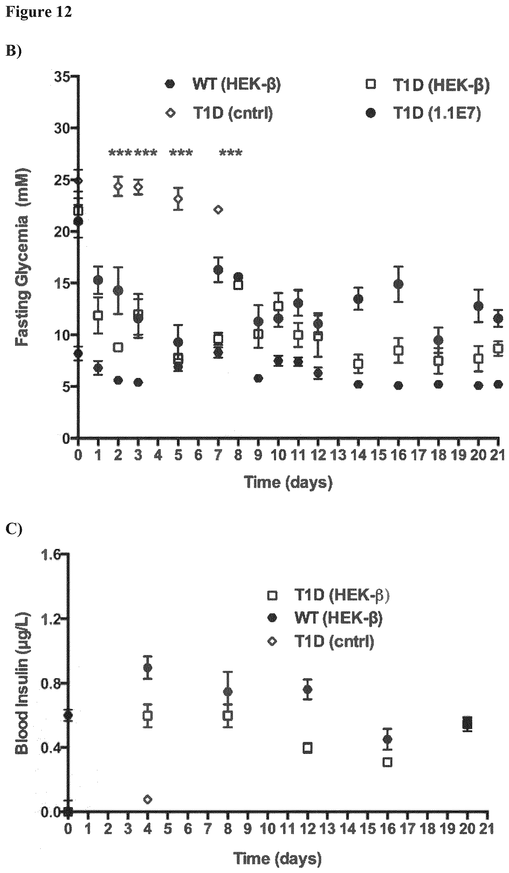

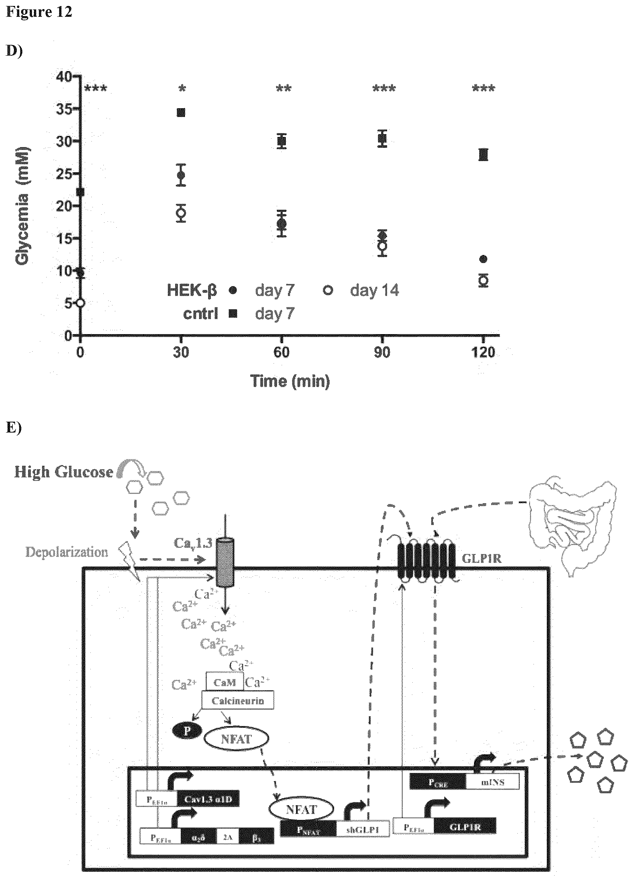

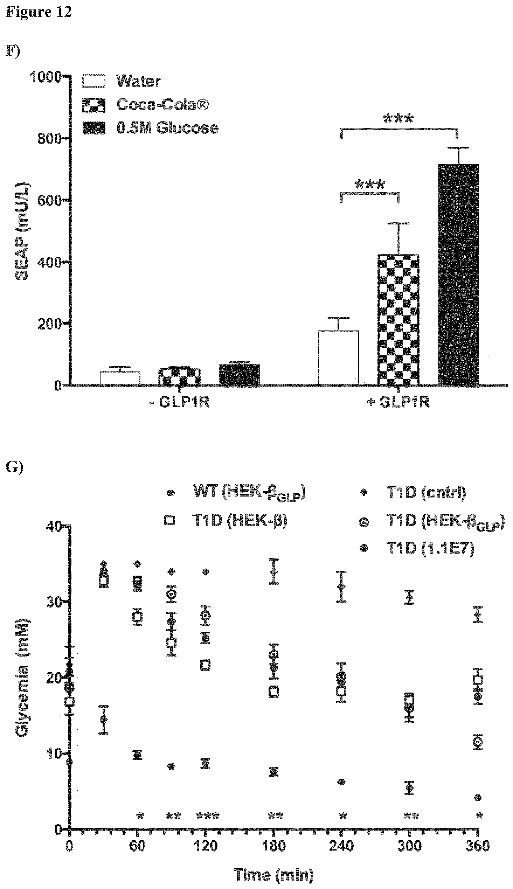

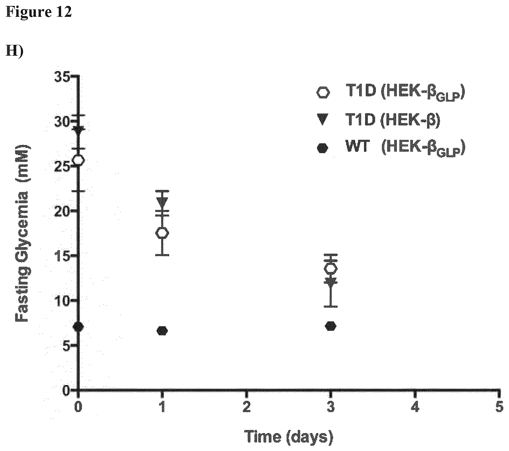

**Figure 12** shows treatment potential of β-cell-mimetic designer cells in type-1 diabetic mice. **(A)** Schematic of HEK-β. Extracellular D-glucose triggers glycolysis-dependent membrane depolarization which activates the voltage-gated calcium channel Caᵥ1.3, resulting in Ca²⁺ influx, induction of the calmodulin/calcineurin signaling cascade and P_{NFAT}-mediated induction of insulin expression and secretion. **(B)** Self-sufficient glycemic control in wild-type and type-1 diabetic mice. 5x10⁶ HEK-β cells or 1.1E7 cells were microencapsulated in alginate-poly-(L-lysine)-alginate beads (500 cells/capsule) and implanted into wild-type (WT) or type-1 diabetic (T1D) mice (1x10⁴ capsules/mice). Control implants contained microencapsulated Caᵥ1.3/pMX115-transgenic HEK-293 cells (cntrl). Fasting glycemia of treated animals was recorded for 3 weeks. T1D mice treated with negative-control implants did not survive the first glucose tolerance test on day 7 shown in (D). **(C)** Self-sufficient insulin expression in wild-type and type-1 diabetic mice. Postprandial blood insulin levels of the treatment groups shown in (B) were profiled every 4 days for up to 3 weeks. **(D)** Intraperitoneal glucose tolerance tests in type-1 diabetic mice. On days 7 and 14, the treatment groups shown in (B, C) received intraperitoneal D-glucose (2g/kg) injections and the glycemic excursion of individual animals was recorded for 30min. **(E)** Schematic of HEK-β_{GLP}. D-glucose activates P_{NFAT}-driven promoters by excitation-transcription coupling and triggers dose-dependent expression of secreted human glucagon-like peptide 1 (shGLP1). shGLP1 activates constitutively expressed GLP-1 receptor (GLP1R) via an autocrine loop and triggers insulin expression from P_{CRE}-driven promoters. *In vivo,* insulin expression by HEK-β_{GLP} cells may also be triggered following postprandial release of GLP-1 by intestinal cells. **(F)** Response of β-cell-mimetic implants to meals. Wild-type mice were implanted with 5x10⁶ microencapsulated Caᵥ1.3/pMX57-transgenic HEK-293 cells (-GLP1R) or Caᵥ1.3/pMX61/pMX258-transgenic HEK_{GLP1R} cells (+GLP1R) and received oral doses of 200µL H₂O, Coca-Cola® or sugared water (0.5M D-Glucose). Resulting blood SEAP levels were quantified after 48h. **(G)** Oral glucose tolerance test of wild-type (WT) and type-1 diabetic (T1D) mice. Mice received 5x10⁶ microencapsulated HEK-β, 1.1E7 or HEK-β_{GLP} cells or negative-control implants containing Caᵥ1.3/pMX115-transgenic HEK-293 cells (cntrl). After oral administration of sugared water (2g/kg D-glucose in H₂O), the glycemic excursions of individual animals were recorded for 6h. **(H)** Self-sufficient glycemic control by implants containing transgenic HEK-β and HEK-β_{GLP} cells. 5x10⁶ microencapsulated HEK-β or HEK-β_{GLP} cells were implanted into wild-type (WT) or type-1 diabetic (T1D) mice (1x10⁴ capsules/mice). Fasting glycemia of treated animals was recorded for 3 weeks. All data are shown as the mean ± SEM, statistics were performed using two-tailed t-test (n=8 mice). *P<0.05, **P<0.01, ***P<0.001 HEK-β vs. cntrl.

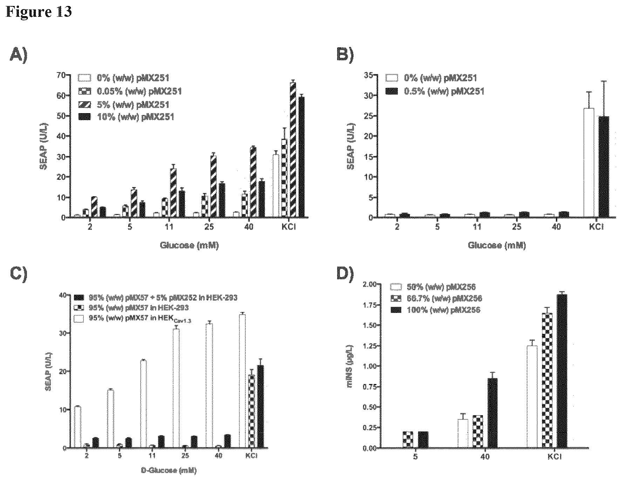

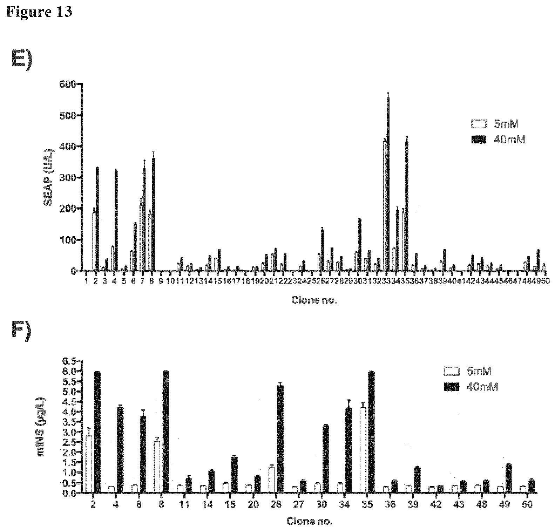

**Figure 13** shows construction and characterization of the stable HEK-β cell line. (A, B) Characterization of HEK_{MX252} stably expressing the Caᵥ1.3 α1D subunit. (A) 3x10⁶ HEK-293 cells were cotransfected with pMX252 (ITR-P_{hEF1α}-Cacna1d-pA:P_{RPBSA}-BFP-P2A-PuroR-pA-ITR; 9500ng) and pCMV-T7-SB100 (P_{hCMV}-SB100X-pA; 500ng), selected with 0.5µg/mL puromycin for two passages and 3x10⁵ cells of the surviving population (HEK_{MX252}) were then cotransfected with pMX57 (P_{NFAT3}-SEAP-pA; 1000ng) and different amounts of pMX251 (ITR-P_{hEF1α}-Cacna2d1-P2A-Cacnb3-pA:P_{RPBSA}-dTomato-P2A-BlastR-pA-ITR; 0-200ng filled to 1000ng with pcDNA3.1(+)). 24h after transfection and cultivation in low glucose medium (2mM), D-glucose or potassium chloride (KCl; 50mM) was added to the indicated final concentrations. SEAP levels were profiled in the culture supernatants 48h after addition of D-glucose. Data presented are mean ±SD, n≥5. (B) Control experiment of HEK-293 cells transfected with pMX57 (1000ng) and different amounts of pMX251 (0-10ng filled to 1000ng with pcDNA3.1(+)). Data presented are mean ±SD, n≥5. (C) Characterization of HEK_{Cav1.3} stably expressing the full Caᵥ1.3 channel componentry (Cacna1d/Cacnb3/Cacna2d1). 3x10⁶ HEK_{MX252} cells were cotransfected with pMX251 (9500ng) and pCMV-T7-SB100 (500ng), selected with 10µg/mL blasticidin for three passages and 3x10⁵ cells of the surviving population (HEK_{Cav1.3}) were then cotransfected with pMX57 (P_{NFAT3}-SEAP-pA; 1900ng) and pcDNA3.1(+) (100ng). HEK-293 cotransfected with either pMX57 alone (1900ng) or in combination with pMX252 (100ng) were used as negative controls. 24h after transfection and cultivation in low glucose medium (2mM), D-glucose or potassium chloride (KCl; 50mM) was added to the indicated final concentrations. SEAP expression levels were profiled in the culture supernatants 48h after the addition of D-glucose. Data presented are mean ±SD, n≥5. (D) Glucose- and depolarization-stimulated insulin expression in HEK_{Cav1.3}. 3x10⁵ HEK_{Cav1.3} cells were cotransfected with different amounts of pMX256 (ITR-P_{NFAT5}-SEAP-P2A-mINS-pA:P_{RPBSA}-EGFP-P2A-ZeoR-pA-ITR, 1000-2000ng filled to 2000ng with pcDNA3.1(+)) and cultivated in low glucose medium (2mM) for 12h before different concentrations of D-glucose and KCl were added. 48h after the addition of the control compounds, mINS levels were profiled in the culture supernatants. Data presented are mean ±SD, n≥3. (E, F) Clonal selection of HEK-β cells. (E) 3x10⁶ HEK_{Cav1.3} cells were cotransfected with pMX256 (9500ng) and pCMV-T7-SB100 (500ng), selected with 100µg/mL zeocin for three passages and 5% of the surviving population showing highest EGFP expression levels were subjected to FACS-mediated single-cell cloning. 50 expanded cell clones were profiled for glucose-stimulated SEAP expression by cultivating 5x10⁴ cells in high-glucose (40mM) or low-glucose medium (5mM) for 48h before SEAP levels were profiled in the culture supernatants. Data presented are mean ±SD, n=3. (F) The 20 clones showing highest glucose-stimulated SEAP inductions in (E) were profiled for glucose-stimulated insulin expression by cultivating 5x10⁴ cells in high-glucose (40mM) or low-glucose medium (5mM) for 48h before mINS levels were profiled in the culture supernatants. HEK-β (cell clone no. 4) was chosen for further analysis. Data presented are mean ±SD, n=3.

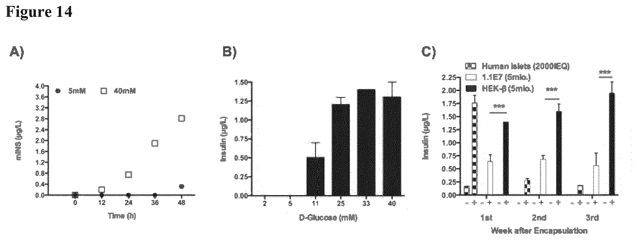

**Figure 14** shows characterization of the monoclonal HEK-β cell line. (A) 3x10⁴ HEK-β cells were cultivated in high-glucose (40mM) or low-glucose medium (5mM) for 48h and mINS levels were profiled in the culture supernatants every 12h after addition of D-glucose. Data presented are mean ±SD, n≥5. (B) 5x10⁴ HEK-β cells were cultivated in low-glucose medium (2mM) for 12h, before different concentrations of D-glucose were added and mINS levels were profiled in the culture supernatants after 24h. Data presented are mean ±SD, n≥5. (C) Reversible glucose-stimulated insulin secretion. Identical capsule batches used for implantation into mice (human islets, Fig. S13; HEK-β and 1.1E7, Fig. 6B) were also maintained in cell culture medium for 3 weeks and glucose-stimulated (-, 2.8mM; +, 30mM) insulin production was profiled for 24h once every week. Data presented are mean ±SD, n≥3.

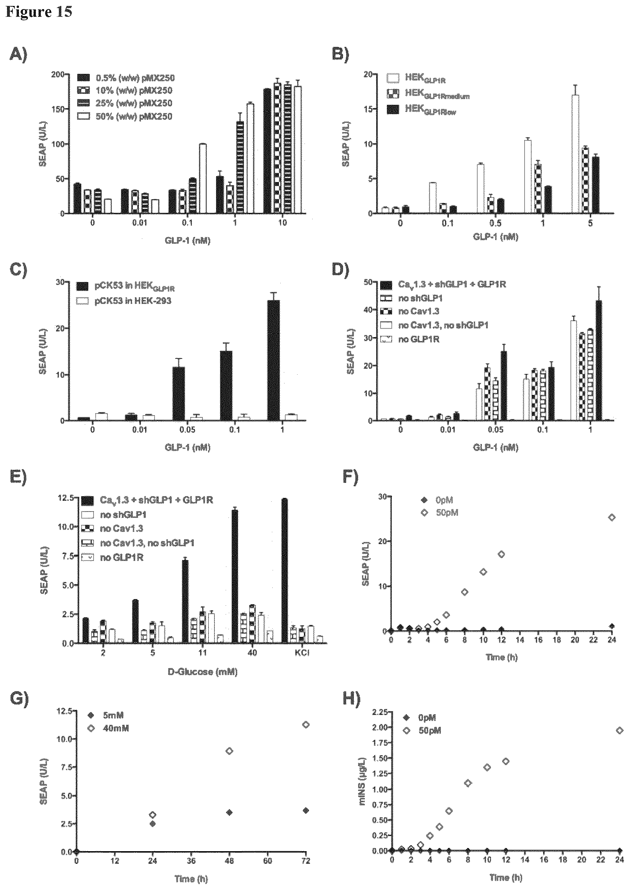

**Figure 15** shows engineering of HEK-β_{GLP}. (A) GLP-1 triggered SEAP expression in HEK-293 cells. 3x10⁵ HEK-293 cells were cotransfected with pCK53 (P_{CRE}-SEAP-pA; 200ng) and different amounts of pMX250 (ITR-P_{hEF1α}-GLP1R-pA:P_{RPBSA}-dTomato-P2A-PuroR-pA-ITR; 10-1000ng filled to 1800ng with pcDNA3.1(+)) before different concentrations of recombinant human GLP-1 was added. 24h after the addition of GLP-1, SEAP levels were profiled in the culture supernatants. Data presented are mean ±SD, n≥5. (B, C) Characterization of HEK_{GLP1R} stably expressing the human GLP-1 receptor (GLP1R). (B) 3x10⁶ HEK-293 cells were cotransfected with pMX250 (9500ng) and pCMV-T7-SB100 (500ng), selected with 1µg/mL puromycin for two passages and the surviving population was FACS-sorted into three populations with different red-fluorescence intensities (HEK_{GLP1R}, HEK_{GLP1Rmedium}, HEK_{GLP1Rlow}). Each population (1x10⁵ cells) was transfected with pCK53 (100ng filled to 2000ng with pcDNA3.1 (+)) before different concentrations of recombinant human GLP-1 were added. 24h after the addition of GLP-1, SEAP levels were profiled in the culture supernatants. Data presented are mean ±SD, n≥3. (C) HEK-293 cells transfected with pCK53 (100ng filled to 2000ng with pcDNA3.1(+)) were used as negative control. Data presented are mean ±SD, n≥5. (D, E) Validation of the HEK-β_{GLP} circuit. HEK_{GLP1R} was cotransfected with Caᵥ1.3 (pCaV1.3/pCaVb3/pCaVα2δ1; 333ng each), pMX61 (P_{NFAT3}-shGLP1-pA; 1000ng) and pCK53 (P_{CRE}-SEAP-pA; 250ng) and cultivated in low-glucose medium (2mM) for 12h before different concentration of (D) recombinant human GLP-1 or (E) D-glucose were added. HEK_{GLP1R} cotransfected with pMX61/pCK53 or Caᵥ1.3/pCK53 and HEK-293 cotreansfected with Caᵥ1.3/pMX61/pCK53 were used as negative controls. (D) 24h after addition of GLP-1 and (E) 72h after addition of D-Glucose, SEAP levels were profiled in the culture supernatants. Data presented are mean ± SD, n≥5. (F, G) SEAP expression kinetics. HEK_{GLP1R} cells were cotransfected with Caᵥ1.3 (pCaV1.3/pCaVb3/pCaVα2δ1; 333ng each), pMX61 (P_{NFAT3}-shGLP1-pA; 1000ng) and pCK53 (P_{CRE}-SEAP-pA; 250ng), cultivated in low-glucose medium (2mM) for 12h before different concentrations of (F) recombinant human GLP-1 or (G) D-glucose were added. SEAP levels were profiled in the culture supernatants (F) 24h or (G) 72h after addition of the respective compounds. Data presented are mean ±SD, n≥5. (H) mINS expression kinetics of HEK-□_{GLP}. HEK_{GLP1R} cells were cotransfected with Caᵥ1.3 (pCaV1.3/pCaVb3/pCaVα2δ1; 333ng each), pMX61 (P_{NFAT3}-shGLP1-pA; 1000ng) and pDA145 (P_{CRE}-mINS-pA; 1000ng), cultivated in low-glucose medium (2mM) for 12h before different concentrations of recombinant human GLP-1 were added. mINS levels were profiled in the culture supernatants for 24h after addition of control compounds. Data presented are mean ±SD, n≥5.

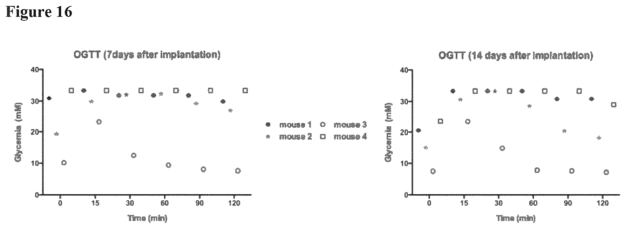

**Figure 16** shows oral glucose tolerance test (OGTT) of type-1 diabetic mice treated with encapsulated human islets. 2000 IEQs of human islets were microencapsulated in alginate-poly-(L-lysine)-alginate beads and injected into each of four type-1 diabetic mice. 7 and 14 days after implantation the animals received oral D-glucose (2g/kg) and their glycemic excursions were recorded over 2h.

### DETAILED DESCRIPTION

So that the invention may be more readily understood, certain terms are first defined. Unless otherwise defined within the specification, all technical and scientific terms used herein have their art-recognized meaning. Although similar or equivalent methods and materials to those described herein can be used in the practice or testing of the invention, suitable methods and materials are described below. All publications, patent applications, patents, and other references mentioned herein are incorporated by reference in their entirety. In case of conflict, the present specification, including definitions, will prevail. The materials, methods, and examples are illustrative only and not intended to be limiting. The terms "comprising", "having", and "including" are to be construed as open-ended terms (i.e., meaning "including, but not limited to,") unless otherwise noted.

As used herein, the term "beta-cell" or "β-cell" refers to a cell type found in the pancreas, in particular in the mammalian, more particular in the human pancreatic islets. Beta cells are the primary producers of insulin.

As used herein, the term "recombinant cell" refers to cells, preferably mammalian cells , more preferably human cells, which have been artificially manipulated to express genes which are introduced to the mammalian cells by e.g. transfection or transformation using nucleic acid constructs, such as e.g. expression vectors in which those genes are incorporated.

As used herein, the term "transfection" or "transfected" refers to the introduction of a nucleic acid e.g. the introduction of a nucleic acid construct as described herein into a cell. In general the nucleic acid is a DNA sequence, in particular a vector or a plasmid carrying a gene of interest like a gene coding for a therapeutic protein as described herein, operably linked to a suitable promoter as described herein. Transfection methods which can be used are e.g. those using carrier molecules like cationic lipids such as DOTAP (Roche), TransFast (Promega), and Lipofectamine (Invitrogene), or polyethylenimine (PEI), calcium phosphate and DEAE dextran. Other useful transfection techniques include electroporation, bombardment with nucleic-acid-coated carrier particles (gene gun), microinjection and using of viral vectors.

As used herein, the term "transiently transfected" of "transient transfection" refer to the transient, i.e. non-permanent expression of the gene of interest due to the episomal nature of the introduced nucleic acid. Episomal nucleic acids, including DNA (plasmids or vectors), is degraded by the cells after two to seven days, and hence the expression of the gene of interest ceases then.

As used herein, the term "stably transfected" or "stable transfection" refers to the permanent expression of a gene of interest due to the integration of the transfected DNA into the genome of the cell. Most if not all cells have the potential to incorporate episomal DNA into their genome albeit at a very low rate. However, sophisticated selection strategies are employed to expand those cells that have integrated the transfected DNA. For that a nucleic acid construct to be stably integrated, a vector carrying the DNA to be transfected normally contains at least one gene that encodes for a selection marker such as e.g. a puromycin-resistance gene.

As used herein, the term "nucleic acid construct" refers to a nucleic acid, preferably to a recombinant nucleic acid construct, i.e. a genetically engineered nucleic acid construct which includes the nucleic acid of a gene and at least one promoter for directing transcription of the nucleic acid in a host cell. Nucleic acid constructs of the present invention are preferably suitable for mammalian cell expression. The nucleic acid construct (also referred to herein as an "expression vector") may include additional sequences that render the construct e.g. the vector, suitable for replication and integration in eukaryotes (e.g. shuttle vectors). In addition, a typical nucleic acid construct such as e.g. a cloning vector may also contain transcription and translation initiation sequences, transcription and translation terminators, and a polyadenylation signal.

As used herein, the term "promoter" refers to a regulatory DNA sequence generally located upstream of a gene that mediates the initiation of transcription by directing RNA polymerase to bind to DNA and initiating RNA synthesis. The term "Pₘᵢₙ" as used herein refers to a minimal promoter, preferably to the promoter as shown in SEQ ID NO: 33. A minimal promoter usually does not contain an enhancer i.e. do not comprise enhancer elements and is not a constitutive promoter. Preferably a minimal promoter shows no or only minimal transcriptional activity in the absence of transcription factors.

As used herein, the term "enhancer" as used herein refers to a nucleotide sequence that acts to potentiate the transcription of genes independent of the identity of the gene, the position of the sequence in relation to the gene, or the orientation of the sequence.

As used herein, the terms "functionally linked" and "operably linked" are used interchangeably and refer to a functional relationship between two or more DNA segments, in particular gene sequences to be expressed and those sequences controlling their expression. For example, a promoter and/or enhancer sequence, including any combination of cis-acting transcriptional control elements is operably linked to a coding sequence if it stimulates or modulates the transcription of the coding sequence in an appropriate host cell or other expression system. Promoter regulatory sequences that are operably linked to the transcribed gene sequence are physically contiguous to the transcribed sequence.

As used herein, the term "promoter which is responsive to carbohydrate metabolism of a cell" refers to a promoter which is activated or repressed for transcription in response to the presence or absence of a product of the carbohydrate metabolism of a cell. A product of the carbohydrate metabolism of a cell can be a metabolic product of the cellular pathway of carbohydrate transformation or carbohydrate degradation e.g a product of glycolysis such as intracellular ATP production or can be a cellular response to the metabolism of a carbohydrate in the cell e.g. production of intracellular ATP lead to the closure of K_{ATP} channels which causes membrane depolarization as cellular response.

As used herein, the term "carbohydrate metabolism of a cell" refers to the various biochemical processes responsible for the formation, breakdown and interconversion of carbohydrates in living organisms. Oligosaccharides and/or polysaccharides are typically cleaved into smaller monosaccharides by enzymes called glycoside hydrolases. The monosaccharide units then enter the cellular pathway of carbohydrate transformation or carbohydrate degradation, i.e. the cellular pathway of glucose transformation or degradation such as glycolysis.

As used herein, the term "cellular component for sensing extracellular carbohydrates" refers to a cellular component such as e.g. a transporter of carbohydrates like e.g. a transporter of glucose or a glucose linked transporter, a receptor involved in the regulation of carbohydrate homeostasis e.g. glucose homeostasis, or a membrane protein like potassium channels, calcium channels or sodium channels which is capable of sensing extracellular carbohydrates such as glucose by activating a gene, preferably a gene coding for a therapeutic protein whose expression level correlates with the extracellular carbohydrate levels. Usually the cellular component for sensing extracellular carbohydrates of the recombinant cell of the present invention is capable of sensing extracellular carbohydrates such as glucose by activating a gene, preferably a gene coding for a therapeutic protein whose expression level correlates with the extracellular carbohydrate levels via a promoter which is responsive to carbohydrate metabolism of said recombinant cell, wherein the promoter is operably linked to a gene coding for the therapeutic protein. Preferably the cellular component for sensing extracellular carbohydrates is of mammalian, more preferably human origin.

As used herein, the term "carbohydrate" or "saccharide" are interchangeably and equivalently used within this context refer to a biological molecule consisting of carbon (C), hydrogen (H) and oxygen (O) atoms, usually with a hydrogen-oxygen atom ratio of 2:1 (as in water); in other words, with the empirical formula Cₘ(H₂O)ₙ (where m could be different from n). Carbohydrates include monosaccharides, disaccharides, oligosaccharides, and polysaccharides, preferably monosaccharides and disaccharides, more preferably monosaccharides, most preferably monosaccharides selected from the group consisting of glucose, galactose, fructose and xylose and epimeric forms thereof like mannose, in particular glucose and mannose. The term "glucose" in its broadest sense relates to glucose and its epimeric forms like mannose. Preferably the term "glucose" relates to glucose (D-Glucose, (2R,3S,4R,5R)-2,3,4,5,6-Pentahydroxyhexanal).

As used herein, the term "membrane protein" refers to a protein molecule that is attached to or associated with the membrane of a cell or organelle. The membrane protein is preferably a membrane protein of a human cell or organelle.

As used herein, the term "fragment of a membrane protein" refers to a region of a membrane protein that is shorter in length as compared with the full-length membrane protein. It is however, a requirement of the present invention that any fragment of a membrane protein used as part retain the activity of the full-length membrane protein.

As used herein, the term "subunit of a membrane protein" or "monomer of a membrane protein" are interchangeably and equivalently used within this context and refers to a separate polypeptide chain that makes a membrane protein which is made up of two or more polypeptide chains joined together. In a membrane protein molecule composed of more than one subunit, each subunit can form a stable folded structure by itself. The amino acid sequences of subunits of a protein can be identical, similar, or completely different.

As used herein, the term "physiological effect of membrane depolarization" refers to a physiological effect due to depolarization of a cell's membrane such as e.g. the variation in intra-cellular cation, e.g. intra-cellular calcium, intra-cellular sodium or intra-cellular potassium concentration, in particular intra-cellular calcium concentration within a cell which has been caused by physiological activities, in particular by the carbohydrate metabolism of said cell.

As used herein, the term "calcium-responsive promoter" refers to a promoter which is activated or repressed for transcription in response to the presence or absence of calcium in the cell.

As used herein, the term "expression system" refers to a set of transgenic genetic elements within a cell as well as proteins encoded by such genetic elements.

The term "G-protein coupled receptors" as used herein refers to a family of transmembrane receptors, that sense molecules outside the cell and activate responses inside the cell by coupling with specific intracellular signaling pathways via G proteins. Preferred G-protein coupled receptors are GPR1, TAS1R2, TAS1R3, GLP1R or anyone of their orthologues. Most preferred G-protein coupled receptors are GPR1 (GenBank: CAA98593.1), TAS1R2 (UniProtKB/Swiss-Prot: Q8TE23.2), TAS1R3 (UniProtKB/Swiss-Prot: Q7RTX0.2) and GLP1R (UniProtKB/Swiss-Prot: P43220.2).

The term "glucoincretin receptor" as used herein refers to glucoincretin receptors such as gastric inhibitory polypeptide receptor (GIPR) and glucagon-like peptide-1 receptor (GLP1R), preferably to human glucoincretin receptors such as human GIPR e.g. human GIPR (UniProtKB: P48546) and/or human GLP1R e.g. human GLP1R (UniProtKB/Swiss-Prot: P43220.2) .

The term "SLC2A family glucose transporters" (also known as GLUT) as used herein refers to a family of transmembrane proteins that catalyze the entry of carbohydrates into mammalian cells. Preferred SLC2A family glucose transporters are GLUT1 (SLC2A1), GLUT2 (SLC2A2), GLUT3 (SLC2A3), GLUT4 (SLC2A4), GLUT5 (SLC2A5), GLUT6 (SLC2A6), GLUT7 (SLC2A7), GLUT8 (SLC2A8), GLUT9 (SLC2A9), GLUT10 (SLC2A10), GLUT11 (SLC2A11), GLUT12 (SLC2A12) and GLUT13 (SLC2A13) or anyone of their orthologues. Most preferred SLC2A family glucose transporters are human GLUT1, human GLUT2 and human GLUT3.

The term "SLC5A family sodium-glucose linked transporters" (also known as SGLT) as used herein refers to a family of transmembrane proteins that mediate sodium-dependent co-transport of carbohydrates across the plasma membrane of mammalian cells. Preferred SLC5A family sodium-glucose linked transporters are SGLT1 (SLC5A1), SGLT2 (SLC5A2) and SGLT3 (SLC5A3) or anyone of their orthologues. Most preferred SLC5A family sodium-glucose linked transporters are human SGLT1 and human SGLT3.

The term "potassium channels" as used herein refers to a family of pore-forming transmembrane proteins that facilitate the transport of potassium ions across the cell plasma membrane. Preferred potassium channels are ATP-sensitive potassium channels (K_{ATP}), calcium-activated potassium channels (BK_{Ca}), inward rectifier potassium channels (Kir) and voltage-dependent potassium channels (Kᵥ). Most preferred potassium channels are human ATP-sensitive potassium channels (K_{ATP}), human calcium-activated potassium channels (BK_{Ca}), human inward rectifier potassium channels (Kir) and human voltage-dependent potassium channels (Kᵥ).

The term "calcium channels" as used herein refers to a family of pore-forming transmembrane proteins that facilitate the transport of calcium ions across the cell plasma membrane. Preferred calcium channels are voltage-gated calcium channels (VGCC), N-methyl-D-aspartate type of Glutamate (NMDA) receptors, Ca²⁺ release-activated Ca²⁺ current (CRAC) channels and transient receptor potential channels (TRPCs). Most preferred calcium channels are human voltage-gated calcium channels (VGCC), human N-methyl-D-aspartate type of Glutamate (NMDA) receptors, human Ca²⁺ release-activated Ca²⁺ current (CRAC) channels and human transient receptor potential channels (TRPCs).

The term "sodium channels" as used herein refers to a family of pore-forming transmembrane proteins that facilitate the transport of sodium ions across the cell plasma membrane. Preferred sodium channels are voltage gated-sodium channels, most preferably human voltage gated-sodium channels

As used herein, the term "voltage-gated calcium channel" (VDCC) refers to a group of voltage-gated ion channels, preferably human voltage-gated calcium channels, found in the membrane of excitable cells whose permeability to the calcium ion Ca²⁺ correlates with the membrane potential. Voltage-dependent calcium channels are formed as a complex of several different subunits. Subunits known are the pore-forming Caᵥα1, the intracellular Caᵥβ, the transmembrane Caᵥγ, and a disulfide-linked dimer Caᵥα2δ. The α1 subunit is the primary subunit necessary for channel functioning in the VDCC, and consists of the characteristic four homologous I-IV domains containing six transmembrane α-helices each forms the ion conducting pore while the associated subunits have several functions including modulation of gating. Voltage-gated calcium channels are functionalized by their α1 subunit, which sets the activation threshold of the entire channel. Non-limiting examples of α1 subunits are Caᵥ1.1, Caᵥ1.2, Caᵥ1.3, Caᵥ1.4, Caᵥ2.1, Caᵥ2.2, Caᵥ2.3, Caᵥ3.1, Caᵥ3.2 and Caᵥ3.3.

As used herein, the term "therapeutic protein" refers to a protein which is therapeutically applicable i.e. a therapeutic protein is any protein or polypeptide that can be expressed to provide a therapeutic effect, in particular a protein that can be expressed to provide a therapeutic effect with respect to metabolic diseases.

As used herein, the term "orthologues " with respect to a protein e.g. a receptor or channel refers to one of two or more homologous gene sequences found in different species.

As used herein, the term "insulin" refers to the protein hormone produced by beta cells in the pancreas which decreases blood glucose concentrations and is therefore involved in the regulation of blood sugar levels. One international unit of insulin (1 IU) is defined as the "biological equivalent" of 34.7 µg pure crystalline insulin, which corresponds to the amount required to reduce the concentration of blood glucose in a fasting rabbit to 45 mg/dl (2.5 mmol/L). Insulin is produced as a proinsulin precursor consisting of the B and A chains of insulin linked together via a connecting C-peptide. Insulin itself is comprised of only the B and A chains. Human insulin is encoded by the INS gene corresponding to GenBank Accession No: NM-000207.2. The term "insulin" or "insulin molecule" is a generic term that designates the 51 amino acid heterodimer comprising the A-chain peptide and the B-chain peptide, wherein the cysteine residues a positions 6 and 11 of the A chain are linked in a disulfide bond, the cysteine residues at position 7 of the A chain and position 7 of the B chain are linked in a disulfide bond, and the cysteine residues at position 20 of the A chain and 19 of the B chain are linked in a disulfide bond. The term "insulin" means the active principle of the pancreas that affects the metabolism of carbohydrates in the animal body and which is of value in the treatment of diabetes mellitus. The term includes synthetic and biotechnologically derived products that are the same as, or similar to, naturally occurring insulins in structure, use, and intended effect and are of value in the treatment of diabetes mellitus.

The term "insulin analogue" as used herein includes any heterodimer analogue or single-chain analogue that comprises one or more modification(s) of the native A-chain peptide and/or B-chain peptide. Modifications include but are not limited to substituting an amino acid for the native amino acid at a position selected from A4, A5, A8, A9, A10, A12, A13, A14, A15, A16, A17, A18, A19, A21, B1, B2, B3, B4, B5, B9, B10, B13, B14, B15, B16, B17, B18, B20, B21, B22, B23, B26, B27, B28, B29, and B30; deleting any or all of positions B1-4 and B26-30; or conjugating directly or by a polymeric or non-polymeric linker one or more acyl, polyethylglycine (PEG), or saccharide moiety (moieties); or any combination thereof. Examples of insulin analogues include but are not limited to the heterodimer and single-chain analogues disclosed in published international application WO20100080606, WO2009099763, and WO2010080609, the disclosures of which are incorporated herein by reference. Examples of single-chain insulin analogues also include but are not limited to those disclosed in published International Applications WO9634882, WO95516708, WO2005054291, WO2006097521, WO2007104734, WO2007104736, WO2007104737, WO2007104738, WO2007096332, WO2009132129; U.S. Pat. Nos. 5,304,473 and 6,630,348; and Kristensen et al., Biochem. J. 305: 981-986 (1995), the disclosures of which are each incorporated herein by reference. The term "insulin analogues" further includes single-chain and heterodimer polypeptide molecules that have little or no detectable activity at the insulin receptor but which have been modified to include one or more amino acid modifications or substitutions to have an activity at the insulin receptor that has at least 1%, 10%, 50%, 75%, or 90% of the activity at the insulin receptor as compared to native insulin and which further includes at least one N-linked glycosylation site. In particular aspects, the insulin analogue is a partial agonist that has from 2× to 100× less activity at the insulin receptor as does native insulin. In other aspects, the insulin analogue has enhanced activity at the insulin receptor, for example, the IGF<B16B17 >derivative peptides disclosed in published international application WO2010080607 (which is incorporated herein by reference). These insulin analogues, which have reduced activity at the insulin growth hormone receptor and enhanced activity at the insulin receptor, include both heterodimers and single-chain analogues.

As used herein, the term "autologous" or "endogenous" refers to any material that is present in a cell or an organism which is native to said recombinant cell or organism.

The term "stem cell" as used herein refers to undifferentiated biological cells that can differentiate into specialized cells and which is capable of proliferation to produce more stem cells.

As used herein, the term "somatic cell" refers to any cell forming the body of an organism, as opposed to germline cells or undifferentiated stem cells.

The terms "individual," "subject" or "patient" are used herein interchangeably. In certain embodiments, the subject is a mammal. Mammals include, but are not limited to primates (including human and non-human primates). In a preferred embodiment, the subject is a human.

The term "about" as used herein refers to +/- 10% of a given measurement.

In a first aspect, the present invention provides a recombinant cell comprising a nucleic acid construct comprising a promoter which is responsive to carbohydrate metabolism of said cell, wherein the promoter is operably linked to a gene coding for a therapeutic protein, wherein the recombinant cell further comprises a nucleic acid construct coding for a cellular component for sensing extracellular carbohydrates, wherein the cellular component for sensing extracellular carbohydrates is a voltage-gated calcium channel and wherein the promoter which is responsive to carbohydrate metabolism is responsive to a physiological effect of membrane depolarization caused by the carbohydrate metabolism of said recombinant cell. Preferably, the promoter which is responsive to carbohydrate metabolism is responsive to glucose metabolism.

Preferably the physiological effect of membrane depolarization caused by the carbohydrates metabolism of the cell is extracellular calcium influx. Thus in a preferred embodiment the promoter which is responsive to carbohydrate metabolism is responsive to a physiological effect of membrane depolarization caused by the carbohydrate metabolism of said recombinant cell, wherein the physiological effect of membrane depolarization caused by the carbohydrates metabolism of said cell is extracellular calcium influx. Thus in a further preferred embodiment the promoter which is responsive to carbohydrate metabolism is responsive to extracellular calcium influx.

In one embodiment the promoter which is responsive to carbohydrate metabolism is a calcium-responsive promoter, preferably a calcium-responsive promoter comprising nucleic acid sequences bound by transcription factors of the NFAT family, the NFkB family, the AP-1 family, and/or the CREB family and/or cFOS, more preferably a calcium-responsive promoter comprising nucleic acid sequences bound by transcription factors of the NFAT family.

The NFAT family is a family of transcription factors shown to be important in immune response. One or more members of the NFAT family is expressed in most cells of the immune system. NFAT is also involved in the development of cardiac, skeletal muscle, and nervous systems. The NFAT family comprises the NFAT1, NFAT2, NFAT3, NFAT4, and NFAT5 proteins that specifically bind their cognate promoters. Preferred promoters that contain NFAT-binding sites are synthetic or natural promoters containing one or multiple 5'-GGAAA-3' consensus sites, more preferred are mammalian cytokine promoters e.g. mammalian cytokine promoters selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPARγ promoter. Most preferred is the murine interleukin (IL)-4 promoter. Thus in one embodiment the promoter which is responsive to carbohydrate metabolism of the recombinant cell is a synthetic or natural promoter that contains NFAT-binding sites containing one or multiple 5'-GGAAA-3' consensus sites, more preferably a mammalian cytokine promoter e.g. a mammalian cytokine promoter selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPAKγ promoter and most preferrably the murine interleukin (IL)-4 promoter. The murine interleukin (IL)-4 promoter is described e.g. in Rooney JW et al., EMBO J 13, 625-633 (1994).

The NFkB family is as family of protein complexes that act as transcription factors controlling the transcription of DNA, cytokine production and cell survival. The NFkB family comprises NF-κB1, NF-κB2, RelA, RelB and c-Rel. Preferred members of the NFkB family are NF-κB1 and NF-κB2, more preferably human NF-κB1 and NF-κB2.

The AP-1 family is as family of transcription factors that regulates gene expression in response to a variety of stimuli, including cytokines, growth factors, stress and infections. AP-1 is a heterodimer composed of proteins belonging to the c-Fos, c-Jun, ATF and JDP families. Preferred members of the AP-1 family are c-Fos and c-Jun. cFOS is a human proto-oncogene that belongs to the FOS family of transcription factors and encodes a 62 kDa protein, which forms a heterodimer with c-jun (part of Jun family of transcription factors), resulting in the formation of AP-1 (Activator Protein-1) complex which binds DNA at AP-1 specific sites at the promoter and enhancer regions of target genes and converts extracellular signals into changes of gene expression.

The CREB (cAMP-responsive element-binding protein) family is as family of transcription factors that binds cAMP response elements (CRE) containing the highly conserved nucleotide sequence, 5'-TGACGTCA-3', thereby modulating target gene expression from CRE-containing promoters (P_{CRE}). Preferred members of the CREB family are CREB1 and ATF4, more preferably human CREB1 and ATF4.

In one embodiment the promoter which is responsive to carbohydrate metabolism is a calcium-responsive promoter, wherein the calcium responsive promoter is a synthetic promoter consisting of one or multiple tandem repeats of binding sites of transcription factors selected from the group consisting of the NFAT family, the NFkB family, the AP-1 family, and/or the CREB family and/or cFOS operably linked to one or multiple promoters wherein the one or multiple promoters do not comprise enhancer elements, i.e. is not a constitutive promoter.

In one embodiment the promoter which is responsive to carbohydrate metabolism is a calcium-responsive promoter, wherein the calcium responsive promoter is a synthetic promoter consisting of one or multiple tandem repeats of binding sites of NFAT operably linked to one or multiple promoters wherein the one or multiple promoters do not comprise enhancer elements, wherein the sequence of the NFAT-binding sites of the calcium responsive promoter contains one or multiple tandem repeats of the binding site of a mammalian cytokine promoter selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPARγ promoter and most preferrably the murine interleukin (IL)-4 promoter in particular one or multiple tandem repeats of the binding site of the murine interleukin (IL)-4 promoter as shown in SEQ ID NO: 34, preferably 3 to 9 tandem repeats, more preferably 3, 5, 7 or 9 tandem repeats. In one embodiment the promoter which is responsive to carbohydrate metabolism is a calcium-responsive promoter, wherein the calcium responsive promoter is a CRE-containing synthetic mammalian promoter preferably the P_{CRE} promoter as described e.g. in Ausländer D et al., Mol Cell 55, 397-408 (2014), more preferably the P_{CRE} promoter comprising SEQ ID NO: 60.

In one embodiment the promoter which is responsive to carbohydrate metabolism comprises SEQ ID NO: 4

In one embodiment the promoter which is responsive to carbohydrate metabolism comprises SEQ ID NO: 5

In one embodiment the promoter which is responsive to carbohydrate metabolism comprises SEQ ID NO:6

In one embodiment the promoter which is responsive to carbohydrate metabolism comprises SEQ ID NO: 39

In one embodiment the recombinant cell may comprise two or more nucleic acid constructs comprising a promoter which is responsive to carbohydrate metabolism of said recombinant cell, wherein the promoter is operably linked to a gene coding for a therapeutic protein. Preferably each nucleic acid construct comprises a gene coding for a different therapeutic protein. Thus in a preferred embodiment, the recombinant cell comprises two or more nucleic acid constructs, wherein the first nucleic acid construct comprises a promoter which is responsive to carbohydrate metabolism of said recombinant cell, wherein the promoter is operably linked to a gene coding for a first therapeutic protein and wherein the second or a further nucleic acid construct comprises a promoter which is responsive to carbohydrate metabolism of said recombinant cell, wherein the promoter is operably linked to a gene coding for a second or a further therapeutic protein, wherein the first therapeutic protein is different from the second or further therapeutic protein.

In one embodiment the recombinant cell comprises a nucleic acid construct comprising a first promoter which is responsive to carbohydrate metabolism of said recombinant cell, wherein the first promoter is operably linked to a gene coding for a first therapeutic protein, and further comprises a nucleic acid construct comprising a second promoter which is responsive to carbohydrate metabolism of said recombinant cell, wherein the promoter is operably linked to a gene coding for a second therapeutic protein, wherein the first therapeutic protein is different from the second therapeutic protein, and wherein the first promoter is different from or identical to the second promoter, preferably the first promoter is different from the second promoter.

In one embodiment the recombinant cell comprises a nucleic acid construct comprising a first promoter which is responsive to carbohydrate metabolism of said recombinant cell, wherein the first promoter is operably linked to a gene coding for a first therapeutic protein, and further comprises a nucleic acid construct comprising a second promoter which is responsive to carbohydrate metabolism of said recombinant cell, wherein the promoter is operably linked to a gene coding for a second therapeutic protein, wherein the first therapeutic protein is different from the second therapeutic protein, and wherein the first promoter is different from or identical to the second promoter, preferably the first promoter is different from the second promoter, wherein the recombinant cell further comprises a nucleic acid construct coding for a cellular component for sensing extracellular carbohydrates.

In one embodiment the recombinant cell further comprises a nucleic acid construct coding for a glucoincretin receptor. The glucoincretin receptor is preferably a gastric inhibitory polypeptide receptor (GIPR) or a glucagon-like peptide-1 receptor (GLP1R), more preferably a human GIPR such as GIPR (UniProtKB: P48546) and/or a human GLP1R such as GLP1R (UniProtKB/Swiss-Prot: P43220.2), most preferably a human GLPIR, in particular GLP1R (UniProtKB/Swiss-Prot: P43220.2). In a preferred embodiment the recombinant cell further comprises a nucleic acid construct coding for a constitutively expressed glucoincretin receptor.

In one embodiment the recombinant cell comprises a nucleic acid construct comprising a first promoter which is responsive to carbohydrate metabolism of said recombinant cell, wherein the first promoter is operably linked to a gene coding for a first therapeutic protein, and further comprises a nucleic acid construct comprising a second promoter which is responsive to carbohydrate metabolism of said recombinant cell, wherein the promoter is operably linked to a gene coding for a second therapeutic protein, wherein the first therapeutic protein is different from the second therapeutic protein, and wherein the first promoter is different from or identical to the second promoter, preferably the first promoter is different from the second promoter, wherein the recombinant cell further comprises a nucleic acid construct coding for a glucoincretin receptor.

In one embodiment the recombinant cell comprises a nucleic acid construct comprising a promoter which is responsive to carbohydrate metabolism of said recombinant cell, wherein the promoter is operably linked to a gene coding for a therapeutic protein and further comprises a nucleic acid construct coding for a cellular component for sensing extracellular carbohydrates and a nucleic acid construct coding for a glucoincretin receptor.

In one embodiment the recombinant cell comprises a nucleic acid construct comprising a first promoter which is responsive to carbohydrate metabolism of said recombinant cell, wherein the first promoter is operably linked to a gene coding for a first therapeutic protein, and further comprises a nucleic acid construct comprising a second promoter which is responsive to carbohydrate metabolism of said recombinant cell, wherein the promoter is operably linked to a gene coding for a second therapeutic protein, wherein the first therapeutic protein is different from the second therapeutic protein, and wherein the first promoter is different from or identical to the second promoter, preferably the first promoter is different from the second promoter, wherein the recombinant cell further comprises a nucleic acid construct coding for a cellular component for sensing extracellular carbohydrates and a nucleic acid construct coding for a glucoincretin receptor.

In one embodiment the recombinant cell further comprises a nucleic acid construct coding for a cellular component for sensing extracellular carbohydrates e.g. extracellular glucose, wherein the cellular component for sensing extracellular carbohydrates e.g. extracellular glucose is a voltage-gated calcium channel, usually a voltage-gated calcium channel selected from the group consisting of Caᵥ1.1, Caᵥ1.2, Caᵥ1.3, Caᵥ1.4, Caᵥ2.1, Caᵥ2.2, Caᵥ2.3, Caᵥ3.1, Caᵥ3.2 and Caᵥ3.3, more preferably Caᵥ1.2, Caᵥ1.3, Caᵥ2.2, and most preferably Caᵥ1.3. In a preferred embodiment the cellular component for sensing extracellular carbohydrates e.g. extracellular glucose is a combination of subunits of a voltage-gated calcium channel, more preferably a combination of a β subunit selected from the group consisting of Caᵥβ₁ (CACNB1), Caᵥβ₂ (CACNB2), Caᵥβ₃ (CACNB3) and Caᵥβ₄ (CACNB4), a α2δ subunit selected from the group consisting of Caᵥα2δ₁ (CACNA2D1), Caᵥα2δ₂ (CACNA2D2), Caᵥα2δ₃ (CACNA2D3) and Caᵥα2δ₄ (CACNA2D4) and a α1 subunit selected from the group consisting of Caᵥ1.1 (CACNA1S), Caᵥ1.2 (CACNA1C), Caᵥ1.3 (CACNA1D), Caᵥ1.4 (CACNA1F), Caᵥ2.1 (CACNA1A), Caᵥ2.2 (CACNA1B), Caᵥ2.3 (CACNA1E), Caᵥ3.1 (CACNA1G), Caᵥ3.2 (CACNA1H) and Caᵥ3.3 (CACNA1I), even more preferably a α1 subunit of Caᵥ1.2 (CACNA1C), Caᵥ1.3 (CACNAID), Caᵥ2.2 (CACNA1B) combined with Caᵥβ₃ (CACNB3) and Caᵥα2δ₁ (CACNA2D1), and most preferably a α1 subunit of Caᵥ1.3 (CACNA1D) combined with Caᵥβ₃ (CACNB3) and Caᵥα2δ₁ (CACNA2D1).

In one embodiment the recombinant cell is a cell which express an autologous cellular component (i.e. a cell which autologously express) a cellular component for sensing extracellular carbohydrates, wherein the autologous cellular component for sensing extracellular carbohydrates is a membrane protein selected from the group consisting of G-protein coupled receptors, the SLC2A family glucose transporters, the SLC5A family sodium-glucose linked transporters, potassium channels, calcium channels and sodium channels, in particular calcium channels, more particular voltage-gated calcium channels. In a preferred embodiment, the recombinant cell autologously express a calcium channel, preferably a voltage-gated calcium channel, in particular a voltage-gated calcium channel selected from the group consisting of Caᵥ1.1, Caᵥ1.2, Caᵥ1.3, Caᵥ1.4, Caᵥ2.1, Caᵥ2.2, Caᵥ2.3, Caᵥ3.1, Caᵥ3.2 and Caᵥ3.3, more preferably Caᵥ1.2, Caᵥ1.3, Caᵥ2.2, and most preferably Caᵥ1.3. In a preferred embodiment the cellular component for sensing extracellular carbohydrates e.g. extracellular glucose is a combination of subunits of a voltage-gated calcium channel, more preferably a combination of a β subunit selected from the group consisting of Caᵥβ₁ (CACNB1), Caᵥβ₂ (CACNB2), Caᵥβ₃ (CACNB3) and Caᵥβ₄ (CACNB4), a α2δ subunit selected from the group consisting of Caᵥα2δ₁, (CACNA2D1), Caᵥα2δ₂ (CACNA2D2), Caᵥα2δ₃ (CACNA2D3) and Caᵥα2δ₄ (CACNA2D4) and a α1 subunit selected from the group consisting of Caᵥ1.1 (CACNA1S), Caᵥ1.2 (CACNA1C), Caᵥ1.3 (CACNAID), Caᵥ1.4 (CACNA1F), Caᵥ2.1 (CACNA1A), Caᵥ2.2 (CACNA1B), Caᵥ2.3 (CACNAIE), Caᵥ3.1 (CACNA1G), Caᵥ3.2 (CACNA1H) and Caᵥ3.3 (CACNA1I), even more preferably a α1 subunit of Caᵥ1.2 (CACNA1C), Caᵥ1.3 (CACNA1D), Caᵥ2.2 (CACNA1B) combined with Caᵥβ₃ (CACNB3) and Caᵥα2δ₁ (CACNA2D1), and most preferably a α1 subunit of Caᵥ1.3 (CACNAID) combined with Caᵥβ₃ (CACNB3) and Caᵥα2δ₁ (CACNA2D1).

Usually the cellular component for sensing extracellular carbohydrates of the recombinant cell of the present invention activates the promoter which is responsive to carbohydrate metabolism comprised by said recombinant cell e.g. the cellular component for sensing extracellular carbohydrates of the recombinant cell of the present invention activates the promoter which is responsive to carbohydrate metabolism comprised by said recombinant cell, wherein the gene coding for a therapeutic protein to which the promoter is operably linked is expressed, wherein the expression level of said gene correlates with the levels of extracellular carbohydrates. In a particular embodiment the cellular component for sensing extracellular carbohydrates of the recombinant cell of the present invention activates the promoter via calcium influx into the recombinant cell.

In one embodiment the recombinant cell is a non-pancreatic cell, preferably a non-pancreatic mammalian cell, more preferably a non-pancreatic human cell.

In one embodiment the recombinant cell is a mammalian cell, preferably a human cell. Recombinant mammalian cells are preferably mammalian cells selected from the group consisting of kidney cells, liver cells, stem cells, blood cells, brain cells, nerve cells, intestinal cells, fibroblasts, and adipose-derived cells, more preferably stem cells, kidney cells or liver cells, most preferably stem cells or kidney cells, in particular kidney cells. Recombinant human cells are preferably human cells selected from the group consisting of HEK-293, HeLa, mesenchymal stem cells (MSC), induced pluripotent stem cells (iPSC), pheochromocytoma of the rat adrenal medulla (PC12), the mouse neuroblastoma cell line (N2A), liver hepatocellular carcinoma (HepG2), enteroendocrine L-cells, human epithelial colorectal adenocarcinoma (Caco2), and clinical-grade human neural stem cell (CTX), preferably HEK-293, HeLa and MSC cells, most preferably HEK-293 cells.

In one embodiment the recombinant cell is a transiently transfected recombinant cell. In one embodiment the recombinant cell is a recombinant cell transiently transfected with a nucleic acid construct comprising a promoter which is responsive to carbohydrate metabolism of said recombinant cell, wherein the promoter is operably linked to a gene coding for a therapeutic protein, preferably a recombinant cell transiently co-transfected with a nucleic acid construct comprising a promoter which is responsive to carbohydrate metabolism of said recombinant cell, wherein the promoter is operably linked to a gene coding for a therapeutic protein and a nucleic acid construct coding for a cellular component for sensing extracellular carbohydrates and/or a nucleic acid construct coding for a glucoincretin receptor.

In one embodiment the recombinant cell is a stably transfected recombinant cell. In one embodiment the recombinant cell is a recombinant cell stably transfected with a nucleic acid construct comprising a promoter which is responsive to carbohydrate metabolism of said recombinant cell, wherein the promoter is operably linked to a gene coding for a therapeutic protein, preferably a recombinant cell stably co-transfected with a nucleic acid construct comprising a promoter which is responsive to carbohydrate metabolism of said recombinant cell, wherein the promoter is operably linked to a gene coding for a therapeutic protein and a nucleic acid construct coding for a cellular component for sensing extracellular carbohydrates and/or a nucleic acid construct coding for a glucoincretin receptor.

In a specific embodiment, the recombinant cell of the present invention comprises a nucleic acid construct comprising a promoter which is responsive to glucose metabolism of said cell, wherein the promoter is operably linked to a gene coding for a therapeutic protein and optionally the recombinant cell further comprises a nucleic acid construct coding for a cellular component for sensing extracellular glucose and/or a nucleic acid construct coding for a glucoincretin receptor.

In another specific embodiment, the recombinant cell of the present invention comprises a nucleic acid construct comprising a calcium-responsive promoter, wherein the promoter is operably linked to a gene coding for a therapeutic protein and optionally the recombinant cell further comprises a nucleic acid construct coding for a membrane protein or a fragment thereof or a subunit of a membrane protein or a fragment thereof, wherein the membrane protein is selected from the group consisting of G-protein coupled receptors, SLC2A family glucose transporters, SLC5A family sodium-glucose linked transporters, potassium channels, calcium channels and sodium channels, in particular calcium channels, and/or a nucleic acid construct coding for a glucoincretin receptor.

In another specific embodiment, the recombinant cell of the present invention comprises a nucleic acid construct comprising a calcium-responsive promoter, wherein the promoter is operably linked to a gene coding for a therapeutic protein and optionally the recombinant cell further comprises a nucleic acid construct coding for a voltage-gated calcium channel and/or a nucleic acid construct coding for a glucoincretin receptor.

A particular, exemplary embodiment of the expression system of the invention is shown in Figure 2B. Herein, HEK-293 cells comprising a nucleic acid construct comprising a synthetic promoter comprising multiple tandem repeats of binding sites of a NFAT transcription factor and a nucleic acid construct coding for a Caᵥ1.3 channel are displayed, whereas extracellular glucose are taken up by the cells to generate increased amounts of ATP. The subsequent closure of ATP-sensitive potassium channels (K_{ATP}) activates Caᵥ1.3, resulting in increased Ca²⁺ influx and the calcium-dependent activation of NFAT-regulated transcription units.

Another particular, exemplary embodiment of the expression system of the invention is shown in Figure 12E. Herein, HEK-293 cells comprising a nucleic acid construct comprising a synthetic promoter comprising multiple tandem repeats of binding sites of a NFAT transcription factor linked to a gene coding for shGLP1, a nucleic acid construct comprising a synthetic promoter comprising binding sites of a CREB transcription factor linked to a gene coding for mINS, a nucleic acid construct coding for a Caᵥ1.3 channel and a nucleic acid construct coding for a GLP-1 receptor are displayed. D-glucose activates P_{NFAT}-driven promoters by excitation-transcription coupling and triggers dose-dependent expression of secreted human glucagon-like peptide 1 (shGLP1). shGLP1 activates constitutively expressed GLP-1 receptor (GLP1R) via an autocrine loop and triggers insulin expression from P_{CRE}-driven promoters.

In one embodiment the therapeutic protein is an insulinogenic agent selected from the group consisting of GLP1R-agonists, insulin, insulin analogues, growth hormones, insulin-like growth factors; an anorexic hormone; or a protein that activates brown fat metabolism, preferably selected from the group consisting of GLP1R-agonists, insulin and insulin analogues.

In one preferred embodiment, the recombinant cell comprises two nucleic acid constructs, wherein the first nucleic acid construct comprises a promoter which is responsive to carbohydrate metabolism of said recombinant cell, wherein the promoter is operably linked to a gene coding for a first therapeutic protein and wherein the second nucleic acid construct comprises a promoter which is responsive to carbohydrate metabolism of said recombinant cell, wherein the promoter is operably linked to a gene coding for a second therapeutic protein, wherein the first therapeutic protein is different from the second therapeutic protein. In a particular embodiment, the first therapeutic protein is a GLP1R-agonist, preferably shGLP1 as shown in SEQ ID NO: 35 or exedin-4 and the second therapeutic protein is an insulin analogue or insulin, preferably human insulin.

A GLP1R-agonist is any molecule that activates the GLP-1 receptor (GLP1R). GLP1R-agonists are usually selected from the group consisting of GLP-1, shGLP1, preferably shGLP1 as shown in SEQ ID NO: 35, exedin-4, exenatide, liraglutide, lixisenatide, albiglutide and dulaglutide. Preferred GLP1R-agonists are selected from the group consisting of shGLP1, preferably shGLP1 as shown in SEQ ID NO: 35 and exedin-4. Most preferred is shGLP1 or shGLP1 as shown in SEQ ID NO: 35.

Insulin analogues are usually selected from the group consisting of compounds derived from insulin that has been altered in its structure for the primary purpose of enhanced pharmaceutics or pharmacology. Preferred insulin analogues are selected from the group consisting of human, rodent, porcine, or bovine insulin, as well as Lispro, Aspart, Glulisine, Glargine and Detemir.

Growth hormones are usually selected from the group of hormones which stimulates growth in animal or plant cells, especially (in animals) that secreted by the pituitary gland. Preferred growth hormones are selected from the group consisting of ephinephrine, norepheniphrine and glucocorticoids.

Insulin-like growth factors are usually selected from the group consisting of hormones that are similar in molecular structure to insulin. Preferred insulin-like growth factors are selected from the group consisting of IGF1, IGF2 and IGFBP-6.

Anorexic hormones are usually selected from the group consisting of adiponectin, amylin, calcitonin, cholecystokinin (CCK), gastrin, gastric inhibitory polypeptide (GIP), ghrelin, leptin, motilin, pramlintide, secretin, somatostatin and peptide YY. Preferred anorexic hormones are selected from the group consisting of amylin, adiponectin, amylin- and adiponectin-analogues. Amylin- and adiponectin-analogues have usually an amino acid sequence identity of at least 70%, preferably at least 80%, more preferably at least 90%, most preferably at least 95%, in particular at least 97%, more particular at least 99% with the naturally occuring amylin and adiponectin, preferably with the naturally occuring human amylin and human adiponectin.

A protein that activates brown fat metabolism is usually selected from the group consisting of β2 AR activators, BMP7 irisin107, fibroblast growth factor 21 and natriuretic peptides. Preferred proteins that activates brown fat metabolism is selected from the group consisting of β2 AR activators and natriuretic peptides.

In a preferred embodiment the therapeutic protein is human insulin, human GLP-1 or a modified or truncated GLP-1, preferably the modified GLP1R-agonist shGLP1as shown in SEQ ID NO: 35.

In one embodiment the therapeutic protein is an agent against a metabolic disease, wherein the metabolic disease is selected from the group consisting of T1D (type-1 diabetes), T2D (type-2 diabetes), diabetic ketoacidosis, obesity, cardiovascular disease, the metabolic syndrome and cancer. Preferably the metabolic disease is selected from the group consisting of T1D (type-1 diabetes), T2D (type-2 diabetes), diabetic ketoacidosis, and the metabolic syndrome.

Type 1 diabetes (also known as diabetes mellitus type 1) is a form of diabetes mellitus that results from the autoimmune destruction of the insulin-producing beta cells in the pancreas. The subsequent lack of insulin leads to increased glucose in blood and urine.

Type 2 diabetes (also known as diabetes mellitus type 2) is a long term metabolic disorder that is characterized by high blood sugar, insulin resistance, and relative lack of insulin.

Cardiovascular diseases are usually selected from the group consisting of consisting of: myocardial interstitial disease, cardiac fibrosis, heart failure such as heart failure with diastolic heart failure (DHF), heart failure with preserved ejection fraction (HFpEF), congestive heart failure (CHF), asymptomatic left ventricular diastolic dysfunction (ALVDD), coronary atherosclerosis, cancer and diabetes, inflammatory bowel disease, chronic prostatitis, infections, pulmonary inflammation, osteomyelitis, renal disease, gout, arthritis and shock.

Metabolic syndrome (also referred to as syndrome X) is a cluster of risk factors that is responsible for increased cardiovascular morbidity and mortality. The National Cholesterol Education Program - Adult Treatment panel (NECP - ATP III) identified metabolic syndrome as an independent risk factor for cardiovascular disease. (National Institutes of Health: Third Report of the National Cholesterol Education Program Expert Panel on Detection, Evaluation, and Treatment of High Blood Cholesterol in Adults (Adult Treatment Panel III). Executive publication no. 01-3670). As used herein, metabolic syndrome is defined according to the World Health Organization criteria (1999) which require presence of diabetes mellitus, impaired glucose tolerance, impaired fasting glucose or insulin resistance, AND two of the following: blood pressure: ≥ 140/90 mmHg; dyslipidaemia: triglycerides (TG): ≥ 1.695 mmol/L and high-density lipoprotein cholesterol (HDL-C) ≤ 0.9 mmol/L (male), ≤ 1.0 mmol/L (female); central obesity: waist:hip ratio > 0.90 (male); > 0.85 (female), and/or body mass index > 30 kg/m2; microalbuminuria: urinary albumin excretion ratio ≥ 20 mg/min or albumin:creatinine ratio ≥ 30 mg/g.

In one specific embodiment the therapeutic protein is an agent against T1D and/or T2D.

In a further aspect the present invention provides an encapsulated cell, comprising the recombinant cell as described above and a semi-permeable membrane.

Encapsulated cell biodelivery is based on the concept of isolating cells from the recipient host's immune system by surrounding the cells with a semipermeable biocompatible membrane before implantation within the host. Cells are immunoisolated from the host by enclosing them within implantable polymeric capsules formed by a semi-permeable membrane. This approach prevents the cell-to-cell contact between host and implanted tissues, eliminating antigen recognition through direct presentation.

The encapsulated cell of the present invention has a semi-permeable membrane which is tailored to control diffusion of molecules, such as growth factor hormones, neurotransmitters, peptides, antibodies and complements, based on their molecular weight. Using encapsulation techniques, cells can be transplanted into a host without immune rejection, either with or without use of immunosuppressive drugs. Useful biocompatible polymer capsules usually contain a core that contains cells, either suspended in a liquid medium or immobilised within an immobilising matrix, and a surrounding or peripheral region of permselective matrix or membrane ("jacket") that does not contain isolated cells, that is biocompatible, and that is sufficient to protect cells in the core from detrimental immunological attack. Encapsulation hinders elements of the immune system from entering the capsule, thereby protecting the encapsulated cells from immune destruction. The semipermeable nature of the membrane also permits the biologically active molecule of interest to easily diffuse from the capsule into the surrounding host tissue and allows nutrients to diffuse easily into the capsule and support the encapsulated cells. The capsule can be made from a biocompatible material. A "biocompatible material" is a material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics as described e.g. by WO 92/19195 or WO 95/05452. Components of the biocompatible material may include a surrounding semipermeable membrane and the internal cell-supporting scaffolding. Preferably, the recombinant cells are seeded onto the scaffolding, which is encapsulated by the permselective membrane. The filamentous cell-supporting scaffold may be made from any biocompatible material selected from the group consisting of acrylic, polyester, polyethylene, polypropylene polyacetonitrile, polyethylene teraphthalate, nylon, polyamides, polyurethanes, polybutester, silk, cotton, chitin, carbon, or biocompatible metals. Also, bonded fibre structures can be used for cell implantation (U.S. Pat. No. 5,512,600, incorporated by reference). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere (WO 98/05304, incorporated by reference). Woven mesh tubes have been used as vascular grafts (WO 99/52573, incorporated by reference). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

Various polymers and polymer blends can be used to manufacture the semipermeable membrane, including alginate, alginate-poly-(L-lysine)-alginate, polycarbonate, polyethylene, polyethylene-terephthalate, collagen, gelatin, agarose, cellulose acetate and cellulose sulfate, polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Such membranes, and methods of making them are disclosed by e.g. U.S. Pat. Nos. 5,284,761 and 5,158,881.

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations, which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired.

The encapsulated cell devices are implanted according to known techniques. Many implantation sites are contemplated for the devices and methods of this invention. These implantation sites include, but are not limited to the intraperitoneal cavity, into the kidney capsules, subcutaneous tissues, the portal vein, the liver and the cerebral cortex.

In one embodiment the encapsulated cell comprises a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In one embodiment the semi-permeable membrane comprises a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In a further aspect the present invention provides thus a method for producing a therapeutic protein in vivo in a mammal, said method comprising:
(a) providing an in vitro population of the recombinant cells as described herein into an implantable semi-permeable device;
(b) implanting the device with the cell population into a mammalian host; and
(c) maturing the cell population in said device in vivo such that at least some cells of the cell population are cells that produce a therapeutic protein in response to carbohydrate stimulation in vivo.

In one embodiment the implantable semi-permeable device are capsules or beads consisting of a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In a further aspect the present invention provides an in vitro cell culture comprising the recombinant cell as described herein, wherein said recombinant cell is expressing a therapeutic protein in the presence of carbohydrates, preferably in the presence of glucose. Cells can be grown and maintained in vitro as generally known. The nutrient medium and the cells generally are contained in a suitable vessel to which an adequate supply of oxygen and carbon dioxide is furnished in order to support cell growth and maintenance. Cell cultures may be batch systems in which nutrients are not replenished during cultivation but oxygen is added as required, fed-batch systems in which both nutrient and oxygen concentrations are monitored and replenished as necessary, and perfusion systems in which nutrient and waste product concentrations are monitored and controlled. Cells may be cultured in a variety of media. Commercially available media such as Ham's F10 (Sigma-Aldrich Chemie GmbH, Buchs, Switzerland), Minimal Essential Medium (MEM; Sigma-Aldrich Chemie GmbH), RPMI-1640 (Sigma-Aldrich Chemie GmbH, Basel, Switzerland), and Dulbecco's Modified Eagle's Medium ((DMEM; Sigma-Aldrich Chemie GmbH) are suitable for culturing the host cells.

In a further aspect the present invention provides a method, preferably an in vitro method, of producing a recombinant cell expressing a therapeutic protein, said method comprising the steps of:
(a) obtaining a population of cells;
(b) transfecting said population of cells with a nucleic acid construct comprising a promoter which is responsive to a product of the carbohydrate metabolism of said cell, wherein the promoter is operably linked to a gene coding for a therapeutic protein;
(c) incubating the population of transfected cell in the presence of carbohydrates for a sufficient time to permit the transfected cells to express a therapeutic protein.

In one embodiment the population of cells is further transfected in step (b) with a nucleic acid construct encoding a cellular component for sensing extracellular carbohydrates. The cells, the promoter which is responsive to a product of the carbohydrates metabolism, and the cellular component for sensing extracellular carbohydrates used in the method are as described above. The carbohydrate used is preferably glucose. Incubation time in the presence of carbohydrates is usually between 0.5 to 96 hours, preferably between 1 to 12 hours.

In a further aspect the present invention provides the recombinant cell or the encapsulated cell as described herein for use as a medicament.

In a further aspect the present invention provides the recombinant cell or the encapsulated cell as described herein for use in a method for the prevention, delay of progression or treatment of a metabolic disease in a subject.

Also provided is the use of the recombinant cell or the encapsulated cell as described herein for the manufacture of a medicament for the prevention, delay of progression or treatment of a metabolic disease in a subject.

Also provided is the use of the recombinant cell or the encapsulated cell as described herein for the prevention, delay of progression or treatment of a metabolic disease in a subject.

Also provided is a method for the prevention, delay of progression or treatment of a metabolic disease in a subject, comprising administering to said subject the recombinant cell or the encapsulated cell as described herein.

In one embodiment the metabolic disease is selected from the group consisting of T1D (type-1 diabetes), T2D (type-2 diabetes), diabetic ketoacidosis, obesity, cardiovascular disease, the metabolic syndrome and cancer. Preferably the metabolic disease is selected from the group consisting of T1D (type-1 diabetes), T2D (type-2 diabetes), diabetic ketoacidosis, and the metabolic syndrome.

In a further aspect the present invention provides a method to deliver a nucleic acid construct to a cell, wherein the nucleic acid construct comprises a promoter which is responsive to a physiological effect of membrane depolarization caused by a product of the carbohydrate metabolism of said cell, wherein the promoter is operably linked to a gene coding for a therapeutic protein, wherein the recombinant cell further comprises
a nucleic acid construct coding for a cellular component for sensing extracellular carbohydrates, wherein the cellular component for sensing extracellular carbohydrates is a voltage-gated calcium channel and wherein the promoter which is responsive to carbohydrate metabolism is responsive to a physiological effect of membrane depolarization caused by the carbohydrate metabolism of said recombinant cell, comprising administering said nucleic acid construct to said cell, whereby said gene coding for a therapeutic protein is expressed in said cell in response to carbohydrate stimulation. In one embodiment the cell is further transfected with a nucleic acid construct encoding a cellular component for sensing extracellular carbohydrates. The promoter which is responsive to carbohydrate metabolism, and the cellular component for sensing extracellular carbohydrates used in the method are as described above. The carbohydrate used is preferably glucose.

In one embodiment the nucleic acid construct is delivered to a cell in a subject e.g. by gene therapy. Thus in a specific embodiment the present invention provides a method to deliver a nucleic acid construct to a cell of a subject, wherein the nucleic acid construct comprises a promoter which is responsive to carbohydrate metabolism of said cell, wherein the promoter is operably linked to a gene coding for a therapeutic protein, comprising administering said nucleic acid construct to said subject, whereby said gene coding for a therapeutic protein is expressed in said cell of said subject in response to carbohydrate stimulation.

For delivery of a nucleic acid construct to a cell in a subject it may be valuable in some instances to utilize or design vectors to deliver the nucleic acid construct to a particular cell type. Certain vectors exhibit a natural tropism for certain tissue types. Cell type specificity or cell type targeting may be achieved in vectors derived from viruses having characteristically broad infectivities by the modification of the viral envelope proteins. For example, cell targeting has been achieved with adenovirus vectors by selective modification of the viral genome knob and fiber coding sequences to achieve expression of modified knob and fiber domains having specific interaction with unique cell surface receptors. Other methods of cell specific targeting have been achieved by the conjugation of antibodies or antibody fragments to the envelope proteins. Alternatively, particularly moieties may be conjugated to the viral surface to achieve targeting. Additionally, the virally encoded nucleic acid construct may also be under control of a tissue specific promoter region allowing expression of the gene coding for a therapeutic protein preferentially in particular cell types.

It will be apparent to one skilled in the art that nucleic acid constructs according to the invention may be introduced into an animal subject in a variety of ways including enterally (orally, rectally or sublingually) or parenterally (intravenously, subcutaneously, or by inhalation). The nucleic acid constructs may be provided to the mammal by e.g. implanted catheters. The nucleic acid constructs can be instilled into a body cavity to facilitate transduction of the surrounding tissues. Examples of such body cavities into which the solutions may be provided for the delivery of nucleic acids include the peritoneal cavity, pleural cavity, and the abdominal cavity. Additionally the nucleic acid constructs may be provided in other fluid containing spaces.

The nucleic acid constructs to be delivered to a cell of a subject may further comprise additional carriers, excipients or diluants. The compositions comprising the nucleic acid constructs may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. The concentration of the nucleic acid constructs in the compositions can vary widely, i.e., from less than about 0.1%, usually at or at least about 2% to as much as 20% to 50% or more by weight, and will be selected primarily by fluid volumes, viscosities, etc., in accordance with the particular mode of administration selected.

### EXAMPLES

### General experimental procedures

***Vector Design.*** Comprehensive design and construction details for all expression vectors are provided in **Table 1.** Some expression vectors were constructed by Gibson assembly using the GeneArt® Seamless Assembly Cloning Kit (Obio Technology, Shanghai, China; cat. no. BACR(C)20144001). Plasmids encoding K_{ATP}-subunits (pCMV Human SUR1 and pCMV6c hKir6.2(BIR)) were kindly provided by Susumu Seino (Kobe University, Kobe, Japan). Plasmids encoding Caᵥ2.2-, Caᵥ1.2- and Caᵥ1.3-subunits (CaV1.3e[8a,11,31b,Δ32,42a], CaV1.2, Cav2.2e[Δa10, Δ18a, Δ24a, 31a, 37a, 46], Cavb3 and CaVα2δ1) were kindly provided by Diane Lipscombe (Brown University, RI, USA).

### Table 1: Plasmids and oligonucleotides used and designed herein

Oligonucleotides: restriction endonuclease-specific sites are underlined in oligonucleotide sequences. Annealing base pairs contained in oligonucleotide sequences are shown in capital letters.

**Abbreviations: *att*R1/2***,* Gateway®-compatible recombination sites; **BFP,** blue fluorescent protein; **BlastR,** gene conferring blasticidin resistance; **Caᵥ1.2,** member 2 of the Caᵥ1 family of L-type voltage-gated Ca²⁺ channels; **Caᵥ1.3,** member 3 of the Caᵥ1 family of L-type voltage-gated Ca²⁺ channels; **Caᵥ2.2,** N-type voltage-gated Ca²⁺ channel; **c-fos,** human proto-oncogene from the Fos family of transcription factors;**Cacnalb,** α1-subunit of rat Caᵥ2.2 (NCBI Gene ID: 257648); **Cacnalc,** α1-subunit of mouse Caᵥ1.2 (NCBI Gene ID: 12288); **Cacnald,** α1-subunit of rat Caᵥ1.3 (NCBI Gene ID: 29716); **Cacnb3,** β3-subunit of rat Caᵥ1.3 (NCBI Gene ID: 25297); **Cacna2d1,** α2δ-subunit of rat Caᵥ1.3 (NCBI Gene ID: 25399); ***ccd*B*****,*** DNA gyrase toxin for positive selection; ***Cmr,*** chloramphenicol-resistance gene for negative selection; **CRE,** cAMP-response element; **dTomato,** destabilized red fluorescent protein variant; **EGFP,** enhanced green fluorescent protein (Genbank: U55762); **GLP-1,** glucagon-like peptide 1; **GLP1R,** human GLP-1 receptor; **GLuc,** *Gaussia princeps* luciferase; **hGCK,** human pancreas glucokinase (also known as hexokinase 4; NCBI Gene ID: 2645); **hGlut2,** human facilitated glucose transporter member 2 (also known as SLC2A2; NCBI Gene ID: 6514); **hGNAT3,** human guanine nucleotide binding protein alpha transducing 3 (NCBI Gene ID: 346562); **hKir6.2,** human inwardly rectifying KATP-channel subunit (also known as KCNJ11; NCBI Gene ID: 3767); **hSUR1,** regulatory sulphonylurea receptor subunit of human K_{ATP}-channels (also known as ABCC8; NCBI Gene ID: 6833); **hT1R2,** member 2 of the human taste receptor type 1 family (NCBI Gene ID: 80834); **hT1R3,** member 3 of the human taste receptor type 1 family (NCBI Gene ID: 83756); **IL2/4,** interleukin 2/4; **ITR,** inverted terminal repeats of SB100X; **K_{ATP}**, adenosine triphosphate-sensitive potassium channel; **Luc,** Firefly Luciferase; **MCS,** multiple cloning site; **mINS,** modified rat insulin variant as shown in SEQ ID NO: 36 for optimal expression in HEK-293 cells; **NFAT,** nuclear factor of activated T-cells; **NF-κB,** nuclear factor kappa-light-chain-enhancer of activated B cells; **P2A,** picornavirus-derived ribosome skipping sequence optimized for bicistronic expression in mammalian cells; **pA,** polyadenylation signal; **P_{cFOS},** synthetic mammalian promoter containing a tetrameric c-fos response element ((c-fos)₄-Pₘᵢₙ; Sheng M et al., Mol Cell Biol 8, 2787-96 (1988)); **PCR,** polymerase chain reaction; **P_{CRE}**, CRE-containing synthetic mammalian promoter; **P_{CREm},** modified PCRE variant; **P_{hEF1α},** human elongation factor 1α promoter; **PEST,** peptide sequence rich in proline, glutamic acid, serine and threonine; **P_{hCMV},** human cytomegalovirus immediate early promoter; **P_{hCMV*-1},** tetracycline-responsive promoter (tetO₇-P_{hCMVmin}); **P_{hCMVmin},** minimal version of P_{hcMV}; **Pₘᵢₙ,** minimal eukaryotic TATA-box promoter (5'-TAGAGGGTATATAATGGAAGCTCGACTTCCAG-3') as shown in SEQ ID NO: 33; **P_{NFAT-IL2},** synthetic mammalian promoter containing three tandem repeats of a murine IL2 NFAT-binding site ((NFAT_{IL2})₃-Pₘᵢₙ; Rooney JW et al., EMBO J 13, 625-633 (1994)); **P_{NFAT-IL4},** synthetic mammalian promoter containing three tandem repeats of a murine IL4 NFAT-binding site ((NFAT_{IL4})₃-Pₙᵢₙ; Rooney JW et al., EMBO J 13, 625-633 (1994)); **P_{NFAT-IL4x5,}** synthetic mammalian promoter containing five tandem repeats of a human IL4 NFAT-binding site ((NFAT_{IL4})₅-Pₘᵢₙ); **P_{NFAT-IL4x7},** synthetic mammalian promoter containing seven tandem repeats of a human IL4 NFAT-binding site ((NFAT_{IL4})₇-Pₘᵢₙ); **P_{NFAT-IL4x9},** synthetic mammalian promoter containing nine tandem repeats of a human IL4 NFATbinding site ((NFAT_{IL4})₉-Pₘᵢₙ); **P_{NFkB},** synthetic mammalian promoter containing a NP-κB-response element (Genbank: EU581860.1); **P_{RPBSA},** constitutive synthetic mammalian promoter; **P_{SV40},** simian virus 40 promoter; **PuroR,** gene conferring puromycin resistance; **rtTA,** reverse TetR-dependent mammalian transactivator; **SB100X,** optimized Sleeping Beauty transposase; **SEAP,** human placental secreted alkaline phosphatase; **shGLP1,** short human glucagon-like peptide 1; ***tetO,*** TetR-specific operator; **TetR,** *Escherichia coli Tn*10-derived tetracycline-dependent repressor of the tetracycline resistance gene; **TurboGFP:destl,** PEST-tagged TurboGFP variant (Evrogen); **ZeoR,** gene conferring zeocin resistance. **Oligonucleotides:** Restriction endonuclease-specific sites are underlined and annealing base pairs are indicated in capital letters.

| **Plasmid** | **Description and Cloning Strategy** | **Reference or Source** |
|---|---|---|
| pCaV1.2 | pcDNA3.1(+)-derived constitutive Cacnalc expression vector (P_{hCMV}-Cacna1c-pA) (Addgene no. 26572). | Helton TD et al., J Neurosci 25, 10247-10251 (2005) |
| pCaV1.3 | pcDNA3.1(+)-derived constitutive Cacna1d expression vector (P_{hCMV}-Cacna1d-pA) (Addgene no. 26576). | Xu W et al., J Neurosci 21,5944-5951 (2001) |
| pCavb3 | pcDNA3. 1(+)-derived constitutive Cacnb3 expression vector (P_{hCMV}-Cacnb3-pA) (Addgene no. 26574). | Prof. Lipscombe Lab |
| pCaVα2δ1 | pcDNA3.1(+)-derived constitutive Cacna2dl expression vector (P_{hCMV}-Cacna2d1-pA) (Addgene no. 26575). | Lin Y et al., J Neurophysiol 92, 2820-2830 (2004) |
| pCav2.2 | pcDNA3.1(+)-derived constitutive Cacnalb expression vector (P_{hCMV}-Cacna1b-pA) (Addgene no. 26569). | Bell TJ et al., Neuron 41, 127-138 (2004) |
| pcDNA3.1(+ ) | Mammalian expression vector (P_{hCMV}-MCS-pA). | Life Technologies, CA |
| pcDNA3.1(+ )-Hygro | pcDNA3.1(+) containing a constitutive expression unit for a gene product conferring Hygromycin B resistance | Life Technologies, CA |
| pcDNA3.2/v 5-DEST | Mammalian Gateway®-compatible destination vector (P_{hCMV}-*att*R1*-Cm^{r}-ccd*B*-att*R2-pA). | Life Technologies, CA |
| pcDNA3.2/v 5-DEST-hGlut2 | pcDNA3.2/v5-DEST containing a constitutive hGlut2 expression unit (P_{hCMV}-hGlut2-pA) (Addgene no. 18086). | Takanaga H et al., FASEB J 24, 2849-2858 (2010) |
| pCK53 | P_{CRE}-driven SEAP-expression vector (P_{CRE}-SEAP-pA). | Kemmer C et al., J Control Release 150, 23-9(2011) |
| pCMV6 | Mammalian expression vector (P_{hCMV}-MCS-pA). | Yamada Y et al., Proc Natl Acad Sci USA 89, 251-255 (1992) |
| pCMV6-hSUR1 | pCMV6 containing a constitutive hSUR1 expression unit (P_{hCMV}-hSUR1-pA). | Beguin P et al., EMBO J 18, 4722-4732 (1999) |
| pCMV6-hKir6.2 | pCMV6 containing a constitutive hKir6.2 expression unit (P_{hCMV}-hKir6.2-pA). | Inagaki N et al., Science 270, 1166-1170 (1995) |
| pCMV-T7-SB100 | Constitutive SB100X expression vector (P_{hCMV}-SB100X-pA) (Addgene no. 34879). | Mates L et al., Nat Genet 41, 753-761 (2009). |
| pDA43 | Tetracycline-responsive GLuc expression vector (P_{hCMV*-1}-GLuc-pA). | Müller M et al., Metab Eng 14, 325-335 (2012) |
| pDA145 | P_{CRE}-driven mINS-expression vector (P_{CRE}-mINS-pA). | Ausländer D et al., Mol Cell 55, 397-408 (2014) |
| pDONR | Gateway®-compatible cloning vector. | Life Technologies, CA |
| pDONR-hGCK | pDONR containing hGCK (Addgene no. 23750). | Johannessen CM et al., Nature 468, 968-972 (2010) |
| pEGFP-N1 | Constitutive EGFP-expression vector (P_{hCMV}-EGFP-pA). | Clontech, CA |
| pGLPIR | Constitutive GLP1R expression vector (P_{hCMV}-GLP1R-pA). | Chepurny OG et al., Cell Tissue Res 307, 191-201 (2002) |
| pGNAT3 | pCMV6 containing a constitutive hGNAT3 expression unit (P_{hCMV}-GNAT3-pA). | OriGene, MD |
| pHY30 | P_{NFAT-IL2}-driven SEAP expression vector ((NFAT_{IL2})₃-Pₘᵢₙ-SEAP-pA). | Ye H et al., Science 332, 1565-1568 (2011) |
| pHY57 | P_{NFAT-IL2}-driven shGLP1 expression vector ((NFAT_{IL2})₃-Pₘᵢₙ-shGLP1-pA). | Ye H et al., Science 332, 1565-1568 (2011) |
| pMM195 | Constitutive GLP1R expression vector (P_{SV40}-GLP1R-pA). GLP1R was excised from pGLP-1-R using *Hind*III/*Xba*I and ligated into the corresponding sites (*Hind*III*Xba*I) of pSEAP2-Control. | Müller of Prof. Fussenegger's lab |
| pSBbi-BP | SB100X-specific transposon containing a constitutive BFP and PuroR expression unit (ITR-P_{hEF1α}-MCS-pA:P_{RPBSA}-BFP-P2A-PuroR-pA-ITR) (Addgene no. 60512). | Kowarz E et al., Biotechnol J 10, 647-653 (2015) |
| pSBbi-RB | SB100X-specific transposon containing a constitutive dTomato and BlastR expression unit (ITR-P_{hEF1α}-MCS-pA:P_{RPBSA}-dTomato-P2A-BlastR-pA-ITR) (Addgene no. 60522). | Kowarz E et al., Biotechnol J 10, 647-653 (2015) |
| pSBbi-RP | SB100X-specific transposon containing a constitutive dTomato and PuroR expression unit (ITR-P_{hEF1α}-MCS-pA:P_{RPBSA}-dTomato-P2A-PuroR-pA-ITR) (Addgene no. 60513). | Kowarz E et al., Biotechnol J 10,647-653 (2015) |
| pSBtet-Pur | SB100X-specific transposon containing a tetracycline-inducible luciferase expression unit and a constitutive rtTA and PuroR expression unit (P_{hCMV*-1}-Luc-pA:P_{RPBSA}-rtTA-P2A-PuroR-pA) (Addgene no. 60507). | Kowarz E et al., Biotechnol J 10,647-653 (2015) |
| pSEAP2-Control | Constitutive SEAP expression vector (P_{SV40}-SEAP-pA). | Clontech, CA |
| pSP16 | P_{CREm}-driven SEAP expression vector (P_{CREm}-SEAP-pA). | Saxena P et al., Nat Commun 7, 11247 (2016) |
| pUC57 | pUC19-derived prokaryotic expression vector. | GeneScript, NJ |
| pZeoSV2(+) | Constitutive mammalian expression vector conferring zeocin resistance (P_{hCMV}-ZeoR-pA). | Life Technologies, CA |
| pFS119 | P_{NFAT-IL4x5}-driven TurboGFP:destl expression vector ((NFAT_{IL4})₅-Pₘᵢₙ-TurboGFP:dest1-pA). | Sedlmayer of Prof. Fussenegger's lab |
| | TurboGFP:destl was PCR-amplified using oligonucleotides oFS200 (5'-tgttggtaaagaattcgcccaccaagctttaagccaccATGGAGAGCGACG AGAGCGGCC-3') and oMX78 (5'-ctttaaaaaacctcccacacctccc-3'), restricted with *Eco*RI/*Hpa*I and cloned into the corresponding sites (*Eco*RI/*Hpa*I) of pMX57. | |
| pKK56 | Constitutive Cacna2dl and Cacnb3 expression vector (P_{hEF1α}-Cacna2dl-P2A-Cacnb3-pA). Custom-designed P_{hEF1α}-Cacna2d1-P2A-Cacnb3 was restricted with *Mlu*I/*Not*I and cloned into the corresponding sites (*Mlu*I/*Not*I) of pcDNA3.1(+)-Hygro | Krawczyk of Prof. Fussenegger's lab |
| pKR32 | P_{NFkB}-driven SEAP expression vector (P_{NFkB}-SEAP-pA). Custom-designed P_{NFkB} was restricted with *Mlu*I/*Hind*III and cloned into the corresponding sites (*Mlu*I/*Hind*III) of pHY30. | Rössger of Prof. Fussenegger's lab |
| pTlR2 | Constitutive hT1R2 expression vector (P_{hCMV}-hT1R2-pA). Custom-designed hT1R2 was restricted with *Eco*RI/*Xba*I and cloned into the corresponding sites (*Eco*RI/*Xba*I) of pcDNA3.1(+). | Wieland of Prof. Fussenegger's lab |
| pTlR3 | Constitutive hT1R3 expression vector (P_{hCMV}-hT1R3-pA). Custom-designed hT1R3 was restricted with *Eco*RI/*Xba*I and cloned into the corresponding sites (*Eco*RI/*Xba*I) of pcDNA3.1(+). | Wieland of Prof. Fussenegger's lab |
| pHY101 | P_{CRE}-driven expression vector for SEAP and mINS (P_{CRE}-SEAP-P2A-mINS-pA). | This work |
| | SEAP was PCR-amplified from pMX57 using oligonucleotides OHY701 (5'-gccacggggatgaagcagaagctgaattcgCCACCATGCTGCTGCTG CTGCTGCTGCTG-3') and OHY702 (5'-ggaaaagttggttgctccTGTCTGCTCGAAGCGGCCGGCCGCC C-3'), P2A-mINS was PCR-amplified from pMX155 using oligonucleotides OHY703 (5'-gggcggccggccgcttcgagcagacaGGAGCAACCAACTTTTCC-3') and OHY704 (5'-cgaagcggccggccgccccgactctagaaagcttTCAGTTGCAGTAGTT CTCCAGT-3') and both fragments were joined and cloned into the *Eco*RI/*Xba*I sites of pCK53 using the GeneArt® Seamless Cloning and Assembly Kit. | |
| pMX53 | P_{cFOS}-driven SEAP expression vector (P_{cFOS}-SEAP-pA; P_{cFOS}, (c-fos)₄-Pₘᵢₙ). | This work |
| | Pₘᵢₙ-SEAP was PCR-amplified from pSP16 using oligonucleotides OMX59 (5'-cgcgtgctagcagcctgacgtttcagagactgacgtttcagagactgacgtttcagaga ctgacgtttcagatctctcga ggtcgacagcggAGACTCTAGAGGGTATATAATG-3') and OMX24 (5'-CTTGAGCACATAGCCTGGACCGTTTCCGTA-3 '), restricted with *Nde*I/*Nhe*I and cloned into the corresponding sites (*Nde*I/*Nhe*I) of pHY30. | |
| pMX56 | P_{NFAT-IL4}-driven SEAP expression vector ((NFAT_{IL4})₃-Pₘᵢₙ-SEAP-pA). | This work |
| | Pₘᵢₙ-SEAP was PCR-amplified from pSP16 using oligonucleotides OMX63 (5'- cgcgtg-[ctagctacattggaaaattttatacacgtt]₃AGACTCTAGAGGGTATAT AA-3') and OMX24 (5'-CTTGAGCACATAGCCTGGACCGTTTCCGTA-3 '), restricted with *Nde*I/*Nhe*I and cloned into the corresponding sites (*Nde*I/*Nhe*I) of pHY30. | |
| pMX57 | P_{NFAT-IL4x5}-driven SEAP expression vector ((NFAT_{IL4})₅-Pₘᵢₙ-SEAP-pA). | This work |
| | P_{NFAT-IL4x5} (5'- cgcgtg-[ctagctacattggaaaattttatacacgtt]₅-agactc-Pₘᵢₙ-cttggcaatccggtactgttggtaaagaattcgcccacc-3') was excised from pMX570 with *Nhe*I/*Eco*RI and cloned into the corresponding sites (*Nhe*I/*Eco*RI) of pMX56. | |
| pMX58 | P_{NFAT-IL4x7}-driven SEAP expression vector ((NFAT_{IL4})₇-Pₘᵢₙ-SEAP-pA). | This work |
| | P_{NFAT-IL4x7} (5'- cgcgtg-[ctagctacattggaaaattttatacacgtt]₇-agactc-Pₘᵢₙ-cttggcaatccggtactgttggtaaagaattcgcccacc-3') was excised from pMX580 with *Nhe*I/*Eco*RI and cloned into the corresponding sites (*Nhe*I/*Eco*RI) of pMX56. | |
| pMX59 | P_{NFAT-IL4x9}-driven SEAP expression vector ((NFAT_{IL4})₉-Pₘᵢₙ-SEAP-pA). | This work |
| | P_{NFAT-IL4x9} (5'- cgcgtg-[ctagctacattggaaaattttatacacgtt]₉-agactc-Pₘᵢₙ-cttggcaatccggtactgttggtaaagaattcgcccacc-3') was excised from pMX590 with *Nhe*I/*Eco*RI and cloned into the corresponding sites (*Nhe*I/*Eco*RI) of pMX56. | |
| pMX61 | P_{NFAT-IL4x5}-driven shGLP1-expression vector ((NFAT_{IL4})₅-Pₘᵢₙ-shGLP1-pA). | This work |
| | shGLPl was PCR-amplified from pHY57 using oligonucleotides OMX70 (5'-ctgttggtaaagaattcgcccaccATGAAGATCATCCTGTGGCTGT GTG-3') and OMX71 (5'-ggagtcgacgcgtGAAGCGGCCGGCCTCATTTACCAGGAGA GTGGG-3'), restricted with *Eco*RI/*Fse*I and cloned into the corresponding sites (*Eco*RI/*Fse*I) of pMX57. | |
| pMX68 | P_{NFAT-IL4x5}-driven mINS-expression vector ((NFAT_{ILA})₅-Pₘᵢₙ-mINS-pA). | This work |
| | mINS was PCR-amplified from pDA145 using oligonucleotides OMX72 (5'-ctgttggtaaagaattcgcCCACCATGGCCCTGTGGATGCGCTT CCTGC-3') and OMX73 (5'-CTGAAACATAAAATGAATGCAATTGTTGTTG-3'), restricted with *Eco*RI/*Mfe*I and cloned into the corresponding sites (*Eco*RI/*Mfe*I) of pMX57. | |
| pMX90 | Constitutive hGCK expression vector (P_{hCMV}-hGCK-pA). GCK was PCR-amplified from pDONR-hGCK using oligonucleotides OMX100 (5'-ctggaattccaccATGCTGGACGACAGAGCCAGG-3') and OMX101 (5'-ctctagatgcatgctcgagTCACTGGCCCAGCATACAGGCCTTC -3'), restricted with *Eco*RI/*Xho*I and cloned into the corresponding sites (*Eco*RI/*Xho*I) ofpcDNA3.1(+). | This work |
| pMX99 | P_{NFAT-IL4x7}-driven mINS-expression vector ((NFAT_{IL4})₇-Pₘᵢₙ-mINS-pA). | This work |
| | mINS was excised from pMX68 using *Eco*RI/*Hpa*I and cloned into the corresponding sites (*Eco*RI/*Hpa*I) of pMX58. | |
| pMX100 | P_{NFAT-IL4x9}-driven mINS-expression vector ((NFAT_{IL4})₉-Pₘᵢₙ-mINS-pA). | This work |
| | mINS was excised from pMX68 using *Eco*RI/*Hpa*I and cloned into the corresponding sites (*Eco*RI/*Hpa*I) of pMX59. | |
| pMX115 | P_{NFAT-IL4x9}-driven shGLP1-expression vector ((NFAT_{IL4})₉-Pₘᵢₙ-shGLP1-pA). shGLPl was excised from pMX61 using *Eco*RI/*Hpa*I and cloned into the corresponding sites (*Eco*RI/*Hpa*I) of pMX59. | This work |
| pMX117 | P_{NFAT-IL4x7}-driven shGLP1-expression vector ((NFAT_{IL4})₉-Pₘᵢₙ-shGLP1-pA). | This work |
| | shGLPl was excised from pMX61 using *Eco*RI/*Hpa*I and cloned into the corresponding sites (*Eco*RI/*Hpa*I) of pMX58. | |
| pMX155 | P_{NFAT5}-driven expression vector for EGFP and mINS (P_{NFAT5}-EGFP-P2A-mINS-pA). | This work |
| | EGFP-P2A was PCR-amplified from pEGFP-N1 using oligonucleotides OMX193 (5'-ctaacgaattcgcATGGTGAGCAAGGGCGAGGAG-3') and OMX192 (5'-cacagggccatgggtccaggattctcctccacgtcgcctgcctgcttcagcagggaaa agttggttgctccCTTGTACAG CTCGTCCATGCCGAGAG-3'), restricted with *Eco*RI/*Nco*I and cloned into the corresponding sites (*Eco*RI/*Nco*I) of pMX100. | |
| pMX245 | SB100X-specific transposon containing a tetracycline-inducible luciferase expression unit and a constitutive ZeoR expression unit (ITR-P_{hCMV*-1}-Luc-pA:P_{RPBSA}-ZeoR-pA-ITR). ZeoR was PCR-amplified from pZeoSV2(+) using oligonucleotides OMX248 (5'-ctgcacctgaggccaccATGGCCAAGTTGACCAGTGCCG-3') and OMX249 (5'-caagcttcacgacaggccttcgaaTCAGTCCTGCTCCTCGGCCACG AAG-3'), restricted with *Bsu*36I/*Bst*BI and cloned into the corresponding sites (*Bsu*36I/*Bst*BI) of pSBtet-Pur. | This work |
| pMX248 | SB100X-specific transposon containing a P_{NFAT5}-driven EGFP and mINS expression unit and a constitutive ZeoR expression unit (ITR-P_{NFAT5}-EGFP-P2A-mINS-pA:P_{RPBSA}-ZeoR-pA-ITR). | This work |
| | GFP-P2A-mINS was PCR-amplified from pMX155 using oligonucleotides OMX251 (5'-catttctctatcgataactagtGAGCTCTTACGCGTGCTAGC-3') and OMX252 (5'-cggggtaccGGTCGACGGATCCTTATCGATTTTACC-3') and restricted with *Spe*I/*Kpn*I. pMX245 was then restricted with *Kpn*I/*Pci*I into a 4476bp fragment A and a 1736bp fragment B. Fragment A was further restricted with *Xba*I/*Nco*I/*Hind*III to yield a 2182bp fragment C. Finally, fragment C (*Pci*I/*Xba*I), fragment B (*Kpn*I/*Pci*I) and GFP-P2A-mINS (*Spe*I/*Kpn*I) were assembled by ligating compatible *Spe*I/*Xba*I ends. | |
| pMX250 | SB 100X-specific transposon containing a constitutive dTomato and PuroR expression unit and a constitutive GLP1R expression unit (ITR-P_{hEF1α}-GLP1R-pA:P_{RPBSA}-dTomato-P2A-PuroR-pA-ITR). | This work |
| | GLP1R was PCR-amplified from pMM195 using oligonucleotides OMX253 (5'-ctaccccaagctggcctctgaggccaccatggctcgagATGGCCGTCACC CCCAGCCTGCTGCGCCTG-3') and OMX245 (5'-tgggctgcaggtcgactctagagTCAGCTGCAGGAATTTTGGCAG GTGGCTGC-'3), restricted with *Nco*I/*Xba*I and cloned into the corresponding sites (*Nco*I/*Xba*I) of pSBbi-RP. | |
| pMX251 | SB100X-specific transposon containing a constitutive dTomato and BlastR expression unit and a constitutive Cacna2dl and Cacnb3 expression unit (ITR-P_{hEF1α}-Cacna2d1-P2A-Cacnb3-pA:P_{RPBSA}-dTomato-P2A-BlastR-pA). Cacna2dl-P2A-Cacnb3 was PCR-amplified from pKK56 using oligonucleotides OMX254 (5'-aaggtgtcgtgaaaactaccccaagctggcctctgaggccaccATGGCTGCT GGCTGCCTGCTGGCCTTGACTCTG-3') and OMX255 (5'-gagaattgatccccaagcttggcctgacaggccCTAGACTCGAGCGGC CGCTCATCAGTAGCTGTCCTTAGGCCAAGGCC GG-3'), restricted with S*fi*I and cloned into the corresponding site (S*fi*I) of pSBbi-RB. | This work |
| pMX252 | SB100X-specific transposon containing a constitutive BFP and PuroR expression unit and a constitutive Cacnald expression unit (ITR-P_{hEF1α}-Cacnald-pA:P_{RPBSA}-BFP-P2A-PuroR-pA-ITR). Cacnald was PCR-amplified from pCaV1.3 using oligonucleotides OMX256 (5'-aaggtgtcgtgaaaactaccccaagctggcctctaggccaccaccATGCAGCAT CAACGGCAGCAGCAAGAGGAC-3') and OMX257 (5'-gagaattgatccccaagcttggcctgacaggccCTAGACTCGAGCGGC CGCTCATCAGAGCATCCGTTCAAGCATCTGTA GGGCGATC-3'), restricted with S*fi*I and cloned into the corresponding site (S*fi*I) of pSBbi-BP. | This work |
| pMX256 | SB100X-specific transposon containing a P_{NFAT5}-driven SEAP and mINS expression unit and a constitutive EGFP and ZeoR expression unit (ITR-P_{NFAT5}-SEAP-P2A-mINS-pA:P_{RPBSA}-EGFP-P2A-ZeoR-pA-ITR). | This work |
| | P_{RPBSA}-EGFP-P2A-ZeoR-pA was excised from pMX257 using *Kpn*I/*Sph*I and cloned into the corresponding sites (*Kpn*I/*Sph*I) of pMX260. | |
| pMX257 | SB100X-specific transposon containing a P_{CRE}-driven SEAP and mINS expression unit and a EGFP and ZeoR constitutive expression unit for (ITR-P_{CRE}-SEAP-P2A-mINS-pA:P_{RPBSA}-EGFP-P2A-ZeoR-pA-ITR). EGFP-P2A was PCR-amplified from pEGFP-N1 using oligonucleotides OWH107 (5'-attgaattcgcgaggccaccaaggccaccATGGTGAGCAAGGGCGA GGAGCT-3') and OWH74 (5'-aggtccaggattctcctccacgtcgcctgcctgcttcagcagggaaaagttggttgctcc agatccCTTGTACAGCTCGT CCATGC-3'), P2A-ZeoR was PCR-amplified from pMX248 using oligonucleotides OWH108 (5'-acttttccctgctgaagcaggcaggcgacgtggaggagaatcctggacctATGGC CAAGTTGACCAGTGCCGTT-3') and OWH29 (5'-AACAACAGATGGCTGGCAACTAGAAG-3'), and both fragments were joined by overlapping PCR using OWH107 and OWH29. P_{CRE}-SEAP-P2A-mINS was then excised from pHY101 using *Mlu*I/*Sal*I and pMX248 was restricted with *Mlu*I/*Sal*I/*Sfi*I/*Dra*III resulting in a 2942bp fragment A and a 635bp fragment B. Finally, EGFP-P2A-ZeoR was restricted with *Sfi*I/*Dra*III and ligated with fragment A (*Dra*III/*Mlu*I)*,* P_{CRE}-SEAP-2A-mINS (*Mlu*I/*Sal*I) and fragment B (*Sal*I/*Sfi*I)*.* | This work |
| pMX258 | SB100X-specific transposon containing a P_{CRE}-driven SEAP and mINS expression unit and a constitutive ZeoR expression unit (ITR-P_{CRE}-SEAP-P2A-mINS-pA:P_{RPBSA}-ZeoR-pA-ITR). P_{CRE}-SEAP-P2A-mINS-pA was excised from pHY101 using *Mlu*I/*Sal*I and cloned into the corresponding sites (*MluI*/*Sal*I) of pMX248. | This work |
| pMX260 | SB100X-specific transposon containing a P_{NFAT5}-driven SEAP and mINS expression unit and a constitutive ZeoR expression unit (ITR-P_{NFAT5}-SEAP-P2A-mINS-pA:P_{RPBSA}-ZeoR-pA-ITR). | This work |
| | SEAP-P2A-mINS-pA was excised from pHY101 using *Eco*RI/*Sal*I and cloned into the corresponding sites (*Eco*RI/*Sal*I) of pMX248. | |
| pMX570 | pUC57-derived vector containing custom-designed P_{NFAT-IL4x5}. | This work |
| pMX580 | pUC57-derived vector containing custom-designed P_{NFAT-IL4x7}. | This work |
| pMX590 | pUC57-derived vector containing custom-designed P_{NFAT-IL4x9}. | This work |
| pWH29 | P_{NFAT-ILAx5}-driven GLuc expression vector ((NFAT_{ILA})₅-Pₘᵢₙ-GLuc-pA). | This work |
| | P_{NFAT-IL4x5} was excised from pMX57 using *Mlu*I/*Eco*RI and cloned into the corresponding sites (*Mlu*I/*Eco*RI) of pDA43. | |

***Cell culture and transfection.*** Human embryonic kidney cells (HEK-293T, ATCC: CRL-11268) were cultured in Dulbecco's modified Eagle's medium (DMEM, Invitrogen) supplemented with 10% (v/v) fetal bovine serum (FBS; Sigma-Aldrich, Buchs, Switzerland; cat. no. F7524, lot no. 022M3395) and 1% (v/v) penicillin/streptomycin solution (PenStrep; Biowest, Nuaillé, France; cat. no. L0022-100) at 37°C in a humidified atmosphere containing 5% CO₂. The human 1.1E7 β-cell line (Sigma-Aldrich, cat. no. EC10070101) was cultured in RPMI 1640 medium (ThermoFisher Scientific, cat. no. 11875085) supplemented with 10% FBS and 1% PenStrep. Human islets (HIR; Prodo Laboratories, Irvine, CA; lot. no. HP-16161-01) were transferred from Prodo Transport medium (PIM(T)®; Prodo Laboratories; cat. no. IMT001GMP) into CMRL medium (ThermoFisher Scientific; cat. no. 11530037) supplemented with 10% FBS, 1% PenStrep, 1% ITS, 5mM D-Glucose, 2mM GlutaMAX, 1mM pyruvate, 10mM nicotinamide and 2.5mM HEPES, and cultivated for seven days prior to encapsulation while changing fresh CMRL medium every third day. For passaging, cells of pre-confluent HEK-293 and 1.1E7 cultures were detached by incubation in 0.05% Trypsin-EDTA (Life Technologies, CA, USA; cat. no. 25300-054) for 3min at 37°C, collected in 10ml cell culture medium, centrifuged for 3min at 290g and resuspended in fresh culture medium at standard cell densities (1.5 x 10⁵ cells / mL), before seeding into new tissue culture plates. Cell number and viability were quantified using an electric field multichannel cell counting device (Casy Cell Counter and Analyzer Model TT, Roche Diagnostics GmbH). For transfection, a solution containing 2-3µg plasmid DNA and 6-9µg polyethyleneimine (PEI; Polysciences, Eppelheim, Germany; cat. no. 24765-2) was incubated in 300µl serum- and antibiotics-free DMEM for 30min at 22°C and subsequently added dropwise to 3 x 10⁵ cells seeded per well of a 6-well plate. 12h after addition of PEI, transfected HEK-293 cells were detached by incubation in Trypsin-EDTA, centrifuged (3min at 290g) and resuspended in low/no-glucose medium (glucose-free DMEM [Life Technologies, CA, USA; cat. no. 11966-025] supplemented with 10% FBS, 1% PenStrep, 0-2mM D-glucose and 0.7mM CaCl₂) and reseeded at a cell density of 2 x 10⁵ / mL. Unless stated otherwise, D-glucose or other control compounds were added to transfected cells after cultivation under low/no-glucose conditions for another 12h.

***Quantification of target gene expression.*** Expression levels of human placental secreted alkaline phosphatase (SEAP) in culture supernatants were quantified according to a *p-*nitrophenylphosphate-based light absorbance time course (Wang H et al., Nucleic Acids Res 43(14):e91 (2015)). SEAP levels in mouse serum were profiled using a chemiluminescence-based assay (Roche Diagnostics GmbH, Mannheim, Germany; cat. no. 11 779 842 001). Human insulin levels secreted by 1.1E7 cells and human islets were quantified using an Ultrasensitive C-peptide ELISA kit (Mercordia, Uppsala, Sweden; cat. no. 10-1141-01). Murine insulin levels (mINS) in culture supernatants and mouse serum were quantified with a Mouse Insulin ELISA kit (Mercordia, Uppsala, Sweden; cat. no. 10-1247-01). Short human glucagon-like peptide 1 (shGLP1) levels in culture supernatants were quantified with a Mouse IgG ELISA Kit (Immunology Consultants Laboratory Inc., Portland, OR; cat. no. E-90G). Bioactive GLP-1 levels in mouse serum were quantified with a High Sensitivity GLP-1 Active ELISA Kit (Merck Millipore, Schaffhausen, Switzerland; cat. no. EZGLPHS-35K). TurboGFP was visualized by fluorescence microscopy using a Nikon Ti-E base Wide Field microscope (Nikon AG, Egg, Switzerland) equipped with a Hammamatsu Orca Flash 4 digital camera, a 20×objective, a 488 nm / 509 nm excitation and emission filter set and NIS Elements AR software (version 4.3.0).

***Generation of stable cell lines.*** The monoclonal HEK-293_{NFAT-SEAP} cell line, transgenic for depolarization-stimulated SEAP expression, was constructed by co-transfecting HEK-293 cells with a 20:1 (w/w) mixture of pMX57 (P_{NFAT3}-SEAP-pA) and pZeoSV2(+) (P_{SV40}-*zeo*-pA), followed by selection in culture medium containing 1mg/mL zeocin (Life Technologies, CA, USA; cat. no. R250-05) and FACS-mediated single-cell cloning. Sixteen cell clones were picked and the best-in-class HEK-293_{NFAT-SEAP1} was used for all follow-up studies.
The polyclonal HEK_{GLP1R} population, transgenic for high-level GLP1R expression, was constructed by cotransfecting 3x10⁶ HEK-293 cells with 9500ng pMX250 (ITR-P_{hEF1_{α}}-GLP1R-pA:P_{RPBSA}-dTomato-P2A-PuroR-pA-ITR) and 500ng of the Sleeping Beauty transposase expression vector pCMV-T7-SB100 (P_{hCMV}-SB100X-pA, (61)). After selection with 1µg/mL puromycin for two passages, the surviving population HEK_{MX250} was FACSsorted into three different subpopulations according to different red-fluorescence intensities. The subpopulation with top 10% dTomato intensity HEKGLP1R showed highest sensitivity to GLP-1 and was used for all follow-up studies.
The polyclonal HEK_{MX252} population, transgenic for stable expression of the α1D subunit of Cav1.3 (Cacnald), was constructed by co-transfecting 3x10⁶ HEK-293 cells with 9500ng pMX252 (ITR-P_{hEF1_{α}}-Cacna1d-pA:P_{RPBSA}-BFP-P2A-PuroR-pA-ITR) and 500ng pCMV-T7-SB100 and selecting with 0.5µg/mL puromycin for two passages.
The polyclonal HEK_{Cav1.3} population, transgenic for stable expression of the full Caᵥ1.3 channel componentry, was constructed by co-transfecting 3x10⁶ HEK_{MX252} cells with 9500ng pMX251 (ITR-P_{hEF1_{α}}-Cacna2d1-P2A-Cacnb3-pA:P_{RPBSA}-dTomato-P2A-BlastR-pAITR) and 500ng pCMV-T7-SB100 and selecting with 10µg/mL of blasticidin for three passages.
The monoclonal HEK-β cell line, transgenic for glucose-stimulated SEAP- and insulin-expression, was constructed by cotransfecting 3x10⁶ HEK_{Cav1.3} cells with 9500ng pMX256 (ITR-P_{NFAT5}-SEAP-P2A-mINS-pA:P_{RPBSA}-EGFP-P2A-ZeoR-pA-ITR) and 500ng pCMV-T7-SB100. After selection with 100µg/mL zeocin for three passages, 5% of the surviving population with highest EGFP expression levels were subjected to FACS-mediated single-cell cloning. 50 cell clones were selected and clone no. 4 showing optimal glucoseinducible insulin expression was used for all follow-up studies.

***FACS-mediated cell sorting.*** HEK-293 cells expressing EGFP (488nm laser, 505nm long-pass filter, 530/30 emission filter) or dTomato (561nm laser, 570nm long-pass filter, 586/15 emission filter) were sorted using a Becton Dickinson LSRII Fortessa flow cytometer (Becton Dickinson, Allschwil, Switzerland) while excluding dead cells and cell doublets. Untreated HEK-293 cells or parental polyclonal populations were used as negative controls.

***RT-PCR.*** Total RNA of untreated HEK-293 cells was isolated using the ZR RNA MiniPrep™ kit (Zymo Research, CA, USA; cat. no. R1064), treated with DNaseI (Thermo Scientific, cat. no. EN0521) and cDNA was synthesized using the Applied Biosystems High Capacity cDNA Reverse Transcription Kit (Life Technologies, CA, USA; cat. no. 4368814). Amplicons of target components were generated by PCR reactions of 30-45 cycles of denaturation (95°C, 20 s), annealing (58 °C, 30 s) and extension (68 °C, 30 s) with primers listed in **Table 2.** PCR products were separated on 1.5% agarose gels supplemented with 1× RedSafe™ (iNtRON Biotechnology, Gyeonggi-do, Republic of Korea; cat. no. 21141) and visualized under UV light. For quantitative analysis, PCR reaction (2min at 50°C, 20s at 95°C and 60 cycles of Is at 95°C followed by 1min at 60°C) was performed on the Eppendorf Realplex² Mastercycler (Eppendorf GmbH, Hamburg, Germany) using the SYBR® Green PCR Master Mix (Life Technologies, CA, USA; cat. no. 4309155) and the primers listed in Table 2 . The relative cycle threshold (CT) was determined and normalized against the endogenous human glyceraldehyde 3-phosphate dehydrogenase (GAPDH) gene.

**Table 2: RT-PCR primers**

| **Target** | **Sequences** | **Amplicon size** | **SEQ ID NO:** | **Reference** |
|---|---|---|---|---|
| GAPD | 5'-ACATCGCTCAGACACCATG-3' | 143bp | 13 | Zhu X et al., J Cell Biochem |
| H | 5'-TGTAGTTGAGGTCAATGAA-3' | | 14 | 113,3069-85 (2012) |
| GLUT1 | 5'-TGAACCTGCTGGCCTTC-3' | 399bp | 15 | Castro MA et al., Pflugers |
| | 5'-GCAGCTTCTTTAGCACA-3' | | 16 | Arch 457, 519-28 (2008) |
| GLUT2 | 5'-TCCAGCTACCGACAGCCTATTC-3' | 253bp | 17 | Limbert C et al., Cytotherapy |
| | 5'-AGATGGCACAAACAAACATCCC-3' | | 18 | 13,802-13 (2011) |
| GLUT3 | 5'-AAGGATAACTATAATGG-3' | 411bp | 19 | Castro MA et al., Pflugers |
| | 5'-GGTCTCCTTAGCAGG-3' | | 20 | Arch 457, 519-28 (2008) |
| SGLT1 | 5'-TCCTGCTTGCTATTTTCTGGA-3' | 150bp | 21 | Kanwal A et al., Anal |
| | 5'-ATAATCGTGGGACAGTTGCTG-3' | | 22 | Biochem 429, 70-5 (2012) |
| SGLT2 | 5'-TCCTCACCCTCACGGTCTC-3' | 180bp | 23 | Kanwal A et al., Anal |
| | 5'-CTGGGGCTCATTCATCTCCA-3' | | 24 | Biochem 429, 70-5 (2012) |
| SUR1 | 5'-TCACACCGCTGTTCCTGCT-3' | 412bp | 25 | Du Q et al., Hum Reprod 25, |
| | 5'-AGAAGGAGCGAGGACTTGCC-3' | | 26 | 2774-82 (2010) |
| SUR2 | 5'-CATTGCCTACTTATTTCTCTCAG-3' | 474bp | 27 | Du Q et al., Hum Reprod 25, |
| | 5'-ACCATTCTGAAGAAAGCCAG-3' | | 28 | 2774-82 (2010) |
| Kir6.1 | 5'-CTGGCTGCTCTTCGCTATC-3' | 234bp | 29 | Du Q et al., Hum Reprod 25, |
| | 5'-AGAATCAAAACCGTGATGGC-3' | | 30 | 2774-82 (2010) |
| Kir6.2 | 5'-CCAAGAAAGGCAACTGCAACG-3' | 449bp | 31 | Du Q et al., Hum Reprod 25, |
| | 5'-ATGCTTGCTGAAGATGAGGGT-3' | | 32 | 2774-82 (2010) |

***Glucose-stimulated insulin secretion (GSIS).*** Encapsulated human islets and 1.1E7 cells were washed (incubation for 30 min) in 0.25mL Krebs-Ringer Bicarbonate Buffer (Sigma-Aldrich, cat. No. K4002; 129mM NaCl, 5mM NaHCO₃, 4.8mM KCl, 1.2mM KH₂PO₄, 1.2mM MgSO₄, 2.5mM CaCl₂, 10mM HEPES, 0.1% BSA, pH7.4) and incubated for 30min in low-glucose (2.8mM) Krebs-Ringer Bicarbonate Buffer. The culture was then switched to high-glucose (30mM) Krebs-Ringer Bicarbonate Buffer for another 30min. The secreted isoform of the connecting peptide (C-peptide) produced during proinsulin processing was quantified using the Ultrasensitive Human C-peptide ELISA and the capsules were then transferred to fresh culture medium and cultivated until the next GSIS assay.

***Chemicals and Soft drinks.*** Ethanol (EtOH; cat. no. 02860), acetic acid (cat. no. A6283), calcium chloride dihydrate (stock solution 0.5M in ddH₂O; cat. no. C7902), D-glucose (stock solution 1M in ddH₂O; cat. no. G-7021), D-mannitol (stock solution 0.1M in ddH₂O; cat. no. M4125), D-mannose (stock solution 1M in ddH₂O; cat. no. M6020), D-galactose (stock solution 0.1M in ddH₂O; cat. no. 48263), magnesium sulfate (MgSO₄; cat. no. M2643), nicotinamide (stock solution 0.5M in ddH₂O; cat. no. N0636), potassium phosphate monobasic (K₂HPO₄; cat. no. P5655), sodium bicarbonate (NaHCO₃; cat. no. S5761), sucrose (stock solution 0.1M in ddH₂O; cat. no. S0389), D-maltose monohydrate (stock solution 0.1M in ddH₂O; cat. no. M9171), D-xylose (stock solution 0.1M in ddH₂O; cat. no. X1500), L-glutamine (stock solution 0.15M in ddH₂O; cat. no. G3126), 3-(N-Morpholino)propanesulfonic acid (MOPS; stock solution 0.1M in ddH₂O; cat. no. M1254), palmitic acid (stock solution 0.02M in EtOH; cat. no. P0500) and alloxan monohydrate (cat. no. A7413) were purchased from Sigma-Aldrich (Buchs, Switzerland). Blasticidin S HCl (cat. no. A1113903), GlutaMAX™ Supplement (cat. no. 35050061), Insulin Transferrin Selenium liquid media supplement (ITS; cat. no. 41400045), N-2-hydroxyethylpiperazine-N-2-ethane sulfonic acid (HEPES; stock solution 1M; cat no. 15630080), puromycin dihydrochloride (cat. no. A1113803), sodium pyruvate (stock solution 100mM; cat no. 11360070) and Zeocin™ selection reagent (cat. no. R25005) were purchased from ThermoFisher Scientific (Reinach, Switzerland). Recombinant human GLP-1 (stock solution 1mM in ddH₂O; cat. no. 130-08; lot no. 0108358), IL-2 (stock solution 10µM in 10mM aqueous acetic acid; cat. no. 200-02; lot no. 051512-1), IL-12 p70 (stock solution 1µM in DMEM; cat. no. 200-12; lot no. 0909S96) and IL-15 (stock solution 1µM in ddH₂O; cat. no. 200-15; lot no. 061024) were purchased from PeproTech EC Ltd (London, UK). D-fructose (stock solution 0.1M in ddH₂O; cat. no. 161350010), L-leucine (stock solution 0.01M in glucose-free DMEM; cat. no. 125121000) and linoleic acid (stock solution 0.02M in EtOH; cat. no. 215040050) were purchased from Acros Organics (Geel, Belgium). L-glucose anhydrous (stock solution 1M in ddH₂O; cat. no. AB116919) was purchased from abcr GmbH (Karlsruhe, Germany). Potassium chloride (KCl; stock solution 4M in ddH₂O; cat. no. A3582) and sodium chloride (NaCl; stock solution 5M in ddH₂O; cat. no. A2942) were purchased from AppliChem (Darmstadt, Germany). Citric acid anhydrous (stock solution 0.1M in ddH₂O; cat. no. sc-211113) was purchased from Santa Cruz Biotechnology (Dallas, USA). Poly(L-lysine) hydrobromide (cat. no. PLKB50) was purchased from Alamanda Polymers (Alabama, USA). Trisodium citrate 2-hydrate (stock solution 0.1M in ddH₂O; cat. no. 6448) was purchased from Merck Millipore (Schaffhausen, Switzerland). Bovine serum albumin (BSA; stock solution 10g/L; cat. no. B9000S) was purchased from NEB Biolabs (Ipswich, MA).Streptozotocin (cat. no. 1621) was purchased from Tocris Bioscience (Bristol, UK). Coke was purchased at local supermarkets, degassed by extensive shaking and directly administered to mice (4 × 200 µl).

***Animal experiments.*** The type 1 diabetes mouse model (T1D) was generated as described previously (Auslander D et al., Mol Cell 55, 397-408 (2014)) . In brief, fasted mice (2x 18h/day) were injected with a single dose of freshly diluted alloxan monohydrate (ALX; 200 mg/kg in 300µl phosphate buffered saline) and persistent fasting hyperglycemia (>20mM) developed after 48h. The type 2 diabetes mouse model (T2D) was generated as described in (Arora S et al., Global J Pharmacol 3, 81-84 (2009)). In brief, fasted mice (20h/day) were injected with daily doses of freshly diluted streptozotocin (STZ; 40mg/kg in 250µl ice-cold sodium citrate buffer [pH 4.5, 0.01M, 0.11g/L NaCl]) for five consecutive days and chronic fasting hyperglycemia (>10mM) developed after 3 weeks. Glycemia of mice were measured with a commercial glucometer (Contour® Next; Bayer HealthCare, Leverkusen, Germany; detection range: 0.5-35mM) purchased at local pharmacies. Intraperitoneal implants were produced by encapsulating transgenic HEK-293 cells, 1.1E7 cells or human islets into coherent alginate-poly-(L-lysine)-alginate beads (400µm; 500 cells or 1-10 IEQs /capsule) using an Inotech Encapsulator Research Unit IE-50R (EncapBioSystems Inc., Greifensee, Switzerland) set to the following parameters: a 200-µm nozzle with a vibration frequency of 1025Hz, a 25-mL syringe operated at a flow rate of 410 units and 1.12-kV voltage for bead dispersion (Ye H et al., PNAS 110, 141-146 (2013)). 5-9-weeks old female wild-type or ALX/STZ-pretreated CD-1 Swiss albino mice (Janvier Labs, Le Genest-Saint-Isle, France) were intraperitoneally injected with 1mL of glucose-free DMEM containing 1 x 10⁴ microcapsules. Blood serum was isolated using microtainer serum separating tubes (SST) according to the manufacturer's instructions (centrifugation for 5 min at 10 000 x g; Becton Dickinson, Plymouth, UK; cat. no. 365967). Most experiments involving animals were performed according to the directive of the European Community Council (2010/63/EU), approved by the French Republic and carried out by Ghislaine Charpin-El Hamri (No. 69266309; project No. DR2013-01 (v2)) and Marie Daoud-El Baba (No. 69266310; project No. DR2013-01 (v2)) at the Institut Universitaire de Technologie, UCB Lyon 1, F-69622 Villeurbanne Cedex, France. Animal experiments related to Fig. 12B, 12C, 12D and 12G were performed according to the protocol (Protocol ID: m20140301) approved by the East China Normal University (ECNU) Animal Care and Use Committee and in direct accordance with the Ministry of Science and Technology of the People's Republic of China on Animal Care Guidelines.

### Example 1: Coupling the β-cell-mimetic cascade of glycolysis-mediated calcium entry to a synthetic excitation-transcription coupling system

Glucose sensing was achieved by coupling the β-cell-mimetic cascade of glycolysis-mediated calcium entry to a synthetic excitation-transcription coupling system (D'Arco M and Dolphin AC, Sci Signal 5, pe34 (2012)) in human embryonic kidney cells. This human cell line is widely used in studies of ion channel activities (Thomas P and Smart TG, J Pharmacol Toxicol Methods 51, 187-200 (2005)) and shows optimal production capacities for antidiabetic proteins (Auslander D et al., Mol Cell 55, 397-408 (2014)). A cell-based assay was constructed in human embryonic kidney cells (HEK-293) to evaluate the stimulus strength of membrane depolarization with a quantitative reporter protein (Fig. 1A). When different calcium-responsive promoters (Hogan P et al., Genes Dev 17, 2205-2232 (2003)) were exposed to 75 mM potassium chloride (KCl), the synthetic promoter P_{NFAT-IL4}, which contains NFAT repeats derived from the murine interleukin 4 (IL4) promoter (Rooney JW et al., EMBO J 13, 625-633 (1994)), was most responsive to chemically induced membrane depolarization (Fig. 1B). Co-transfection of a voltage-gated calcium channel such as Caᵥ1.2 resulted in an amplified excitation-transcription coupling as well as a shift of the dose-response curve to higher sensitivity (Fig. 1C). The promoter architecture of five tandem NFAT_{IL4}-repeat sequences preceding a minimal eukaryotic TATA-box promoter ((NFAT_{IL4})₅-Pₘᵢₙ) showed optimal induction ratios between resting and depolarized states of membrane potentials (Fig. 1D). Because this promoter could also distinguish between signals generated by voltage-gated calcium channels of different activation thresholds (Fig. IE), we used pMX57 ((NFAT_{IL4})₅-Pₘᵢₙ -SEAP-pA) as a reporter system for all subsequent studies that involved excitation-transcription coupling.

To experimentally evaluate the contributing effects of each β-cell-derived component for sensing glucose (GLUT2, GCK, K_{ATP}, Caᵥ1.3) with the pMX57-based depolarization-induced transcriptional system, a combinatorial screening approach (Fig. 2A) was used. The expression of K_{ATP} components did not show significant contributions to glucose sensing, whereas GLUT2 overexpression increased overall glucose-induced calcium-dependent transcription (Fig. 2A, condition #10), and GCK overexpression might cause toxic effects at higher extracellular glucose concentrations (Fig. 2A, conditions #6, #14, #16). Indeed, semi-quantitative transcriptional profiling confirmed that the GLUT1 and GLUT3 glucose transporters (Castro M et al., Pflugers Arch 457, 519-528 (2008); Elsner M et al., Diabetologia 45, 1542-1549 (2002)) as well as the K_{ATP} subunits of metabolism-dependent potassium channels are endogenously expressed in wild-type HEK-293 cells (Fig. 7A), and most mammalian cell types express at least one hexokinase isoform (Robey RB and Hay N, Oncogene 25, 4683-4696 (2006)). Therefore, ectopic expression of the Caᵥ1.3 channel was sufficient to confer glucose sensitivity to HEK-293 cells (Fig. 2A, inset), HeLa (Fig. 3A) and human mesenchymal stem cells (Fig. 3B). In contrast, putative glucose sensors reported in the literature, such as the T1R2/T1R3 sweet taste receptors (Jang H et al., PNAS 104, 15069-15074 (2007)), failed to mediate target gene regulation in response to glucose levels that are relevant for glycaemic control (Fig. 7B). It can be concluded that Caᵥ1.3 represents the missing link to reconstitute an intact, physiologically relevant glucose-sensing cascade in HEK-293 cells (Fig. 2B), and that only minimal, targeted engineering is required to mimic β-cell function in non-pancreatic human cells.
To quantitatively analyse the system, ensure consistency in the design steps, and eventually predict circuit operation *in vivo,* we developed a dynamic mathematical model for the β-cell-derived glucose-sensing cascade. Briefly, this ordinary differential equation (ODE) model covers the components shown in Fig. 2B, a detailed representation of the cell's electrophysiology and a previously developed, simplified representation of *in vivo* glucose-insulin interactions (Auslander D et al., Mol Cell 55, 397-408 (2014)). We parametrized the model with experimental data across conditions and experimental assays to establish a single, quantitative representation of the system. The model reproduced, among others, the potassium (Fig. 1E) and glucose (Fig. 2A) dose-response curves. We used the model for quantitative circuit characterization and, ultimately, to make essential predictions of *in vivo* behaviours.

### Example 2: Substrate specificity of the Caᵥ1.3/P_{NFAT-IL4}-constituted glucose-sensing system

To test the substrate specificity of the Caᵥ1.3/P_{NFAT-IL4}-constituted glucose-sensing system, Caᵥ1.3/pMX57-transgenic HEK-293 cells were cultured in cell culture medium containing different sugar compounds such as osmotic controls (Fig. 4A), common dietary sugars (Fig. 4B) and other nutrients (Fig. 7C). D-glucose and D-mannose were the only component among the tested substrates in HEK-293 cells that, at their physiologically relevant concentrations, activated SEAP expression from the synthetic excitation-transcription coupling system. HEK-293 cells use the GLUT1 transporter (Fig. 7A), which is most specific to glucose. Other mammalian cells express other transporters such as GLUT2 (not the case for HEK-293) which are also permeable for other carbohydrates such as D-fructose and D-galactose (Augustin R, IUBMB Life 62, 315-333 (2010)). Similar performances as with HEK-293 cells would be expected with other sugars if the sugar transport is mediated by e.g. GLUT2. Mannose is an epimer of glucose with an almost identical structure and may be transported into HEK-293 cells and metabolized by HEK-293 cells the same way as HEK-293 cells treats glucose. Capitalizing on the tight induction kinetics of the P_{NFAT3}-regulated gene expression system (Fig. 4C), and on the system's strict Caᵥ1.3-dependent activation (Fig. 4C and Fig. 7D), a stable human HEK-293_{NFAT-SEAP1} reporter cell line transgenic for P_{NFAT-IL4}-driven SEAP expression (Fig. 8) was constructed. Ectopic expression of the Caᵥ1.3 channel in HEK-293_{NFAT-SEAP1} resulted in improved induction ratios between low and high extracellular glucose concentrations (Fig. 5A; ∼4.6 fold-induction from 5mM to 25mM glucose) compared with its transiently constructed counterpart system (Fig. 2A; ∼2.6 fold-induction). The induction of Caᵥ1.3-transgenic HEK-293_{NFAT-SEAP1} cells was clearly significant with the first 24 h and reached maximal SEAP expression levels after culture in high-glucose medium for 48 h (Fig. 5B). Additionally, SEAP expression could be switched to dose-dependent regulation even after maintaining the Caᵥ1.3-transgenic HEK-293_{NFAT}-_{SEAP1} cells in low-glucose conditions (2 mM) for different periods of time (Fig. 5C), and an independent time-course experiment showing glucose-mediated system sensitization as well as starvation-mediated desensitization confirmed the reversibility of the synthetic excitation-transcription coupling system (Fig. 5D). The mathematical model reproduced this behaviour quantitatively (Fig. 5, A, B and D; Fig. 9), and independent predictions for varying Caᵥ1.3 dosages agreed well with experiments (Fig. 5A), emphasizing the model's consistency across constructs and conditions.

### Example 3: Diabetes treatment with microencapsulated Cav1.3-transgenic HEK-293NFAT-SEAP1 cells

To test the application potential of the glucose-induced excitation-transcription coupling system for diabetes treatment, Caᵥ1.3-transgenic HEK-293_{NFAT-SEAP1} cells were microencapsulated into coherent, semi-permeable and immunoprotective alginate-poly-(L-lysine)-alginate beads and implanted them into the peritoneum of mice, where they become vascularized and connected to the animal's bloodstream with appropriate oxygen supply (Jacobs-Tulleneers-Thevissen D et al., Diabetologia 56, 1605-1614 (2013)). Also *in vivo,* the transcriptional regulation system operated in a dose- and Caᵥ1.3-dependent manner, as recapitulated by the same *in vitro* dynamic model coupled to a mathematical representation of mouse physiology (Fig. 10A). Importantly, the system translated the characteristic average fasting glycaemia values of wild-type as well as T1D- and T2D-diabetic mouse models into correspondingly expressed SEAP levels in the serum (Fig. 6A). When wild-type non-diabetic mice were fed concentrated sugar solutions, such as aqueous D-glucose (0.5 M) or Coca-Cola® (11% w/w of total sugars), SEAP expression in the bloodstream was not significantly upregulated as compared with control groups receiving the same portions of water (Fig. 10B), thus indicating an intact glucose tolerance in healthy animals. Therefore, this glucose-induced transcriptional system might be tailored to exclusively target conditions of chronic hyperglycaemia while remaining insensitive to standard temporary glycaemic fluctuations such as nutrition.
State-of-the-art treatment options for diabetes mellitus are either long-acting drugs, such as stabilized GLP-1 variants, in which the frequency of drug injection can be reduced to weekly periods (T2D) (Trujillo J et al., Ther Adv Endocrinol Metab 6, 19-28 (2015)), or portable, external pump systems that self-sufficiently inject fast-acting insulin analogues according to the patient's instantaneous glycaemia (T1D) (Pickup JC, N Engl J Med 366, 1616-1624 (2012)). To test whether the expression levels achieved with the glucose-inducible excitation-transcription coupling system were compatible with antidiabetic therapeutic activities, HEK-293 cells were co-transfected with Caᵥ1.3 and the previously reported short human GLP-1 (shGLP1) construct (Ye H et al., Science 332, 1565-1568 (2011)) (pMX115; (NFAT_{IL4})₉-Pₘᵢₙ-shGLP1-pA) to engineer therapeutic mammalian cells that express GLP-1 exclusively under hyperglycaemic conditions (Fig. 10C). When implanting 5x10⁶ microencapsulated Caᵥ1.3/pMX115-transgenic HEK-293 cells into type-2 diabetic mice (Arora S et al., Global J Pharmacol 3, 81-84 (2009)), the self-sufficient exogenous expression of bioactive GLP-1 in the bloodstream (Fig. 6B) improved endogenous regulation of glucose-stimulated insulin secretion (Fig. 6C) and it substantially ameliorated glucose tolerance (Fig. 6D). Similarly, co-transfection of Caᵥ1.3 with an isogenic expression vector for insulin (mINS; modified rat insulin variant for optimized secretion) (Auslander D et al., Mol Cell 55, 397-408 (2014)) (pMX100; (NFAT_{IL4})₉-Pₘᵢₙ-mINS-pA) into HEK-293 cells generated glycaemia-triggered insulin-expressing cells (Fig. 10D). In agreement between experiments and model, self-sufficient insulin expression from Caᵥ1.3/pMX100-transgenic implants not only restored the typical insulin-deficiency (Polonsky KS, N Engl J Med 367, 1332-1340 (2012)) in a type-1 diabetic mouse model (Auslander D et al., Mol Cell 55, 397-408 (2014)) (Fig. 6E) but it also corrected the animals' persistent hyperglycaemia within 2-3 days (Fig. 6F). Importantly, hypoglycaemic side effects resulting from basal or excessive insulin expression at normoglycaemic levels that are often observed in classical insulin monotherapies (Pickup JC, N Engl J Med 366, 1616-1624 (2012)) were not detected (Fig. 6, E and F). Further development of the engineered system into the treatment option of choice to achieve lifelong glycaemic control in T1D patients also appears to be realistic because T1D mice developed hyperglycaemia with a high fatal outcome within one week (37.5% death cases without insulin treatment; n=24) whereas T1D animals treated with the Caᵥ1.3/pMX100-transgenic cell implant showed no hyperglycaemia and an improved life quality (0% death cases; n=24) (Fig. 11).

### Example 4: Diabetes treatment with microencapsulated HEK-β cells

β-cells modulate the insulin release not only in response to glucose but also by the action of glucoincretins such as GLP-1 (Lee YS, Metabolism 63, 9-19 (2014)). We therefore engineered HEK-293 for HEK-β componentry as well as for constitutive expression of the GLP-1 receptor (GLP1R) and P_{CRE}-driven insulin expression (Fig. 12E). The resulting HEK-β_{GLP} showed not only substantially improved insulin secretion dynamics (Fig. 15) compared to HEK-β, but was also sensitive to meals taken up by the animals (Fig. 12F). Although HEK-β_{GLP} was as potent as HEK-β in attenuating glycemic excursions in oral glucose tolerance tests (Fig. 12G), glucose homeostasis was less efficiently restored in type-1 diabetic mice compared to HEK-β (Fig. 12H). This finding was confirmed by model simulations and established HEK-β as the prime β-cell-mimetic design.
Implantation of microencapsulated HEK-β cells (Fig. 12A) stably transgenic for reversible glucose-stimulated insulin secretion (Figs. 13, 14) restored glucose (Fig. 12B) and blood insulin homeostasis (Fig. 12C) in type-1 diabetic mice as predicted by the mathematical model. Importantly, glucose homeostasis of treated T1D mice was robust during the entire 3-week study (Fig. 12B) and treated animals challenged by glucose tolerance tests (Fig. 12D) to simulate meal responses did not suffer from glycemic excursions during the HEK-β-mediated restoration of normoglycemia (Fig. 12B). In contrast, T1D mice receiving negative-control implants (Caᵥ1.3/pMX115-transgenic HEK-293 cells) remained hyperglycemic and did not survive the first glucose tolerance test at day 7 (Fig. 12B,D).
In a comparative analysis of reversible glucose-stimulated insulin secretion by the β-cell-mimetic HEK-β, the pancreatic β-cell line 1.1E7 (McCluskey JT, The Journal of biological chemistry 286, 21982-21992 (2011)) and human islets over three weeks, HEK-β showed higher insulin secretion capacity than 1.1E7 and human islets *in vitro* (Fig. 14C). In type-1 diabetic mice, implanted microencapsulated HEK-β and 1.1E7 were equally efficient in establishing postprandial glucose metabolism (Fig. 12G), but HEK-β restored glucose homeostasis more efficiently than 1.1E7 after 2 weeks and reached fasting glycemia levels of wild-type mice over the 3-week period (Fig. 12B). As for human islets, postprandial glucose metabolism could only be restored in two out of four T1D mice (Fig. 16), which confirms performance variations of encapsulated human islets. However, in the two T1D mice where postprandial glucose metabolism could be restored (Fig. 16), the human islets showed similar efficiency in providing glucose tolerance as HEK-β (Fig. 12D).
Coupling of Caᵥ1.3-based glucose sensing to insulin production and secretion resulted in the β-cell-mimetic HEK-β that provided increased 3-week insulin secretion profiles compared to the pancreatic cell line 1.1E7 and human islets *in vitro.* Control of postprandial glucose metabolism was similar between HEK-β and 1.1E7 but only HEK-β reached the blood glucose levels of healthy mice. Interestingly, since the different insulin release dynamics of HEK-β, 1.1E7 and human islets *in vitro* had apparently no significant impact on postprandial glucose metabolism, the differences in the secretion modality - constitutive for HEK-β, vesicular for 1.1E7 and human islets - may not be as relevant in response to meals as generally thought. This is supported by our model simulations and by the latest generation of basal insulin analogs such as insulin degludec (Tresiba®, Novo Nordisk), which provides autonomous glucose control for up to 42 hours without the need to synchronize its administration with meals.

Implantation of microencapsulated mammalian cells into patients does not necessitate immunosuppression, as host and graft communicate via secretory metabolites that diffuse across a semi-permeable biocompatible membrane (Jacobs-Tulleneers-Thevissen D et al., Diabetologia 56, 1605-1614 (2013)) . Since the first implantable alginate-poly-(L-lysine) capsules harbouring rat islet cells were presented almost 35 years ago, techniques for microencapsulated pancreatic β-cells have been continuously optimized for diabetes treatment, with several clinical trials already approved by governments. However, although current advances in stem cell research (Pagliuca FW et al., Cell 159, 428-439 (2014)) have successfully solved the previous issues of poor source availability and differentiation efficiency to generate adequate numbers of functional β-cells that elicit observable antidiabetic functions (Kobayashi N, Cell Transplant 15, 849-854 (2006)), regenerated pancreatic β-cells are generally restricted to the treatment of insulin-deficient type-1 diabetes (Bruin JE et al., Stem Cell Reports 4, 605-620 (2015)). Type-2 diabetes, however, is much more common, accounting for more than 95% of all diabetes cases and with a pathogenesis that often involves an impaired sensitivity of body cells to excessive levels of circulating insulin (Johnson AM and Olefsky JM, Cell 152, 673-684 (2013)). Glucagon-like peptide 1 (GLP-1) is an incretin hormone naturally released from the intestine after meal ingestion that not only acts on β-cells to stimulate their postprandial release of insulin (Drucker DJ et al., Lancet 368, 1696-1705 (2006)) but also circulates to other somatic cells to (i) promote satiety, (ii) improve hepatic insulin sensitivity, (iii) slow gastric emptying and (iv) inhibit glucagon secretion (Ye H et al., PNAS 110, 141-146 (2013)). Currently, subcutaneous injection of long-acting GLP-1 analogues is the state-of-the-art treatment option for most type-2 diabetes cases (Trujillo J et al., Ther Adv Endocrinol Metab 6, 19-28 (2015)) .
The quest for a cell-based glucose sensor has always been in great demand for biomedical research (Auslander D et al., Mol Cell 55, 397-408 (2014)). Although a variety of putative glucose-sensing components, including GPCRs (Jang H et al., PNAS 104, 15069-15074 (2007)), bacterial transcriptional repressors (Gaigalat L et al., BMC Mol Biol 8, 104 (2007)) and human nuclear receptors (Mitro N et al., Nature 445, 219-223 (2007)) have been characterized, to the best of our knowledge, a successful translation of the glucose input into a transcriptional message that includes antidiabetic activities *in vivo* has not yet been achieved. In this work, we used the low threshold voltage-gated calcium channel Caᵥ1.3, which permits long-lasting Ca²⁺ influxes during weak membrane depolarizations (Lipscombe D et al., J Neurophysiol 92, 2633-2641 (2004)), to sense glycaemia-relevant extracellular glucose levels but to remain insensitive to other potential trigger compounds such as metabolites and salts within their physiologically relevant concentration range (Fig. 4B, Fig. 7C); it represented the single missing component for engineering a glucose-inducible transcription unit in human embryonic kidney cells. Therefore, this work indicated that the glucose-sensing mechanism of glycolysis-stimulated calcium entry may not be restricted to β-cells and that it might serve as a blueprint for conferring glucose responsiveness to other mammalian cell types. Collectively, our engineered glucose-inducible excitation-transcription system integrated all potential advantages of an improved alternative for diabetes therapy in future clinical applications: First, unlike technology profiling glucose levels indirectly via low blood pH during diabetic ketoacidosis (Auslander D et al., Mol Cell 55, 397-408 (2014)), our cell-based glucose sensor directly quantifies hyperglycaemia in the absence of any interference with other types of acidosis such as lactic or alcohol acidosis. Second, hyperglycaemia-inducible expression of a glycaemia-lowering protein, such as insulin, allows for the engineering of a closed-loop prosthetic gene circuit that self-(in)activates in an automated manner, i.e., insulin expression occurs exclusively under diabetic hyperglycaemic conditions and remains inactive under normoglycaemic conditions. Such a self-sufficient theranostic device (Kojima R et al., Curr Opin Chem Biol 28, 29-38 (2015)) would guarantee delivery of a therapeutic protein at optimal bioavailable concentrations. Third, the device enables flexible and patient-centered customization of the output protein. The hyperglycaemic disease marker can be coupled to the self-sufficient expression of not only insulinogenic agents such as insulin (T1D) and GLP-1 (T2D) but in principle to any drug against other hyperglycaemia-related diseases, such as cardiovascular disease or the metabolic syndrome. Fourth, engineering of synthetic biology-inspired gene circuits is economical in cost and time when compared to other techniques such as islet transplantation or stem cell differentiation, thus compensating for the main limitation of a regular need for cell replacements in microcapsule implants (Yang HK and Yoon KH, J Diabetes Complications 5, 737-43 (2015)).
β-cell-mimetic designer cells such as HEK-β could combine the best of all strategies: (i) they use glucose-sensor components evolved in native β-cells, (ii) they take advantage of parental cell lines with a track record in biopharmaceutical manufacturing that are known for their robustness and reliability and (iii) they show glucose-induced insulin release performance comparable to β-cell lines and human islets. Additionally, rational programming of designer cells enables (iv) straightforward fine-tuning of performance parameters and provides (v) flexibility to couple glucose-sensing to the production of other therapeutic proteins such as GLP-1 required for the treatment of type-2 diabetes.

Although an ideal implant genotype for T1D therapy might also include postprandial insulin release to attenuate immediate perturbations of blood glucose levels following dietary sugar intake, our experimental and computational analyses showed that the glucose-induced insulin expression system achieved rapid attenuation of life-threatening hyperglycaemia in a T1D animal model by restoring near-homeostatic blood insulin and glucose levels in a self-sufficient and (predicted) robust manner. In particular, glycaemic control in T1D mice was restored in less than one week following implantation - a response time that compares favourably with experimental cell-based therapies in animals using pancreatic progenitor cells (30 weeks; (Rezania A et al., Diabetes 61, 2016-2029 (2012)) or β-cell mimetics produced from human pluripotent stem cells by a seven-stage *in vitro* differentiation protocol (40 days; (Rezania A et al., Nat Biotechnol 32, 1121-1133 (2014)). Furthermore, the animal experiments demonstrated an absolutely increased survival rate already with the current implant version and within only one week of treatment, which should be the foremost criterion to judge the system's efficiency. This work is therefore considered as a proof-of-principle study that introduces an attractive alternative concept for diabetes treatment with therapeutic features that have already been achieved at a standard of practical relevance.

## Claims

1. A recombinant cell comprising
a nucleic acid construct comprising a promoter which is responsive to carbohydrate metabolism of said recombinant cell, wherein the promoter is operably linked to a gene coding for a therapeutic protein, wherein the recombinant cell further comprises a nucleic acid construct coding for a cellular component for sensing extracellular carbohydrates, wherein the cellular component for sensing extracellular carbohydrates is a voltage-gated calcium channel and wherein the promoter which is responsive to carbohydrate metabolism is responsive to a physiological effect of membrane depolarization caused by the carbohydrate metabolism of said recombinant cell.

2. The recombinant cell of anyone of claim 1, wherein the promoter which is responsive to carbohydrate metabolism is a calcium-responsive promoter.

3. The recombinant cell of anyone of claims 1-2, wherein the promoter which is responsive to carbohydrate metabolism is a calcium-responsive promoter comprising nucleic acid sequences bound by transcription factors of the NFAT family, the NFkB family, the AP-1 family, and/or the CREB family and/or cFOS.

4. The recombinant cell of anyone of claims 1-3, wherein the therapeutic protein is an insulinogenic agent selected from the group consisting of GLP1R-agonists, insulin, insulin analogues, growth hormones, insulin-like growth factors; an anorexic hormone; or a protein that activates brown fat metabolism.

5. The recombinant cell of anyone of claims 1-4, wherein the therapeutic protein is an agent against a metabolic disease, wherein the metabolic disease is selected from the group consisting of T1D (type-1 diabetes), T2D (type-2 diabetes), diabetic ketoacidosis, obesity, cardiovascular disease, the metabolic syndrome and cancer.

6. An encapsulated cell comprising the recombinant cell of anyone of claims 1-5 and a semi-permeable membrane.

7. The recombinant cell of anyone of claims 1-5 or the encapsulated cell of claim 6 for use as a medicament.

8. The recombinant cell of anyone of claims 1-5 or the encapsulated cell of claim 6 for use in a method for the prevention, delay of progression or treatment of a metabolic disease in a subject.

9. A method of producing a recombinant cell expressing a therapeutic protein, said method comprising the steps of:
(a) obtaining a population of cells;
(b) transfecting said population of cells with a nucleic acid construct comprising a promoter which is responsive to a physiological effect of membrane depolarization caused by a product of the carbohydrate metabolism of said cell, wherein the promoter is operably linked to a gene coding for a therapeutic protein ,
and wherein said population of cells is further transfected with a nucleic acid construct encoding a cellular component for sensing extracellular carbohydrates, wherein
the cellular component for sensing extracellular carbohydrates is a voltage-gated calcium channel;
(c) incubating the population of transfected cell in the presence of carbohydrates for a sufficient time to permit the transfected cells to express a therapeutic protein.

10. A method to deliver a nucleic acid construct to a cell, wherein the nucleic acid construct comprises a promoter which is responsive to a physiological effect of membrane depolarization caused by the carbohydrate metabolism of said cell and wherein said cell is further transfected with a nucleic acid construct encoding a cellular component for sensing extracellular carbohydrates, wherein the cellular component for sensing extracellular carbohydrates is a voltage-gated calcium channel, wherein the promoter is operably linked to a gene coding for a therapeutic protein, comprising administering said nucleic acid construct to said cell, whereby said gene coding for a therapeutic protein is expressed in said cell in response to carbohydrate stimulation.

## Patentansprüche

1. Rekombinante Zelle, umfassend
ein Nukleinsäurekonstrukt, umfassend einen Promotor, der auf den Kohlenhydratstoffwechsel der rekombinanten Zelle anspricht, wobei der Promotor mit einem Gen operabel verbunden ist, das für ein therapeutisches Protein kodiert, wobei die rekombinante Zelle ferner ein Nukleinsäurekonstrukt umfasst, das für eine zelluläre Komponente zum Erfassen von extrazellulären Kohlenhydraten kodiert, wobei die zelluläre Komponente zum Erfassen von extrazellulären Kohlenhydraten ein spannungsgesteuerter Calciumkanal ist und wobei der Promotor, der auf den Kohlenhydratstoffwechsel anspricht, auf einen physiologischen Effekt der Membrandepolarisation anspricht, der durch den Kohlenhydratstoffwechsel der rekombinanten Zelle verursacht wird.

2. Rekombinante Zelle nach einem von Anspruch 1, wobei der Promotor, der auf den Kohlenhydratstoffwechsel anspricht, ein auf Calcium ansprechender Promotor ist.

3. Rekombinante Zelle nach einem der Ansprüche 1-2, wobei der Promotor, der auf den Kohlenhydratstoffwechsel anspricht, ein auf Calcium ansprechender Promotor ist, umfassend Nukleinsäuresequenzen, die durch Transkriptionsfaktoren der NFAT-Familie, der NFkB-Familie, der AP-1-Familie und/oder der CREB-Familie und/oder cFOS gebunden sind.

4. Rekombinante Zelle nach einem der Ansprüche 1-3, wobei das therapeutische Protein ein insulinogenes Mittel ausgewählt ist aus der Gruppe bestehend aus GLP1R-Agonisten, Insulin, Insulinanaloga, Wachstumshormonen, Insulin-ähnlichen Wachstumsfaktoren, einem Anorexiehormon; oder ein Protein, das den Braunfettstoffwechsel aktiviert.

5. Rekombinante Zelle nach einem der Ansprüche 1-4, wobei das therapeutische Protein ein Mittel gegen eine Stoffwechselerkrankung ist, wobei die Stoffwechselerkrankung ausgewählt ist aus der Gruppe bestehend aus T1D (Typ-1-Diabetes), T2D (Typ-2-Diabetes), diabetischer Ketoazidose, Fettleibigkeit, Herz-Kreislauf-Erkrankungen, metabolischem Syndrom und Krebs.

6. Eingekapselte Zelle, umfassend die rekombinante Zelle nach einem der Ansprüche 1 bis 5 und eine semipermeable Membran.

7. Rekombinante Zelle nach einem der Ansprüche 1-5 oder eingekapselte Zelle nach Anspruch 6 zur Verwendung als Medikament.

8. Rekombinante Zelle nach einem der Ansprüche 1-5 oder eingekapselten Zelle nach Anspruch 6 zur Verwendung in einem Verfahren zum Vorbeugen, Verzögern der Entwicklung oder Behandlung einer Stoffwechselerkrankung bei einem Subjekt.

9. Verfahren zum Erzeugen einer rekombinanten Zelle, die ein therapeutisches Protein exprimiert, wobei das Verfahren die folgenden Schritte umfasst:
(a) Erhalten einer Population von Zellen;
(b) Transfizieren der Zellpopulation mit einem Nukleinsäurekonstrukt, das einen Promotor umfasst, der auf einen physiologischen Effekt der Membrandepolarisation anspricht, der durch ein Produkt des Kohlenhydratstoffwechsels der Zelle verursacht wird, wobei der Promotor mit einem Gen operabel verbunden ist, das für ein therapeutisches Protein kodiert,
und wobei die Zellpopulation ferner mit einem Nukleinsäurekonstrukt transfiziert wird, das für eine zelluläre Komponente zum Erfassen extrazellulärer Kohlenhydrate kodiert, wobei die zelluläre Komponente zum Erfassen extrazellulärer Kohlenhydrate ein spannungsgesteuerter Calciumkanal ist;
(c) Inkubieren der Population transfizierter Zellen in Gegenwart von Kohlenhydraten für eine ausreichende Zeit, damit die transfizierten Zellen ein therapeutisches Protein exprimieren können.

10. Verfahren zum Einführen eines Nukleinsäurekonstrukts in eine Zelle, wobei das Nukleinsäurekonstrukt umfasst: einen Promotor, der auf einen physiologischen Effekt der Membrandepolarisation anspricht, der durch den Kohlenhydratstoffwechsel der Zelle verursacht wird und wobei die Zelle ferner mit einem Nukleinsäurekonstrukt transfiziert wird, das für eine zelluläre Komponente zum Erfassen extrazellulärer Kohlenhydrate kodiert, wobei die zelluläre Komponente zum Erfassen extrazellulärer Kohlenhydrate ein spannungsgesteuerter Calciumkanal ist, wobei der Promotor mit einem Gen operabel verbunden ist, das für ein therapeutisches Protein kodiert, umfassend das Verabreichen des Nukleinsäurekonstrukts an die Zelle, wobei das für ein therapeutisches Protein kodierende Gen in der Zelle als Reaktion auf eine Kohlenhydratstimulation exprimiert wird.

## Revendications

1. Cellule recombinante comprenant
une construction d'acides nucléiques comprenant un promoteur qui est sensible au métabolisme des glucides de ladite cellule recombinante, dans laquelle le promoteur est lié, de manière opérationnelle, à un gène codant pour une protéine thérapeutique, la cellule recombinante comprenant en outre une construction d'acides nucléiques codant pour un composant cellulaire destiné à détecter des glucides extracellulaires, dans laquelle le composant cellulaire destiné à détecter des glucides extracellulaires est un canal calcique dépendant du voltage et dans laquelle le promoteur qui est sensible au métabolisme des glucides est sensible à un effet physiologique de dépolarisation membranaire causé par le métabolisme des glucides de ladite cellule recombinante.

2. Cellule recombinante de l'une quelconque de la revendication 1, dans laquelle le promoteur qui est sensible au métabolisme des glucides est un promoteur sensible au calcium.

3. Cellule recombinante de l'une quelconque des revendications 1 à 2, dans laquelle le promoteur qui est sensible au métabolisme des glucides est un promoteur sensible au calcium comprenant des séquences d'acides nucléiques liées par des facteurs de transcription de la famille des NFAT, la famille des NFkB, la famille des AP-1 et/ou la famille des CREB et/ou cFOS.

4. Cellule recombinante de l'une quelconque des revendications 1 à 3, dans laquelle la protéine thérapeutique est un agent insulinogène choisi dans le groupe constitué par : des agonistes du GLP1R, l'insuline, des analogues de l'insuline, des hormones de croissance, des facteurs de croissance du type insuline ; une hormone de l'anorexie ; ou une protéine qui active le métabolisme de la graisse brune.

5. Cellule recombinante de l'une quelconque des revendications 1 à 4, dans laquelle la protéine thérapeutique est un agent contre une maladie métabolique, dans laquelle la maladie métabolique est choisie dans le groupe constitué par le T1D (diabète de type 1), le T2D (diabète de type 2), une céto-acidose diabétique, l'obésité, une maladie cardiovasculaire, le syndrome métabolique et un cancer.

6. Cellule encapsulée comprenant la cellule recombinante de l'une quelconque des revendications 1 à 5 et une membrane semi-perméable.

7. Cellule recombinante de l'une quelconque des revendications 1 à 5 ou cellule encapsulée de la revendication 6 destinée à être utilisée comme médicament.

8. Cellule recombinante de l'une quelconque des revendications 1 à 5 ou cellule encapsulée de la revendication 6 destinée à être utilisée dans un procédé destiné à la prévention, au retard de la progression ou au traitement d'une maladie métabolique chez un sujet.

9. Procédé de production d'une cellule recombinante exprimant une protéine thérapeutique, ledit procédé comprenant les étapes :
(a) d'obtention d'une population de cellules ;
(b) de transfection de ladite population de cellules avec une construction d'acides nucléiques comprenant un promoteur qui est sensible à un effet physiologique de dépolarisation membranaire causé par un produit du métabolisme des glucides de ladite cellule, dans lequel le promoteur est lié, de manière opérationnelle, à un gène codant pour une protéine thérapeutique,
et dans lequel ladite population de cellules est transfectée en outre avec une construction d'acides nucléiques codant pour un composant cellulaire destiné à détecter des glucides extracellulaires, dans lequel le composant cellulaire destiné à détecter des glucides extracellulaires est un canal calcique dépendant du voltage ;
(c) d'incubation de la population de cellules transfectées en présence de glucides pendant un temps suffisant pour permettre aux cellules transfectées d'exprimer une protéine thérapeutique.

10. Procédé de délivrance d'une construction d'acides nucléiques à une cellule, dans lequel la construction d'acides nucléiques comprend un promoteur qui est sensible à un effet physiologique de dépolarisation membranaire causé par le métabolisme des glucides de ladite cellule et dans lequel ladite cellule est transfectée en outre avec une construction d'acides nucléiques codant pour un composant cellulaire destiné à détecter des glucides extracellulaires, dans lequel le composant cellulaire destiné à détecter des glucides extracellulaires est un canal calcique dépendant du voltage, dans lequel le promoteur est lié, de manière opérationnelle, à un gène codant pour une protéine thérapeutique, comprenant l'étape d'administration de ladite construction d'acides nucléiques à ladite cellule, moyennant quoi ledit gène codant pour une protéine thérapeutique est exprimé dans ladite cellule en réponse à une stimulation par des glucides.
